(19) Europäisches Patentamt

European Patent Office

Office européen des brevets

(11) **EP 2 623 121 A1**

(12) **EUROPEAN PATENT APPLICATION**

(43) Date of publication:
**07.08.2013 Bulletin 2013/32**

(51) Int Cl.:
***A61K 47/48*** (2006.01) ***C12N 15/87*** (2006.01)

(21) Application number: **12153388.9**

(22) Date of filing: **31.01.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
• **Bayer Innovation GmbH
51373 Leverkusen (DE)**
• **Curevac GmbH
72076 Tübingen (DE)**

(72) Inventors:
• **Jarczowski, Franziska
06114 Tübingen (DE)**

• **Klimyuk, Victor
06114 Halle (Saale) (DE)**
• **Kramps, Thomas
72070 Tübingen (DE)**
• **Kallen, Karl-Josef
72076 Tübingen (DE)**
• **Butler-Ransohoff, John Edward
42327 Wuppertal (DE)**

(74) Representative: **BIP Patents
c/o Bayer Intellectual Property GmbH
Creative Campus Monheim
Alfred-Nobel-Straße 10
40789 Monheim (DE)**

(54) **Pharmaceutical composition comprising a polymeric carrier cargo complex and an antigen**

(57) The present invention is directed to a pharmaceutical composition including (eg for use as an adjuvant) a polymeric carrier cargo complex, comprising as a carrier a polymeric carrier formed by disulfide-crosslinked cationic components and as a cargo at least one nucleic acid (molecule) and at least one antigen associated with a tumour or cancer disease selected from; an idiotype immunoglobulin (e.g. an idiotype antibody or an idiotype B cell receptor); or at least one idiotype T cell receptor, or in each case a fragment, variant and/or derivative thereof. The inventive pharmaceutical composition allows for efficient induction of an adaptive immune response directed against the idiotype immunoglobulin or T cell receptor, particularly of a Thi-shiftet immune response. The present invention furthermore provides kits or kits of parts comprising the components of the inventive pharmaceutical composition, as well as the use of the inventive pharmaceutical composition or the inventive kit as a vaccine, particularly in the therapy of a tumour or cancer disease such as lymphoma, particularly B cell or T cell lymphoma.

**Description**

The present invention is directed to a pharmaceutical composition comprising a polymeric carrier cargo complex and at least one antigen, wherein the polymeric carrier cargo complex comprises a polymeric carrier formed by disulfide-crosslinked cationic components as a carrier and at least one nucleic acid (molecule) as a cargo and wherein the antigen is associated to a cancer or tumour disease, particularly to lymphoma or a lymphoma associated disease.

The inventive pharmaceutical composition allows for efficient induction of an adaptive immune response directed against an idiotype immunoglobulin of a lymphoid blood cell or an idiotype T cell receptor of a lymphoid blood cell, particularly it allows for induction of a Th1-shifted immune response.

The present invention furthermore provides kits or kits of parts comprising the components of the inventive pharmaceutical composition, as well as the use of the inventive pharmaceutical composition or the inventive kit or kit of parts as a vaccine, particularly in the treatment of lymphoma, particularly B cell or T cell lymphoma. Furthermore the invention provides: (a) a polymeric carrier cargo complex for use in therapy in combination with at least one idiotype immunoglobulin or at least one idiotype T cell receptor or a fragment, variant and/or derivative thereof, and (b) at least one idiotype immunoglobulin or at least one idiotype T cell receptor or a fragment, variant and/or derivative thereof for use in therapy in combination with a polymeric carrier cargo complex, in each case (a) and (b), particularly for use in therapy of lymphoma.

Many diseases today require administration of adjuvants to provide an innate immune response and to support an adaptive immune response, particularly in the context of vaccinations.

Particularly cancer vaccines need the support of an adjuvant because often self-antigens are used for vaccinations which only bare a low immunogenicity and for which the body has developed tolerance. For this reason an adjuvant is needed which improves the induction of a tumour antigen-specific immune response by induction of an innate immune response.

In this context, lymphoma is the most common type of blood cancer in the United States. It is the seventh most common cancer in adults and the third most common in children. In the United States, about 66,000 new cases of Non-Hodgkin's lymphoma (NHL) and 8,500 new cases of Hodgkin's lymphoma (HL) were expected to be diagnosed in 2010, and the overall incidence is increasing each year. Furthermore, about 20,000 deaths due to NHL were expected in 2010 as well as 1,300 deaths due to HL.

Lymphoma is a malignant transformation of either B or T cells or their subtypes and therefore consists of a group of cancers that affect the cells that play a role in the immune system. As mentioned above, lymphomas fall into one of two major categories: Hodgkin's lymphoma (HL) and all other lymphomas (non-Hodgkin's lymphomas or NHLs).

Non-Hodgkin's lymphoma can be divided in B cell neoplasms, T cell neoplasms and NK cell neoplasms. In this context the immunoglobulin (B cell receptor) idiotype or the T cell receptor idiotype displayed on the surface on malignant B or T cells is a patient- and tumor-specific antigen that can be used for tumour-specific vaccination, because they are specific for the malignant cells.

The specificity of immunoglobulin molecules (antibodies or B cell receptors) are determined by their variable regions at the amino termini of the heavy and the light chains. The variable regions of heavy and light chains combine to form the unique antigen-recognition site of the immunoglobulin protein. These variable regions contain determinants (molecular shapes) that can themselves be recognized as antigens or idiotypes. B-cell tumours are composed of clonal proliferations of cells synthesizing a single antibody molecule with unique variable regions in the heavy and light chains (Kwak, L. W., M. J. Campbell, et al. (1992). "Induction of immune responses in patients with B-cell lymphoma against the surface-immunoglobulin idiotype expressed by their tumors." N Engl J Med 327(17): 1209-15).

Similar to immunoglobulins, also T cell receptors comprise variable domains which allow for specific binding of their antigen. Therefore, it is possible to use these specific structures as tumour-specific markers or tumor-specific antigens of the malignant T cells.

These domains specific for immunoglobulin or T cell receptor are also called idiotypes.

As such the idiotypes represent only weak antigens which must be improved by the coadministration of adjuvants which increase the immunogenicity of the idiotype antigens or circumvent the immune tolerance of the body.

Furthermore it could be shown that active immunization against idiotypic determinants on malignant B cells has produced resistance to tumour growth in several models of syngeneic tumours, as specific anti-tumour therapy against established tumours (reviewed in Kwak *et al.* 1992, see above).

To improve the immunogenicity of idiotypes, Kwak *et al.* conjugated the strongly immunogenic carrier protein KLH) (keyhole-limpet hemocyanin) to the patient-derived immunoglobulins and emulsified it in a squalene-based adjuvant.

But KLH) is an expensive natural product derived from a marine mollusk. Furthermore it is highly reactogenic and very immunogenic (which means that a KLH-specific adaptive immune response is induced directed against KLH). While the reactogenicity is inconvenient to the vaccinee, its immunogenicity may supersede desired vaccine-specific effects due to immunodominance. (Immunodominance means that primarily a KLH-specific adaptive immune response is induced instead of the desired antigen-specific adaptive immune response.)

The idiotype vaccines currently used in clinical trials are based on patient-specific idiotypes (e.g. immunoglobulins) conjugated to KLH, which are administered together with the adjuvant GM-CSF (granulocyte-macrophage colony-stimulating factor (reviewed in Bendandi, M. (2009). "Idiotype vaccines for lymphoma: proof-of-principles and clinical trial failures." Nat Rev Cancer 9(9): 675-81.).

But it turned out that the combination of KLH) and GM-CSF (additionally to the above mentioned disadvantages) induces a $Th_2$-type immunity which is contraindicated for tumor immunotherapy and does not induce effective T cell responses that are increasingly viewed as important (Houot, R. and R. Levy (2009). "Vaccines for lymphomas: idiotype vaccines and beyond." Blood Rev 23(3): 137-42.). Furthermore, the complex vaccination schedule and side-effects may decrease feasibility or compliance in some clinical settings.

Adjuvants are usually defined as compounds that can increase and/or modulate the intrinsic immunogenicity of an antigen. To reduce negative side effects, idiotype vaccines are produced by recombinant expression and therefore have a more defined composition that often leads to a low immunogenicity. Adjuvants are therefore required to assist these idiotype vaccines to induce potent and persistent immune responses, with the additional benefit that less antigen and fewer injections are needed. Now it is clear that the adaptive immune response mainly depends on the level and specificity of the initial danger signals perceived by innate immune cells following infection or vaccination (Guy, B. (2007), Nat Rev Microbiol 5(7): 505-17.).

Unfortunately, only a few licensed adjuvants are available so far. Most prominent is Alum, which is known to be safe, but also represents a very weak adjuvant. Many further adjuvants have been developed, e.g. including the administration of pathogens, CpG-nucleotides, etc. Most of these new or "established" adjuvants, however, still do not satisfy the above requirements, since many new and emerging problems have to be considered and solved.

Particularly for the development of therapeutic lymphoma vaccines, the induction of a T cell immunity is very important, because the vaccination is normally combined with rituximab treatment (anti-CD20 antibody) which depletes B cells and impairs the induction of antibody responses (reviewed in Neelapu, S. S., S. T. Lee, et al. (2006). "Therapeutic lymphoma vaccines: importance of T-cell immunity." Expert Rev Vaccines 5(3): 381-94.). Therefore a Th1-shifted immune response is desirable. But this represents a very challenging future target. To enable vaccine development against lymphoma, more potent adjuvants will be necessary. Such new adjuvants will need to offer advantages, including more heterologous antibody responses, induction of potent functional antibody responses, ensuring tumour cell killing or neutralization, induction of more effective T cell responses, for direct and indirect tumour cell killing, particularly the induction of cytotoxic T cells which are part of a Th1 immune response and support of the antigen to overcome the immune tolerance in the body. In addition, adjuvants may be necessary to achieve more pragmatic effects, including antigen dose reduction because the production of idiotype vaccines in a sufficient amount is still a major problem.

Furthermore safety aspects play a major role for the development of new adjuvants, therefore it is from particular importance that no uncontrolled cytokine storm is initiated by the adjuvant.

In a recent published study, Hong *et al.* (Hong, S., J. Qian, et al. (2011) "CpG or IFN-alpha are more potent adjuvants than GM-CSF to promote anti-tumor immunity following idiotype vaccine in multiple myeloma." Cancer Immunol Immunother. [Epub ahead of print]) examined the combination of idiotype vaccination with CpG or IFN-alpha in a myeloma mouse model. Their results show that these combinations protected the mice from developing myeloma and eradicated established myeloma and additionally that CpG and IFN-alpha are better adjuvants in this context than GM-CSF. Furthermore, they could show that the induced immune responses were associated with an induction of strong humoral immune responses including anti-Id antibodies, and cellular immune responses including Id- and myeloma-specific $CD_8$ (+) cytotoxic T lymphocytes (CTLs), $CD_4$(+) type-1 T-helper (Th1) cells and memory T cells.

Despite of some success, the development of new efficient and safe pharmaceutical compositions including adjuvants for vaccination purposes directed against idiotypes which support induction and maintenance of an adaptive immune response by initiating or boosting a parallel innate immune response to overcome the immune tolerance of the body, represents a main challenging problem. Particularly the induction of the anti-viral cytokine IFN-alpha; which preferably supports a Th1 shifted immune response remains a challenging problem in the context of vaccination against idiotypes.

As already explained above, adjuvants or immunostimulating agents usually act via their capability to induce an innate immune response. The innate immune system forms the dominant system of host defense in most organisms and comprises barriers such as humoral and chemical barriers including, e.g., inflammation, the complement system and cellular barriers. The innate immune system is typically based on a small number of receptors, called pattern recognition receptors. They recognize conserved molecular patterns that distinguish foreign organisms, like viruses, bacteria, fungi and parasites, from cells of the host. Such pathogen-associated molecular patterns (PAMP) include viral nucleic acids, components of bacterial and fungal walls, flagellar proteins, and more. The first family of pattern recognition receptors (PAMP receptors) studied in detail was the Toll-like receptor (TLR) family. TLRs are transmembrane proteins which recognize ligands of the extracellular milieu or of the lumen of endosomes. Following ligand-binding they transduce the signal via cytoplasmic adaptor proteins which leads to triggering of a host-defence response and entailing production of antimicrobial peptides, proinflammatory chemokines and cytokines, antiviral cytokines, etc. (see e.g. Meylan, E., J. Tschopp, et al. (2006), Nature 442(7098): 39-44). Further relevant components of the immune system include e.g. the endosomal TLRs, cytoplasmic receptors, Type I interferons and cytoplasmic receptors. Therefore, the immunostimulating agents or adjuvants are defined herein preferably as inducers of an innate immune response, which activate pattern recognition receptors (PAMP receptors). Hereby, a cascade of signals is elicited, which e.g. may result in the release of cytokines (e.g. IFN-alpha) supporting the innate immune response. Accordingly, it is preferably a feature of an immunostimulating agent or adjuvant to bind to such receptors and activate such PAMP receptors. Ideally, such as an agent or adjuvant additionally supports the adaptive immune response by e.g. shifting the immune response such that the preferred class of Th cells is activated. In the context of the underlying invention a shift to a Th1-based immune reponse may be preferred.

As an example, among the newly developed adjuvants, some nucleic acids like CpG DNA oligonucleotides or is RNA (immunostimulating RNA) turned out to be promising candidates for new immunostimulating agents or adjuvants as they allow the therapeutic or prophylactic induction of an innate immune response. Comprehensibly, such nucleic acid based adjuvants usually have to be delivered effectively to the site of action to allow induction of an effective innate immune response without unnecessary loss of adjuvant activity and, in some cases, without the necessity to increase the administered volume above systemically tolerated levels.

One approach to solve this issue may be the transfection of cells which are part of the innate immune system (e.g. dendritic cells, plasmacytoid dendritic cells (pDCs)) with immunostimulatory nucleic acids, which are ligands of PAMP receptors, (e.g. Toll-like receptors (TLRs)), and thus may lead to immunostimulation by the nucleic acid ligand. Further approaches may be the direct transfection of nucleic acid based adjuvants. All of these approaches, however, are typically impaired by inefficient delivery of the nucleic acid and consequently diminished adjuvant activity, in particular when administered locally.

However, one main disadvantage of such nucleic acid based adjuvant approaches until today is their limited ability to cross the plasma membrane of mammalian cells, resulting in poor cellular access and inadequate therapeutic efficacy. Until today this hurdle represents a major challenge for nucleic acid transfection based applications, e.g. biomedical developments and accordingly the commercial success of many biopharmaceuticals (see e.g. Foerg, C. & Merkle, H.P., J Pharm Ski 97, 144-62 (2008).

Transfection of nucleic acids or genes into cells or tissues has been investigated up to date in the context of *in vitro* transfection purposes and in the context of gene therapeutic approaches. However, no adjuvants are available so far which are based on such gene delivery techniques which are efficient and safe, in particular no licensed adjuvants. This is presumably due to the complex requirements of adjuvants in general in combination with stability issues to be solved in the case of nucleic acid based adjuvants.

Nevertheless, transfection of nucleic acids or genes into cells or tissues for eliciting an (innate and/or adaptive) immune response appears to provide a promising approach to provide new adjuvants.

Even if a lot of transfection methods are known in the art, transfer or insertion of nucleic acids or genes into an individual's cells still represents a major challenge today and is not yet solved satisfactorily. To address this complex issue a variety of methods were developed in the last decade. These include transfection by calcium phosphate, cationic lipids, cationic polymers, and liposomes. Further methods for transfection are electroporation and viral transduction. However, as known to a skilled person, systems for transfer or insertion of nucleic acids or genes have to fulfil several requirements for *in vivo* applications which include efficient nucleic acid delivery into an individual's cells with high functionality, protection of the nucleic acid against ubiquitously occurring nucleases, release of the nucleic acid in the cell, no safety concerns, feasible manufacturing in a commercially acceptable form amenable to scale-up and storage stability under low cost conditions (e.g feasible lyophilisation). These requirements are to be added to the complex requirements of an adjuvant particularly if it is in the form of a nucleic acid as outlined above.

Some successful strategies for the transfer or insertion of nucleic acids may be found in using synthetic vector-based nucleic acid delivery. These approaches typically use synthetic or naturally occurring compounds (e.g. cationic lipids, cationic polymers, lipid-polymer hybrid systems) as carriers to deliver the nucleic acid into the cells. Although significant progress has been made in the basic science and applications of various nonviral nucleic acid delivery systems, the majority of non-viral approaches are still much less efficient than viral vectors, (see e.g. Gao, X., Kim, K. & Liu, D., AAPS J 9, E92-104 (2007)).

Such transfection agents as defined above typically have been used successfully solely in *in vitro* reactions. For application of nucleic acids *in vivo,* however, further requirements have to be fulfilled. For example, complexes between nucleic acids and transfection agents have to be stable in physiological salt solutions with respect to agglomerisation. Furthermore, such complexes typically must not interact with parts of the complement system of the host and thus must not be immunogenic itself as the carrier itself shall not induce an adaptive immune response in the individual. Additionally, the complex shall protect the nucleic acid from early extracellular degradation by ubiquitously occurring nucleases.

In the art many transfection reagents are available, especially cationic lipids, which show excellent transfection activity in cell culture. However, most of these transfection reagents do not perform well in the presence of serum, and only a few are active *in vivo.* A dramatic change in size, surface charge, and lipid composition occurs when lipoplexes are exposed to the overwhelming amount of negatively charged and often amphipathic proteins and polysaccharides that are present in blood, mucus, epithelial lining fluid, or tissue matrix. Once administered *in vivo,* lipoplexes tend to interact with negatively charged blood components and form large aggregates that could be absorbed onto the surface of circulating red blood cells, trapped in a thick mucus layer, or embolized in microvasculatures, preventing them from reaching the intended target cells in the distal location. Some even undergo dissolution after they are introduced to the blood circulation (see e.g. Gao, X., Kim, K. & Liu, D., AAPS J 9, E92-104 (2007)).

One more promising approach utilizes cationic polymers. Cationic polymers turned out to be efficient in transfection of nucleic acids, as they can tightly complex and condense a negatively charged nucleic acid. Thus, a number of cationic polymers have been explored as carriers for *in vitro* and *in vivo* gene delivery. These include polyethylenimine (PEI), polyamidoamine and polypropylamine dendrimers, polyallylamine, cationic dextran, chitosan, cationic proteins and cationic peptides. Although most cationic polymers share the function of condensing DNA into small particles and facilitate cellular uptake via endocytosis through charge-charge interaction with anionic sites on cell surfaces, their transfection activity and toxicity differs dramatically.

Only in one approach in the art, the immunostimulatory effect of RNA complexed to short cationic peptides was demonstrated by Fotin-Mleczek et al. (WO 2009/030481). These formulations appear to efficiently induce the cytokine production in immunocompetent cells. Unfortunately Fotin-Mleczek *et al.* did not assess the induction of the preferable anti-viral cytokine IFN-$\alpha$ by these complexes. Additionally, these complexes turned out to be unstable during lyophilisation.

In the above context, cationic polymers exhibit better transfection efficiency with rising molecular weight. However, a rising molecular weight also leads to a rising toxicity of the cationic polymer. In this above context, (high molecular weight) PEI is perhaps the most active and most studied polymer for transfection of nucleic acids, in particular for gene delivery purposes. Unfortunately, it exhibits the same drawback due to its non-biodegradable nature and toxicity. Furthermore, even though polyplexes formed by high molecular weight polymers exhibit improved stability under physiological conditions, data have indicated that such polymers can hinder vector unpacking. To overcome this negative impact, Read *et al.* (see Read, M.L. et al., J Gene Med. 5, 232-245 (2003); and Read, M.L. et al., Nucleic Acids Res 33, e86 (2005)) developed a new type of synthetic vector based on a linear reducible polycation (RPC) prepared by oxidative polycondensation of the peptide Cys-Lys$_{10}$-Cys. This peptide Cys-Lys$_{10}$-Cys can be cleaved by the intracellular environment to facilitate release of nucleic acids. In this context, Read *et al.* (2003, supra) could show that polyplexes formed by these RPCs are destabilised by reducing conditions enabling efficient release of DNA and mRNA.

Unfortunately, neither Read *et al.* (2003, supra) nor Read *et al.* (2005, supra) did assess as to whether RPCs can be directly used for *in vivo* applications. In their study in 2005, transfections were performed in the absence of serum to avoid masking the ability of histidine residues to enhance gene transfer that may have arisen from binding of serum proteins to polyplexes restricting cellular uptake. Preliminary experiments, however, indicated that the transfection properties of histidine-rich RPC polyplexes can be affected by the presence of serum proteins with a 50% decrease in GFP-positive cells observed in 10% FCS. For *in vivo* application Read *et al.* (2005, supra) proposed modifications with the hydrophilic polymer poly-[N-(2hydroxypropyl)methacrylamide]. Unfortunately, they could not prevent aggregation of polyplexes and binding of polycationic complexes to serum proteins.

In an approach similar to Read *et al. McKenzie et al.* (McKenzie, D. L., K. Y. Kwok, et al. (2000), J Biol Chem 275(14): 9970-7. and McKenzie, D. L., E. Smiley, et al. (2000), Bioconjug Chem 11(6): 901-9) developed cross-linking peptides as gene delivery agents by inserting multiple cysteines into short synthetic peptides. To be noted, the approach of McKenzie et *al.* (2000, supra) is additionally subject of a patent (US 6,770,740 B1), which particularly discloses the transfection of coding nucleic acids, antisense nucleic acids and ribozymes.

Summarizing the above, the prior art does not provide efficient lymphoma vaccines, which allows for efficient vaccination against idiotypes by induction of the particularly preferred Th1-shifted immune response.

Accordingly, it is the object of the present invention to provide such means or methods, which address these problems.

The object underlying the present invention is solved by the subject matter of the present invention, preferably by the subject matter of the attached claims.

For the sake of clarity and readability the following definitions are provided. Any technical features disclosed thereby can be part of each and every embodiment of the invention. Additional definitions and explanations can be provided in the context of this disclosure.

Nucleic acid: The term nucleic acid means any DNA- or RNA-molecule and is used synonymous with polynucleotide. Furthermore, modifications or derivatives of the nucleic acid as defined herein are explicitly included in the general term "nucleic acid".

Monocistronic RNA: A monocistronic RNA may typically be a RNA, preferably a mRNA, that encodes only one open reading frame. An open reading frame in this context is a sequence of several nucleotide triplets (codons) that can be translated into a peptide or protein.

Bi-/multicistronic RNA: RNA, preferably a mRNA, that typically may have two (bicistronic) or more (multicistronic) open reading frames (ORF). An open reading frame in this context is a sequence of several nucleotide triplets (codons) that can be translated into a peptide or protein.

5'-Cap structure: A 5' Cap is typically a modified nucleotide, particularly a guanine nucleotide, added to the 5' end of a RNA-molecule. Preferably, the 5'-Cap is added using a 5'-5'-triphosphate linkage.

Poly(C) sequence: A poly(C) sequence is typically a long sequence of cytosine nucleotides, typically about 10 to about 200 cytidine nucleotides, preferably about 10 to about 100 cytidine nucleotides, more preferably about 10 to about 70 cytidine nucleotides or even more preferably about 20 to about 50 or even about 20 to about 30 cytidine nucleotides. A poly(C) sequence may preferably be located 3' of the coding region comprised by a nucleic acid.

Poly(A) tail: A poly(A) tail also called "3'-poly(A) tail" is typically a long sequence of adenine nucleotides of up to about 400 adenosine nucleotides, e.g. from about 25 to about 400, preferably from about 50 to about 400, more preferably from about 50 to about 300, even more preferably from about 50 to about 250, most preferably from about 60 to about 250 adenosine nucleotides, added to the 3' end of a RNA.

Stabilized nucleic acid: A stabilized nucleic acid, typically, may be essentially resistant to *in vivo* degradation (e.g. degradation by an exo- or endo-nuclease) and/or ex vivo degradation (e.g. by the manufacturing process prior to vaccine administration, e.g. in the course of the preparation of the vaccine solution to be administered). Stabilization of mRNA can, e.g., be achieved by providing a 5'-Cap structure, a Poly(A) tail, a Poly(C) tail, or any other UTR modification. It can also be achieved by backbone modification or modification of the G/C-content of the nucleic acid. Various other methods are conceivable in the context of the invention.

Modification of a nucleic acid (modified nucleic acid): Modification of a nucleic acid molecule may contain backbone modifications, sugar modifications or base modifications. A backbone modification in connection with the present invention is a modification in which phosphates of the backbone of the nucleotides contained in the nucleic acid molecule are chemically modified. A sugar modification in connection with the present invention is a chemical modification of the sugar of the nucleotides of the nucleic acid. Furthermore, a base modification in connection with the present invention is a chemical modification of the base moiety of the nucleotides of the nucleic acid molecule. Therefore a modified nucleic acid is also defined herein as a nucleic acid molecule which may include nucleotide analogues. Furthermore a modification of a nucleic acid molecule can contain a lipid modification. Such a lipid-modified nucleic acid typically comprises a nucleic acid as defined herein. Such a lipid-modified nucleic acid molecule typically further comprises at least one linker covalently linked with that nucleic acid molecule, and at least one lipid covalently linked with the respective linker. Alternatively, the lipid-modified nucleic acid molecule comprises at least one nucleic acid molecule as defined herein and at least one (bifunctional) lipid covalently linked (without a linker) with that nucleic acid molecule. According to a third alternative, the lipid-modified nucleic acid molecule comprises a nucleic acid molecule as defined herein, at least one linker covalently linked with that nucleic acid molecule, and at least one lipid covalently linked with the respective linker, and also at least one (bifunctional) lipid covalently linked (without a linker) with that nucleic acid molecule.

A modification of a nucleic acid may also comprise the modification of the G/C content of the coding region of a nucleic acid molecule, especially if the nucleic acid molecule is in the form of an mRNA. In this context it is particularly preferred that the G/C content of the coding region of the nucleic acid molecule is increased, compared to the G/C content of the coding region of its particular wild type coding sequence, i.e. the unmodified mRNA. The encoded amino acid sequence of the nucleic acid sequence is preferably not modified compared to the coded amino acid sequence of the particular wild type mRNA. The modification of the G/C-content of the nucleic acid molecule, especially if the nucleic acid molecule is in the form of an mRNA or codes for an mRNA, is based on the fact that the sequence of any mRNA region to be translated is important for efficient translation of that mRNA. Thus, the composition and the sequence of various nucleotides are important. In particular, sequences having an increased G (guanosine)/C (cytosine) content are more stable than sequences having an increased A (adenosine)/U (uracil) content. Therefore, the codons of the coding sequence or mRNA are therefore varied compared to its wild type coding sequence or mRNA, while retaining the translated amino acid sequence, such that they include an increased amount of G/C nucleotides. In respect to the fact that several codons code for one and the same amino acid (so-called degeneration of the genetic code), the most favourable codons for the stability can be determined (so-called alternative codon usage). Preferably, the G/C content of the coding region of the nucleic acid molecule, especially if the nucleic acid is in the form of an mRNA or codes for an mRNA, is increased by at least 7%, more preferably by at least 15%, particularly preferably by at least 20%, compared to the G/C content of the coded region of the wild type mRNA. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the region coding for a protein or peptide as defined herein or its fragment, variant and/or derivative thereof or the whole sequence of the wild type mRNA sequence or coding sequence are substituted, thereby increasing the G/C content of said sequence. In this context, it is particularly preferable to increase the G/C content of the nucleic acid molecule, especially if the nucleic acid is in the form of an mRNA or codes for an mRNA, to the maximum (i.e. 100% of the substitutable codons), in particular in the region coding for a protein, compared to the wild type sequence. Furthermore a modification of the nucleic acid, especially if the nucleic acid is in the form of an mRNA or codes for an mRNA, is based on the finding that the translation efficiency is also determined by a different frequency in the occurrence of tRNAs in cells. Thus, if so-called "rare codons" are present in the nucleic acid molecule, especially if the nucleic acid is in the form of an mRNA or codes for an mRNA, to an increased extent, the corresponding modified nucleic acid molecule is translated to a significantly poorer degree than in the case where codons coding for relatively "frequent" tRNAs are present. Therefore, especially if the modified nucleic acid molecule is in the form of an mRNA or codes for an mRNA, the coding region of the modified nucleic acid is preferably modified compared to the corresponding region of the wild type mRNA or coding sequence such that at least one codon of the wild type sequence which codes for a tRNA which is relatively rare in the cell is exchanged for a codon which codes for a tRNA which is relatively frequent in the cell and carries the same amino acid as the relatively rare tRNA. By this modification, the sequences of the nucleic acid molecule, especially if the nucleic acid is in the form of an mRNA or codes for an mRNA, is modified such that codons for which frequently occurring tRNAs are available are inserted. In other words, by this modification all codons of the wild type sequence which code for a tRNA which is relatively rare in the cell can in each case be exchanged for a codon which codes for a tRNA which is relatively frequent in the cell and which, in each case, carries the same amino acid as the relatively rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons which use for the particular amino acid the tRNA which occurs the most frequently, e.g. the Gly codon, which uses the tRNA which occurs the most frequently in the (human) cell, are particularly preferred. In this context, it is particularly preferable to link the sequential G/C content which is increased, in particular maximized, in the modified nucleic acid molecule, especially if the nucleic acid is in the form of an mRNA or codes for an mRNA, with the "frequent" codons without modifying the amino acid sequence of the protein encoded by the coding region of the nucleic acid molecule. This preferred embodiment allows provision of a particularly efficiently translated and stabilized (modified) nucleic acid, especially if the nucleic acid is in the form of an mRNA or codes for an mRNA.

Derivative of a nucleic acid molecule: A derivative of a nucleic acid molecule is defined herein in the same manner as a modified nucleic acid, as defined above.

Nucleotide analogues: Nucleotides structurally similar (analogue) to naturally occurring nucleotides which include phosphate backbone modifications, sugar modifications, or r modifications of the nucleobase.

UTR modification: A nucleic acid molecule, especially if the nucleic acid is in the form of a coding nucleic acid molecule, preferably has at least one 5' and/or 3' stabilizing sequence (UTR modification). These stabilizing sequences in the 5' and/or 3' untranslated regions have the effect of increasing the half-life of the nucleic acid in the cytosol. These stabilizing sequences can have 100% sequence identity to naturally occurring sequences which occur in viruses, bacteria and eukaryotes, but can also be partly or completely synthetic. The untranslated sequences (UTR) of the (alpha-)globin gene, e.g. from *Homo sapiens* or *Xenopus laevis* may be mentioned as an example of stabilizing sequences which can be used for a stabilized nucleic acid. Another example of a stabilizing sequence has the general formula (C/U)CCAN$_x$CCC (U/A)Py$_x$UC(C/U)CC which is contained in the 3'UTR of the very stable RNA which codes for (alpha-)globin, type(I)-collagen, 15-lipoxygenase or for tyrosine hydroxylase (cf. Holcik et al., Proc. Natl. Acad. Sci. USA 1997, 94: 2410 to 2414). Such stabilizing sequences can of course be used individually or in combination with one another and also in combination with other stabilizing sequences known to a person skilled in the art.

Nucleic acid synthesis: Nucleic acid molecules used according to the invention as defined herein may be prepared using any method known in the art, including synthetic methods such as e.g. solid phase synthesis, as well as *in vitro* methods, such as *in vitro* transcription reactions.

For preparation of a nucleic acid molecule, especially if the nucleic acid is in the form of an mRNA, a corresponding DNA molecule may be transcribed *in vitro*. This DNA matrix preferably comprises a suitable promoter, e.g. a T7 or SP6 promoter, for *in vitro* transcription, which is followed by the desired nucleotide sequence for the nucleic acid molecule, e.g. mRNA, to be prepared and a termination signal for *in vitro* transcription. The DNA molecule, which forms the matrix of the at least one RNA of interest, may be prepared by fermentative proliferation and subsequent isolation as part of a plasmid which can be replicated in bacteria. Plasmids which may be mentioned as suitable for the present invention are e.g. the plasmids pT7Ts (GenBank accession number U26404; Lai et al., Development 1995, 121: 2349 to 2360), pGEM® series, e.g. pGEM®-1 (GenBank accession number X65300; from Promega) and pSP64 (GenBank accession number X65327); cf. also Mezei and Storts, Purification of PCR Products, in: Griffin and Griffin (ed.), PCR Technology: Current Innovation, CRC Press, Boca Raton, FL, 2001.

Fragments or variants of nucleic acids: These fragments or variants may typically comprise a sequence having a sequence identity with a nucleic acid, or with a protein or peptide, if encoded by the nucleic acid molecule, of at least 5%, 10%, 20%, 30%, 40%, 50%, 60%, preferably at least 70%, more preferably at least 80%, equally more preferably at least 85%, even more preferably at least 90% and most preferably at least 95% or even 97%, 98% or 99%, to the entire wild type sequence, either on nucleic acid level or on amino acid level.

Protein: A protein typically consists of one or more polypeptides folded into 3-dimensional form, facilitating a biological function.

Peptide: A peptide is typically a short polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units, more specifically between 50 and 300 monomeric units. Furthermore a "peptide" is defined herein also to include any peptidyl molecule, including peptide analogues.

Peptide analogues: A peptide analogue may comprise naturally or non-naturally occurring amino acids which may be used for the purpose of the invention. For example they can comprise amino acids selected from an isostere or a chiral analog (D-amino acid or L-amino acid) of an amino acid. Additionally, the analog may comprise one or more amino acids, preferably selected from hydroxyproline, β-alanine, 2,3-diaminopropionic acid, α-aminoisobutyric acid, N-methylglycine (sarcosine), ornithine, citrulline, t-butylalanine, t-butylglycine, N-methylisoleucine, phenylglycine, cyclohexylalanine, norleucine, naphthylalanine, pyridylananine 3- benzothienyl alanine 4-chlorophenylalanine, 2-fluorophenylalanine, 3- fluorophenylalanine, 4-fluorophenylalanine, penicillamine, 1,2,3,4-tetrahydro-tic isoquinoline-3-carboxylic acid [beta-2-thienylalanine, methionine sulfoxide, homoarginine, N-acetyl lysine, 2,4-diamino butyric acid, p-aminophenylalanine , N-methylvaline, homocysteine, homoserine, ε-amino hexanoic acid, δ-amino valeric acid, 2,3-diaminobutyric acid. A peptide analogue as defined herein may further contain modified peptides. The term specifically includes peptide back-bone modifications (i.e., amide bond mimetics) known to those skilled in the art. Such modifications include modifications of the amide nitrogen, the α-carbon, amide carbonyl, complete replacement of the amide bond, extensions, deletions or backbone crosslinks. Several peptide backbone modifications are known, including $\Psi[CH_2S]$, $\Psi CH_2NH]$, $\Psi[CSNH_2]$, $\Psi[NHCO]$, $\Psi[COCH_2]$, and $\Psi[(E)$ or $(Z)$ CH=CH]. In the nomenclature used above, $\Psi$ indicates the absence of an amide bond. The structure that replaces the amide group is specified within the brackets. Other modifications include, for example, an N-alkyl (or aryl) substitution ($\Psi[CONR]$), or backbone crosslinking to construct lactams and other cyclic structures, C-terminal hydroxymethyl modifications, O-modified modifications (e.g., C-terminal hydroxymethyl benzyl ether), N-terminal modifications including substituted amides such as alkylaniides and hydrazides.

Peptide synthesis: A peptide including peptide analogues or derivatives is preferably synthesized using a chemical method known to the skilled artisan. For example, synthetic peptides are prepared using known techniques of solid phase, liquid phase, or peptide condensation, or any combination thereof, and can include natural and/or unnatural amino acids. Generally, chemical synthesis methods comprise the sequential addition of one or more amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complementary (amino or carboxyl) group suitably protected, under conditions that allow for the formation of an amide linkage. The protecting group is then removed from the newly added amino acid residue and the next amino acid (suitably protected) is then added, and so forth. After the desired amino acids have been linked in the proper sequence, any remaining protecting groups (and any solid support, if solid phase synthesis techniques are used) are removed sequentially or concurrently, to render the final polypeptide. These methods are suitable for synthesis of a peptide used for the purpose of the present invention (including a peptide analogue or derivative). Typical protecting groups include t-butyloxycarbonyl (Boc), 9-fluorenylmethoxycarbonyl (Fmoc) benzyloxycarbonyl (Cbz); p-toluenesulfonyl (Tx); 2,4-dinitrophenyl ; benzyl (Bzl); biphenylisopropyloxycarboxy-carbonyl, t- amyloxycarbonyl, isobornyloxycarbonyl, o-bromobenzyloxycarbonyl, cyclohexyl, isopropyl, acetyl, o-nitrophenylsulfonyl and the like. Typical solid supports are cross-linked polymeric supports. These can include divinylbenzene cross-linked-styrene-based polymers, for example, divinylbenzene- hydroxymethylstyrene copolymers, divinylbenzene- chloromethylstyrene copolymers and divinylbenzene-benzhydrylaminopolystyrene copolymers.

Recombinant peptide or protein production: A peptide or protein or derivative thereof may be produced using recombinant protein or peptide production. To facilitate the production of a recombinant peptide or protein, at least one nucleic acid encoding same is preferably isolated or synthesized. Typically the nucleic acid encoding the recombinant protein or peptide is isolated using a known method, such as, for example, amplification (e.g., using PCR) or isolated from nucleic acid from an organism using one or more restriction enzymes or isolated from a library of nucleic acids. For expressing a protein or peptide by recombinant means, a protein/peptide-encoding nucleic acid is placed in operable connection with a promoter or other regulatory sequence capable of regulating expression in a cell-free system or cellular system. For example, nucleic acid comprising a sequence that encodes a peptide or protein is placed in operable connection with a suitable promoter and maintained in a suitable cell for a time and under conditions sufficient for expression to occur. Typical expression vectors for in vitro expression, cell-free expression or cell-based expression have been described and are well known for the skilled person. In this context cell-free expression systems may include E. coli $S_{30}$ fraction, rabbit reticulocyte lysate and wheat germ extract and a cellular system may be selected from bacterial (e.g. *E. coli),* insect, plant, or mammalian cells (e.g., 293, COS, CHO, 1OT cells, 293T cells).

Secretory signal peptide: Such signal peptides are sequences, which typically exhibit a length of about 15 to 30 amino acids and are preferably located at the N-terminus of the encoded peptide, without being limited thereto. Signal peptides as defined herein preferably allow the transport of the protein or peptide into a defined cellular compartment, preferably the cell surface, the endoplasmic reticulum (ER) or the endosomal-lysosomal compartment.

<u>Carrier / polymeric carrier:</u> A carrier in the context of the invention may typically be a compound that facilitates transport and/or complexation of another compound. A polymeric carrier is typically a carrier that is formed of a polymer.

<u>Cationic component:</u> The term "cationic component" typically refers to a charged molecule, which is positively charged (cation) at a pH value of about typically 1 to 9, preferably of a pH value of or below 9 (e.g. 5 to 9), of or below 8 (e.g. 5 to 8), of or below 7 (e.g. 5 to 7), most preferably at physiological pH values, e.g. about 7.3 to 7.4. Accordingly, a cationic peptide, protein or polymer according to the present invention is positively charged under physiological conditions, particularly under physiological salt conditions of the cell *in vivo.* A cationic peptide or protein contains a larger number of cationic amino acids, e.g. a large number of Arg, His, Lys or Orn than other residues. The definition "cationic" may also refer to "polycationic" components.

<u>Pharmaceutically effective amount:</u> A pharmaceutically effective amount in the context of the invention is typically understood to be an amount that is sufficient to induce an immune response.

<u>Immune system:</u> The immune system may protect organisms from infection. If a pathogen breaks through a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts contains so called humoral and cellular components.

<u>Immune response:</u> An immune response may typically either be a specific reaction of the adaptive immune system to a particular antigen (so called specific or adaptive immune response) or an unspecific reaction of the innate immune system (so called unspecific or innate immune response). The invention relates to the core to specific reactions (adaptive immune responses) of the adaptive immune system. However, this specific response can be supported by an additional unspecific reaction (innate immune response). Therefore, the invention also relates to a compound for simultaneous stimulation of the innate and the adaptive immune system to evoke an efficient adaptive immune response.

<u>Adaptive immune response:</u> The adaptive immune response is typically understood to be antigen-specific. Antigen specificity allows for the generation of responses that are tailored to specific antigens, antigen-expressing cells, pathogens or pathogen-infected cells. The ability to mount these tailored responses is maintained in the body by "memory cells". Should a pathogen infect the body more than once, these specific memory cells are used to quickly eliminate it. In this context, the first step of an adaptive immune response is the activation of naive antigen-specific T cells or different immune cells able to induce an antigen-specific immune response by antigen-presenting cells. This occurs in the lymphoid tissues and organs through which naive T cells are constantly passing. Cell types that can serve as antigen-presenting cells are inter alia dendritic cells, macrophages, and B cells. Each of these cells has a distinct function in eliciting immune responses. Dendritic cells take up antigens by phagocytosis and macropinocytosis and are stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. Presenting the antigen on MHC molecules leads to activation of T cells which induces their proliferation and differentiation into armed effector T cells. The most important function of effector T cells is the killing of infected cells by $CD_8$+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind antigen directly, but instead recognize short peptide fragments e.g. of pathogen-derived protein antigens, which are bound to MHC molecules on the surfaces of other cells.

<u>Adaptive immune system:</u> The adaptive immune system is composed of highly specialized, systemic cells and processes that eliminate or prevent pathogenic growth. The adaptive immune response provides the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of that cell will then inherit genes encoding the same receptor specificity, including the Memory B cells and Memory T cells that are the keys to long-lived specific immunity. Immune network theory is a theory of how the adaptive immune system works, that is based on interactions between the variable regions of the receptors of T cells, B cells and of molecules made by T cells and B cells that have variable regions.

<u>Innate immune system:</u> The innate immune system, also known as non-specific immune system, comprises the cells and mechanisms that defend the host from infection by other organisms in a non-specific manner. This means that the cells of the innate system recognize and respond to pathogens in a generic way, but unlike the adaptive immune system, it does not confer long-lasting or protective immunity to the host. The innate immune system may be e.g. activated by ligands of pathogen-associated molecular patterns (PAMP) receptors, e.g. Toll-like receptors (TLRs) or other auxiliary substances such as lipopolysaccharides, TNF-alpha, $CD_{40}$ ligand, or cytokines, monokines, lymphokines, interleukins or chemokines, IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. Typically a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system through a process known as antigen presentation; and/or acting as a physical and chemical barrier to infectious agents.

Cellular immunity/cellular immune response: Cellular immunity relates typically to the activation of macrophages, natural killer cells (NK), antigen-specific cytotoxic T-lymphocytes, and the release of various cytokines in response to an antigen. In a more general way, cellular immunity is not related to antibodies but to the activation of cells of the immune system. A cellular immune response is characterized e.g. by activating antigen-specific cytotoxic T-lymphocytes that are able to induce apoptosis in body cells displaying epitopes of an antigen on their surface, such as virus-infected cells, cells with intracellular bacteria, and cancer cells displaying tumor antigens; activating macrophages and natural killer cells, enabling them to destroy pathogens; and stimulating cells to secrete a variety of cytokines that influence the function of other cells involved in adaptive immune responses and innate immune responses.

Humoral immunity/humoral immune response: Humoral immunity refers typically to antibody production and the accessory processes that may accompany it. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Antigen: According to the present invention, the term "antigen" refers to a substance which is recognized by the immune system and is capable of triggering an antigen-specific immune response, e.g. by formation of antibodies or antigen-specific T-cells as part of an adaptive immune response. Typically an antigen is a protein or peptide. In this context, the first step of an adaptive immune response is the activation of naive antigen-specific T cells by antigen-presenting cells. This occurs in the lymphoid tissues and organs through which naive T cells are constantly passing. The three cell types that can serve as antigen-presenting cells are dendritic cells, macrophages, and B cells. Each of these cells has a distinct function in eliciting immune responses. Tissue dendritic cells take up antigens by phagocytosis and macropinocytosis and are stimulated by infection to migrate to the local lymphoid tissue, where they differentiate into mature dendritic cells. Macrophages ingest particulate antigens such as bacteria and are induced by infectious agents to express MHC class II molecules. The unique ability of B cells to bind and internalize soluble protein antigens via their receptors may be important to induce T cells. By presenting the antigen on MHC molecules leads to activation of T cells which induces their proliferation and differentiation into armed effector T cells. The most important function of effector T cells is the killing of infected cells by CD8$^+$ cytotoxic T cells and the activation of macrophages by TH1 cells which together make up cell-mediated immunity, and the activation of B cells by both TH2 and TH1 cells to produce different classes of antibody, thus driving the humoral immune response. T cells recognize an antigen by their T cell receptors which does not recognize and bind antigen directly, but instead recognize short peptide fragments e.g. of pathogens' protein antigens, which are bound to MHC molecules on the surfaces of other cells. T cells fall into two major classes that have different effector functions. The two classes are distinguished by the expression of the cell-surface proteins CD4 and CD8. These two types of T cells differ in the class of MHC molecule that they recognize. There are two classes of MHC molecules - MHC class I and MHC class II molecules - which differ in their structure and expression pattern on tissues of the body. CD4$^+$ T cells bind to a MHC class II molecule and CD8$^+$ T cells to a MHC class I molecule. MHC class I and MHC class II molecules have distinct distributions among cells that reflect the different effector functions of the T cells that recognize them. MHC class I molecules present peptides from pathogens, commonly viruses to CD8$^+$ T cells, which differentiate into cytotoxic T cells that are specialized to kill any cell that they specifically recognize. Almost all cells express MHC class I molecules, although the level of constitutive expression varies from one cell type to the next. But not only pathogenic peptides from viruses are presented by MHC class I molecules, also self-antigens like tumour antigens are presented by them. MHC class I molecules bind peptides from proteins degraded in the cytosol and transported in the endoplasmic reticulum. Thereby MHC class I molecules on the surface of cells infected with viruses or other cytosolic pathogens display peptides from these pathogen. The CD8$^+$ T cells that recognize MHC class I:

peptide complexes are specialized to kill any cells displaying foreign peptides and so rid the body of cells infected with viruses and other cytosolic pathogens. The main function of CD4$^+$ T cells (CD4$^+$ helper T cells) that recognize MHC class II molecules is to activate other effector cells of the immune system. Thus MHC class II molecules are normally found on B lymphocytes, dendritic cells, and macrophages, cells that participate in immune responses, but not on other tissue cells. Macrophages, for example, are activated to kill the intravesicular pathogens they harbour, and B cells to secrete immunoglobulins against foreign molecules. MHC class II molecules are prevented from binding to peptides in the endoplasmic reticulum and thus MHC class II molecules bind peptides from proteins which are degraded in endosomes. They can capture peptides from pathogens that have entered the vesicular system of macrophages, or from antigens internalized by immature dendritic cells or the immunoglobulin receptors of B cells. Pathogens that accumulate in large numbers inside macrophage and dendritic cell vesicles tend to stimulate the differentiation of TH1 cells, whereas extracellular antigens tend to stimulate the production of TH2 cells. TH1 cells activate the microbicidal properties of macrophages and induce B cells to make IgG antibodies that are very effective of opsonising extracellular pathogens for ingestion by phagocytic cells, whereas TH2 cells initiate the humoral response by activating naive B cells to secrete IgM, and induce the production of weakly opsonising antibodes such as IgG1 and IgG$_3$ (mouse) and IgG2 and IgG4 (human) as well as IgA and IgE (mouse and human).

Vaccine: A vaccine is typically understood to be a prophylactic or therapeutic material providing at least one antigen or antigenic function. The antigen or antigenic function stimulates the body's adaptive immune system to provide an adaptive immune response.

Immunostimulating agent: The term "immunostimulating agent" is typically understood not to include agents as e.g. antigens (of whatever chemical structure), which elicit an adaptive/cytotoxic immune response, e.g. a "humoral" or "cellular" immune response, in other words elicit immune reponses (and confer immunity by themselves) which are characterized by a specific response to structural properties of an antigen recognized to be foreign by immune competent cells. Rather, by "immunostimulating agent", it is typically understood to mean agents/compounds/complexes which do not trigger any adaptive/cytotoxic immune response by themselves, but which may exlusively enhance such an adaptive/ cytotoxic immune reponse in an unspecific way, by e.g. activating "PAMP" receptors and thereby triggering the release of cytokines which support the actual adaptive/cytotoxic immune response. Accordingly, any immunostimulation by agents (e.g. antigens) which evoke an adaptive and/or cytotoxic immune response by themselves (conferring immunity by themselves directly or indirectly) is typically disclaimed by the phrase "immunostimulating agent".

Adjuvant: The term "adjuvant" is also understood not to comprise agents which confer immunity by themselves. Accordingly, adjuvants do not by themselves typically confer immunity, but assist the immune system in various ways to enhance the antigen-specific immune response by e.g. promoting presentation of an antigen to the immune system. Hereby, an adjuvant may preferably e.g. modulate the antigen-specific immune response by e.g. shifting the dominating Th1-based antigen specific response to a more Th$_2$-based antigen specific response or vice versa. Accordingly, the terms "immunostimulating agent" and "adjuvant" in the context of the present invention are typically understood to mean agents, compounds or complexes which do not confer immunity by themselves, but exclusively support the immune reponse in an unspecific way (in contrast to an antigen-specific immune response) by effects, which modulate the antigen-specific (adaptive cellular and/or humoral immune response) by unspecific measures, e.g. cytokine expression/secretion, improved antigen presentation, shifting the nature of the arms of the immune response etc.. Accordingly, any agents evoking by themselves immunity are typically disclaimed by the terms "adjuvant" or "immunostimulating agent".

Immunostimulatory RNA: An immunostimulatory RNA (is RNA) in the context of the invention may typically be a RNA that is able to induce an innate immune response itself. It usually does not have an open reading frame and thus does not provide a peptide-antigen but elicits an innate immune response e.g. by binding to a specific kind of pathogen-associated molecular patterns (PAMP) receptors (e.g. Toll-like-receptor (TLR) or other suitable receptors). However, of course also mRNAs having an open reading frame and coding for a peptide/protein (e.g. an antigenic function) may induce an innate immune response.

Fragments of proteins or peptides: Fragments of proteins or peptides in the context of the present invention may comprise a sequence of a protein or peptide as defined herein, which is, with regard to its amino acid sequence (or its encoded nucleic acid molecule), N-terminally, C-terminally and/or intrasequentially truncated compared to the amino acid sequence of the original (native) protein (or its encoded nucleic acid molecule). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid molecule of such a protein or peptide. Likewise, "fragments" of nucleic acids in the context of the present invention may comprise a sequence of a nucleic acid as defined herein, which is, with regard to its nucleic acid molecule 5'-, 3'- and/or intrasequentially truncated compared to the nucleic acid molecule of the original (native) nucleic acid molecule. A sequence identity with respect to such a fragment as defined herein may therefore preferably refer to the entire nucleic acid as defined herein. Fragments of proteins or peptides in the context of the present invention may furthermore comprise a sequence of a protein or peptide as defined herein, which has a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form. Fragments of proteins or peptides may comprise at least one epitope of those proteins or peptides. Furthermore also domains of a protein, like the extracellular domain, the intracellular domain or the transmembran domain and shortened or truncated versions of a protein may be understood to comprise a fragment of a protein.

Epitope (also called "antigen determinant"): T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form.

B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Variants of proteins or peptides: Variants of proteins or peptides as defined in the context of the present invention may be generated, having an amino acid sequence which differs from the original sequence in one or more mutation (s), such as one or more substituted, inserted and/or deleted amino acid(s). Preferably, these fragments and/or variants have the same biological function or specific activity compared to the full-length native protein, e.g. its specific antigenic property.

"Variants" of proteins or peptides as defined in the context of the present invention may comprise conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

Additionally variants of proteins or peptides may comprise peptide analogues as defined herein.

Furthermore, variants of proteins or peptides as defined herein, which may be encoded by a nucleic acid molecule, may also comprise those sequences, wherein nucleotides of the nucleic acid are exchanged according to the degeneration of the genetic code, without leading to an alteration of the respective amino acid sequence of the protein or peptide, i.e. the amino acid sequence or at least part thereof may not differ from the original sequence in one or more mutation(s) within the above meaning.

In order to determine the percentage to which two sequences are identical, e.g. nucleic acid sequences or amino acid sequences as defined herein, preferably the amino acid sequences encoded by a nucleic acid sequence of the polymeric carrier as defined herein or the amino acid sequences themselves, the sequences can be aligned in order to be subsequently compared to one another. Therefore, e.g. a position of a first sequence may be compared with the corresponding position of the second sequence. If a position in the first sequence is occupied by the same component as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program. A "variant" of a protein or peptide may have at least 70%, 75%, 80%, 85%, 90%, 95%, 98% or 99% amino acid identity over a stretch of 10, 20, 30, 50, 75 or 100 amino acids of such protein or peptide. Analogously, a "variant" of a nucleic acid sequence may have at least 70%, 75%, 80%, 85%, 90%, 95%, 98% or 99% nucleotide identity over a stretch of 10, 20, 30, 50, 75 or 100 nucleotide of such nucleic acid sequence

Derivative of a protein or peptide: A derivative of a peptide or protein is a molecule that is derived from another molecule, such as said peptide or protein. A "derivative" of a peptide or protein also encompasses fusions comprising a peptide or protein used in the present invention. For example, the fusion comprises a label, such as, for example, an epitope, e.g., a FLAG epitope or a V5 epitope or an HA epitope. For example, the epitope is a FLAG epitope. Such a tag is useful for, for example, purifying the fusion protein. The term "derivative" of a peptide or protein also encompasses a derivatised peptide or protein, such as, for example, a peptide or protein modified to contain one or more-chemical moieties other than an amino acid. The chemical moiety may be linked covalently to the peptide or protein e.g., via an amino terminal amino acid residue, a carboxyl terminal amino acid residue, or at an internal amino acid residue. Such modifications include the addition of a protective or capping group on a reactive moiety in the peptide or protein, addition of a detectable label, and other changes that do not adversely destroy the activity of the peptide or protein compound. For example, a derivative may comprise a PEG moiety, radionuclide, coloured latex, etc. A derivative generally possesses or exhibits an improved characteristic relative to a e.g., enhanced protease resistance and/or longer half-life and/or enhanced transportability between cells or tissues of the human or animal body and/or reduced adverse effect(s) and/or enhanced affinity or immunogenicity. WO 2010/003193 describes various methodologies to provide peptide or protein derivatives which may be employed separately or in combination using standard procedures known to the person of ordinary skill, including derivatisation of a protein or peptide by e.g. PEGylation, HESylation, or glycosylation.

Idiotype immunoglobulin: Immunoglobulins typically have a so-called variable region which is responsible for recognition and binding of a particular antigen. This variable region has a characteristic 3-dimensional structure (molecular shape) which may also function as an epitope, i.e. the variable region may be a target for antibody recognition, too. The particular variable region of a B-cell is determined by so-called somatic or V(D)J recombination. Once a B cell has produced a functional immunoglobulin gene during V(D)J recombination, it cannot express any other variable region due to allelic exclusion. Thus, each B cell is restricted to produce immunoglobulins containing only one type of variable region. In this context, idiotypic immunoglobulins are typically understood to be immunglobulins having a variable region with an identical molecular shape. The particular molecular shape of the variable region may be called the idiotype of the immunoglobulin. Accordingly, each B-cell-clone may be characterized by expression of an unique immunoglobulin-variant having a particular idiotype. Thus, immunoglobulin idiotypes, i.e. peptides or proteins having the particular molecular shape of the variable region of an immunoglobulin expressed by a particular B-cell-clone, may be used as antigens for eliciting an immune response directed against this particular type of B-cells from that B-cell-clone, particularly against malignant B-cells. Typically, e.g. B-cell lymphoma diseases are characterized by mass-production of malignant B-cells which are all characterized by the very same idiotypic immunoglobuline, since they are derived from one single highly proliferative progenitor cell.

Idiotype T cell receptor: Like immunoglobulins in B-cells, in T-cells, T cell receptors do typically have a specific variable region obtained by somatic recombination and, after maturation, each T-cell can produce only a single and specific type of T-cell receptors. In this context, idiotypic T cell receptors are typically understood to be T cell receptors having a variable region with an identical molecular shape. The particular molecular shape of the variable region may be called the idiotype of the T cell receptor. Accordingly, each T-cell-clone may be characterized by expression of an unique T cell receptor-variant having a particular idiotype. Thus, T cell receptor idiotypes, i.e. peptides or proteins having the particular molecular shape of the variable region of a T cell receptor expressed by a particular T-cell-clone, may be used as antigens for eliciting an immune response directed against this particular type of T-cells from that T-cell-clone, particularly against malignant T-cells. Typically, e.g. T-cell lymphoma diseases are characterized by mass-production of malignant T-cells which are all characterized by the very same idiotypic immunoglobuline, since they are derived from one single highly proliferative progenitor cell.

According to a first aspect, one or more objects underlying the present invention are solved by a pharmaceutical composition comprising:

(A) a polymeric carrier cargo complex, comprising:

a) a polymeric carrier formed by disulfide-crosslinked cationic components, and

b) at least one nucleic acid molecule,

and

at least one antigen associated with a tumour or cancer disease, particularly lymphoma or a lymphoma associated disease, wherein said antigen is an immunoglobulin idiotype of a lymphoid blood cell or an T cell receptor idiotype of a lymphoid blood cell, or a fragment, variant and/or derivative of such an idiotype immunoglobulin or idiotype T cell receptor.

In this context lymphoid blood cells may be, preferably, malignant B-cells or malignant T-cells which are obtainable from a patient suffering from a lymphoma disease or a lymphoma associated disease. A lymphoma associated disease may be, e.g., a cancerous or tumourous disease affecting the blood or bone marrow. Analysis of said malignant cells may provide the particular idiotype of immunoglobulin or T cell receptor of those cells. The pharmaceutical composition of the invention may then be used for treatment of the patient. For example, the number of malignant cells may be reduced by chemotherapy while at the same time or subsequently the patient may be vaccinated with the inventive pharmaceutical composition. This may induce a specific reaction of the adaptive immune system against the provided idiotype of immunoglobulins or T cell receptors. In turn, the immune system may fight remaining or newly developing malignant cells of the particular B-cell or T-cell type.

The polymeric carrier cargo complex of the inventive pharmaceutical composition may be for use as an adjuvant.

Preferably, the at least one polymeric carrier cargo complex, may comprise

a) a polymeric carrier formed by disulfide-crosslinked cationic components as a carrier, and
b) at least one nucleic acid molecule as a cargo.

Such a polymeric carrier cargo complex comprised in the inventive pharmaceutical composition allows provision of a more efficient and/or safer adjuvant for vaccination purposes. Advantageously, the polymeric carrier cargo complex is suited for *in vivo* delivery of nucleic acids, in particular for compacting and stabilizing a nucleic acid for the purposes of nucleic acid transfection, such as exhibiting one or more reduced negative side effects of high-molecular weight polymers as discussed above, such as poor biodegradability or high toxicity, agglomeration, low transfection activity in *vivo,* etc. The polymeric carrier cargo complex also provides for improved nucleic acid transfer *in vivo* particularly via intradermal or intramuscular routes, including serum stability, salt stability, efficiency of uptake, reduced complement activation, nucleic acid release, etc. Such a polymeric carrier cargo complex, furthermore may support induction and maintenance of an adaptive immune response by initiating or boosting a parallel innate immune response. Additionally, the polymeric carrier cargo complex may exhibit improved storage stability, particularly during lyophilisation.

The polymeric carrier cargo complex as defined above comprises as one component a polymeric carrier formed by disulfide-crosslinked cationic components. The term "cationic component" typically refers to a charged molecule, which is positively charged (cation) at a pH value of about 1 to 9, preferably of a pH value of or below 9, of or below 8, of or below 7, most preferably at physiological pH values, e.g. about 7.3 to 7.4. Accordingly, a cationic peptide, protein or polymer according to the present invention is positively charged under physiological conditions, particularly under physiological salt conditions of the cell *in vivo.* The definition "cationic" may also refer to "polycationic" components.

In this context the cationic components, which form basis for the polymeric carrier of the polymeric carrier cargo complex by disulfide-crosslinkage, are typically selected from any suitable cationic or polycationic peptide, protein or polymer suitable for this purpose, particular any cationic or polycationic peptide, protein or polymer capable to complex a nucleic acid as defined according to the present invention, and thereby preferably condensing the nucleic acid. The cationic or polycationic peptide, protein or polymer, is preferably a linear molecule, however, branched cationic or polycationic peptides, proteins or polymers may also be used.

Each cationic or polycationic protein, peptide or polymer of the polymeric carrier contains at least one -SH moiety, most preferably at least one cysteine residue or any further chemical group exhibiting an -SH moiety, capable to form a disulfide linkage upon condensation with at least one further cationic or polycationic protein, peptide or polymer as cationic component of the polymeric carrier as mentioned herein.

Each cationic or polycationic protein, peptide or polymer or any further component of the polymeric carrier is preferably linked to its neighbouring component(s) (cationic proteins, peptides, polymers or other components) via disulfide-crosslinking. Preferably, the disulfide-crosslinking is a (reversible) disulfide bond (-S-S-) between at least one cationic or polycationic protein, peptide or polymer and at least one further cationic or polycationic protein, peptide or polymer or other component of the polymeric carrier. The disulfide-crosslinking is typically formed by condensation of -SH-moieties of the components of the polymeric carrier particularly of the cationic components. Such an -SH-moiety may be part of the structure of the cationic or polycationic protein, peptide or polymer or any further component of the polymeric carrier prior to disulfide-crosslinking or may be added prior to disulfide-crosslinking by a modification as defined below. In this context, the sulphurs adjacent to one component of the polymeric carrier, necessary for providing a disulfide bond, may be provided by the component itself, e.g. by a -SH moiety as defined herein or may be provided by modifying the component accordingly to exhibit a -SH moiety. These -SH-moieties are typically provided by each of the component, e.g. via a cysteine or any further (modified) amino acid or compound of the component, which carries a -SH moiety. In the case that the cationic component or any further component of the polymeric carrier is a peptide or protein it is preferred that the -SH moiety is provided by at least one cysteine residue. Alternatively, the component of the polymeric carrier may be modified accordingly with a -SH moiety, preferably via a chemical reaction with a compound carrying a -SH moiety, such that each of the components of the polymeric carrier carries at least one such -SH moiety. Such a compound carrying a -SH moiety may be e.g. an (additional) cysteine or any further (modified) amino acid or compound of the component of the polymeric carrier, which carries a -SH moiety. Such a compound may also be any non-amino compound or moiety, which contains or allows to introduce a -SH moiety into the component as defined herein. Such non-amino compounds may be attached to the component of the polymeric carrier according to the present invention via chemical reactions or binding of compounds, e.g. by binding of a 3-thio propionic acid or 2-iminothiolane (Traut's reagent), by amide formation (e.g. carboxylic acids, sulphonic acids, amines, etc.), by Michael addition (e.g maleinimide moieties, $\alpha,\beta$ unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow $S_n$-type substitution reactions (e.g halogenalkans, thiols, alcohols, amines, hydrazines, hydrazides, sulphonic acid esters, oxyphosphonium salts) or other chemical moieties which can be utilized in the attachment of further components. In some cases the -SH moiety may be masked by protecting groups during chemical attachment to the component. Such protecting groups are known in the art and may be removed after chemical coupling. In each case, the -SH moiety, e.g. of a cysteine or of any further (modified) amino acid or compound, may be present at the terminal ends or internally at any position of the component of the polymeric carrier. As defined herein, each of the components of the polymeric carrier typically exhibits at least one -SH-moiety, but may also contain two, three, four, five, or even more -SH-moieties. Additionally to binding of cationic components a -SH moiety may be used to attach further components of the polymeric carrier as defined herein, particularly an amino acid component, e.g. antigen epitopes, antigens, antibodies, cell penetrating peptides (e.g. TAT), ligands, etc.

As defined above, the polymeric carrier of the polymeric carrier cargo complex is formed by disulfide-crosslinked cationic (or polycationic) components.

According to one alternative, at least one cationic (or polycationic) component of the polymeric carrier may be selected from cationic or polycationic peptides or proteins. Such cationic or polycationic peptides or proteins preferably exhibit a length of about 3 to 100 amino acids, preferably a length of about 3 to 50 amino acids, more preferably a length of about 3 to 25 amino acids, e.g. a length of about 3 to 10; 5 to 20; 5 to 15; 8 to 15, 16 or 17; 10 to 15, 16, 17, 18, 19, or 20; or 15 to 25 amino acids. Alternatively or additionally, such cationic or polycationic peptides or proteins may exhibit a molecular weight of about 0.01 kDa to about 100 kDa, including a molecular weight of about 0.5 kDa to about 100 kDa, preferably of about 10 kDa to about 50 kDa, even more preferably of about 10 kDa to about 30 kDa. In this context also analogues and derivatives of proteins or peptides as defined herein are explicitly encompassed.

Said cationic or polycationic peptides or proteins may be prepared by all methods known to a person of ordinary skill or by recombinant peptide or protein production as defined herein or by peptide synthesis as defined herein.

In the specific case that the cationic component of the polymeric carrier comprises a cationic or polycationic peptide or protein, the cationic properties of the cationic or polycationic peptide or protein or of the entire polymeric carrier, if the polymeric carrier is entirely composed of cationic or polycationic peptides or proteins, may be determined upon its content of cationic amino acids. Preferably, the content of cationic amino acids in the cationic or polycationic peptide or protein and/or the polymeric carrier is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even more preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in the cationic or polycationic peptide or protein, or in the entire polymeric carrier, if the polymeric carrier is entirely composed of cationic or polycationic peptides or proteins, is 100%.

In this context, cationic amino acids are preferably the naturally occurring amino acids Arg (Arginine), Lys (Lysine), His (Histidine), and Orn (Ornithin). However, in a broader sense any (non-natural) amino acid carrying a cationic charge on its side chain may also be envisaged to carry out the invention. However, those cationic amino acids are preferred which comprise side chains which are positively charged under physiological pH conditions. In a more preferred embodiment, these amino acids are Arg, Lys, and Orn.

Preferably, such cationic or polycationic peptides or proteins of the polymeric carrier, which comprise or are additionally modified to comprise at least one -SH moiety, are selected from, without being restricted thereto, cationic peptides or proteins such as protamine, nucleoline, spermine or spermidine, oligo- or poly-L-lysine (PLL), basic polypeptides, oligo or poly-arginine, cell penetrating peptides (CPPs), chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia),* pAntp, plsl, etc., antimicrobial-derived CPPs e.g. Buforin-2, Bac715-24, SynB, SynB(1), pVEC, hCT-derived peptides, SAP, MAP, KALA, PpTG20, Loligomere, FGF, Lactoferrin, histones, VP22 derived or analog peptides, HSV, VP22 (Herpes simplex), MAP, KOALA or protein transduction domains (PTDs, PpT620, prolin-rich peptides, arginine-rich peptides, lysine-rich peptides, Pep-1, L-oligomers, Calcitonin peptide(s), etc.

Alternatively or additionally, such cationic or polycationic peptides or proteins of the polymeric carrier, which comprise or are additionally modified to comprise at least one -SH moeity, are selected from, without being restricted thereto, following cationic peptides having the following sum formula (III):

$$\{(Arg)_l;(Lys)_m;(His)_n;(Orn)_o;(Xaa)_x\};$$

wherein 1 + m + n +o + x = 3-100, and 1, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred. Even more preferred peptides of this formula are oligoarginines such as e.g. $Arg_7$, $Arg_8$, $Arg_9$, $Arg_{12}$, $His_3Arg_9$, $ArggHis_3$, $His_3ArggHis_3$, $His_6Arg_9His_6$, $His_3Arg_4His_3$, $His_6Arg_4His_6$, $TyrSer_2Arg_9Ser_2Tyr$, $(ArgLysHis)_4$, $Tyr(ArgLysHis)_2Arg$, etc.

According to a particular preferred embodiment, such cationic or polycationic peptides or proteins of the polymeric carrier having the empirical sum formula (III) as shown above, may, without being restricted thereto, comprise at least one of the following subgroup of formulae:

$Arg_7$, $Arg_8$, $Arg_9$, $Arg_{10}$, $Arg_{11}$, $Arg_{12}$, $Arg_{13}$, $Arg_{14}$, $Arg_{15-30}$;

$Lys_7$, $Lys_8$, $Lys_9$, $Lys_{10}$, $Lys_{11}$, $Lys_{12}$, $Lys_{13}$, $Lys_{14}$, $Lys_{15-30}$;

$His_7$, $His_8$, $His_9$, $His_{10}$, $His_{11}$, $His_{12}$, $His_{13}$, $His_{14}$, $His_{15-30}$;

$Orn_7$, $Orn_8$, $Orng$, $Orn_{10}$, $Orn_{11}$, $Orn_{12}$, $Orn_{13}$, $Orn_{14}$, $Orn_{15-30}$;

According to a further particularly preferred embodiment, cationic or polycationic peptides or proteins of the polymeric carrier, having the empirical sum formula (III) as shown above and which comprise or are additionally modified to comprise at least one -SH moiety, may be preferably selected from, without being restricted thereto, at least one of the following subgroup of formulae. The following formulae (as with empirical formula (III)) do not specify any amino acid order, but are intended to reflect empirical formulae by exclusively specifying the (number of) amino acids as components of the respective peptide. Accordingly, as an example, empirical formula $Arg_{(7-29)}Lys_1$ is intended to mean that peptides falling under this formula contain 7 to 19 Arg residues and 1 Lys residue of whatsoever order. If the peptides contain 7 Arg residues and 1 Lys residue, all variants having 7 Arg residues and 1 Lys residue are encompassed. The Lys residue may therefore be positioned anywhere in the e.g. 8 amino acid long sequence composed of 7 Arg and 1 Lys residues. The subgroup preferably comprises:

$Arg_{(4-29)}Lys_1$, $Arg_{(4-29)}His_1$, $Arg_{(4-29}Orn_1$, $Lys_{(4-29)}His_1$, $Lys_{(4-29)}Orn_1$, $His_{(4-29)}Orn_1$, $Arg_{(3-28)}Lys_2$, $Arg_{(3-28)}His_2$, $Arg_{(3-28)}Orn_2$, $Lys_{(3-28)}His_2$, $Lys_{(3-28)}Orn_2$, $His_{(3-28)}Orn_2$, $Arg_{(2-27)}Lys_3$, $Arg_{(2-27)}His_3$, $Arg_{(2-27)}Orn_3$, $Lys_{(2-27)}His_3$, $Lys_{(2-27)}Orn_3$, $His_{(2-27)}Orn_3$, $Arg_{(1-26)}Lys_4$, $Arg_{(1-26)}His_4$, $Arg_{(1-26)}Orn_4$, $Lys_{(1-26)}His_4$, $Lys_{(1-26)}Orn_4$, $His_{(1-26)}Orn_4$,

$Arg_{(3-28)}Lys_1His_1$, $Arg_{(3-28)}Lys_1Orn_1$, $Arg_{(3-28)}His_1Orn_1$, $Arg_1Lys_{(3-28)}His_1$, $Arg_1Lys_{(5-28)}Orn_1$, $Lys_{(3-28)}His_1Orn_1$, $Arg_1Lys_1His_{(3-28)}$, $Arg_1His_{(3-28)}Orn_1$, $Lys_1His_{(3-28)}Orn_1$;

$Arg_{(2-27)}Lys_2His_1$, $Arg_{(2-27)}Lys_1His_2$, $Arg_{(2-27)}Lys_2Orn_1$, $Arg_{(2-27)}Lys_1Orn_2$, $Arg_{(2-27)}His_2Orn_1$, $Arg_{(2-27)}His_1$,$Orn_2$, $Arg_2Lys_{(2-27)}His_1$, $Arg_1Lys_{(2-27)}His_2$, $Arg_2Lys_{(2-27)}Orn_1$, $Arg_1Lys_{(2-27)}Orn_2$, $Lys_{(2-27)}His_2Orn_1$, $Lys_{(2-27)}His_1Orn_2$, $Arg_2Lys_1His_{(2-27)}$, $Arg_1Lys_2His_{(2-27)}$, $Arg_2His_{(2-27)}Orn_1$, $Arg_1His_{(2-27)}Orn_2$, $Lys_2His_{(2-27)}Orn_1$, $Lys_1His_{(2-27)}Orn_2$;

$Arg_{(1-26)}Lys_3His_1$, $Arg_{(1-26)}Lys_2His_2$, $Arg_{(1-26)}Lys_1His_3$, $Arg_{(1-26)}Lys_3Orn_1$, $Arg_{(1-26)}Lys_2Orn_2$, $Arg_{(1-26)}Lys_1Orn_3$,

$Arg_{(1-26)}His_3Orn_1$, $Arg_{(1-26)}His_2Orn_2$, $Arg_{(1-26)}His_1Orn_3$, $Arg_3Lys_{(1-26)}His_1$, $Arg_2Lys_{(1-26)}His_2$, $Arg_1Lys_{(1-26)}His_3$, $Arg_3Lys_{(1-26)}Orn_1$, $Arg_2Lys_{(1-26)}Orn_2$, $Arg_1Lys_{(1-26)}Orn_3$, $Lys_{(1-26)}His_3Orn_1$, $Lys_{(1-26)}His_2Orn_2$, $Lys_{(1-26)}His_1Orn_3$, $Arg_3Lys_1His_{(1-26)}$, $Arg_2Lys_2His_{(1-26)}$, $Arg_1Lys_3His_{(1-26)}$, $Arg_3His_{(1-26)}Orn_1$, $Arg_2His_{(1-26)}Orn_2$, $Arg_1His_{(1-26)}Orn_3$, $Lys_3His_{(1-26)}Orn_1$, $Lys_2His_{(1-26)}Orn_2$, $Lys_1His_{(1-26)}Orn_3$;

$Arg_{(2-27)}Lys_1His_1Orn_1$, $Arg_1Lys_{(2-27)}His_1Orn_1$, $Arg_1Lys_1His_{(2-27)}Orn_1$, $Arg_1Lys_1His_1Orn_{(2-27)}$;

$Arg_{(1-26)}Lys_2His_1Orn_1$, $Arg_{(1-26)}Lys_1His_2Orn_1$, $Arg_{(1-26)}Lys_1His_1Orn_2$, $Arg_2Lys_{(1-26)}His_1Orn_1$, $Arg_1Lys_{(1-26)}His_2Orn_1$, $Arg_1Lys_{(1-26)}His_1Orn_2$ $Arg_2Lys_1His_{(1-26)}Orn_1$, $Arg_1Lys_2His_{(1-26)}Orn_1$, $Arg_1Lys_1His_{(1-26)}Orn_2$, $Arg_2Lys_1His_1Orn_{(1-26)}$, $Arg_1Lys_2His_1Orn_{(1-26)}$, $Arg_1Lys_1His_2Orn_{(1-26)}$;

According to a further particular preferred embodiment, cationic or polycationic peptides or proteins of the polymeric carrier, having the empirical sum formula (III) as shown above and which comprise or are additionally modified to comprise at least one -SH moiety, may be, without being restricted thereto, selected from the subgroup consisting of generic formulas $Arg_7$ (also termed as $R_7$), $Arg_9$ (also termed $R_9$), $Arg_{12}$ (also termed as $R_{12}$).

According to a further particular preferred embodiment, the cationic or polycationic peptide or protein of the polymeric carrier, when defined according to formula a $\{(Arg)_l;(Lys)_m;(His)_n;(Orn)_o;(Xaa)_x\}$ (formula (III)) as shown above and which comprise or are additionally modified to comprise at least one -SH moeity, may be, without being restricted thereto, selected from subformula (IIIa):

$$\{(Arg)_l;(Lys)_m;(His)_n;(Orn)_o;(Xaa')_x (Cys)_y\} \qquad \text{formula (IIIa)}$$

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide.

This embodiment may apply to situations, wherein the cationic or polycationic peptide or protein of the polymeric carrier, e.g. when defined according to empirical formula $(Arg)_l;(Lys)_m;(His)_n;(Orn)_o;(Xaa)_x$ (formula (III)) as shown above, comprises or has been modified with at least one cysteine as -SH moiety in the above meaning such that the cationic or polycationic peptide as cationic component carries at least one cysteine, which is capable to form a disulfide bond with other components of the polymeric carrier.

According to another particular preferred embodiment, the cationic or polycationic peptide or protein of the polymeric carrier, when defined according to formula a $\{(Arg)_l;(Lys)_m;(His)_n;(Orn)_o;(Xaa)_x\}$ (formula (III)) as shown above, may be, without being restricted thereto, selected from subformula (IIIb):

$$Cys_1 \{(Arg)_l;(Lys)_m;(His)_n;(Orn)_o;(Xaa)_x\} Cys_2, \qquad \text{(formula (IIIb))}$$

wherein empirical formula $\{(Arg)_l;(Lys)_m;(His)_n;(Orn)_o;(Xaa)_x\}$ (formula (III)) is as defined herein and forms a core of an amino acid sequence according to (semiempirical) formula (III) and wherein $Cys_1$, and $Cys_2$, are Cysteines proximal to, or terminal to $\{(Arg)_l;(Lys)_m;(His)_n;(Orn)_o;(Xaa)_x\}$. Exemplary examples may comprise any of the above sequences flanked by two Cys and following sequences:

$Cys(Arg_7)Cys$, $Cys(Arg_8)Cys$, $Cys(Arg_9)Cys$, $Cys(Arg_{10})Cys$, $Cys(Arg_{11})Cys$, $Cys(Arg_{12})Cys$, $Cys(Arg_{13})Cys$, $Cys(Arg_{14})Cys$, $Cys(Arg_{15})Cys$, $Cys(Arg_{16})Cys$, $Cys(Arg_{17})Cys$, $Cys(Arg_{18})Cys$, $Cys(Arg_{19})Cys$, $Cys(Arg_{20})Cys$ (SEQ ID NOs:1-14):

$CysArg_7Cys$  Cys-Arg-Arg-Arg-Arg-Arg-Arg-Arg-Cys (SEQ ID NO.1)
$CysArg_8Cys$  Cys-Arg-Arg-Arg-Arg-Arg-Arg-Arg-Arg-Cys (SEQ ID NO.2)
$CysArg_9Cys$:  Cys-Arg-Arg-Arg-Arg-Arg-Arg-Arg-Arg-Arg-Cys (SEQ ID NO. 3)
$CysArg_{10}Cys$  Cys-Arg-Arg-Arg-Arg-Arg-Arg-Arg-Arg-Arg-Arg-Cys (SEQ ID NO.4)
$CysArg_{11}Cys$  Cys-Arg-Arg-Arg-Arg-Arg-Arg-Arg-Arg-Arg-Arg-Cys (SEQ ID NO. 5)
$CysArg_{12}Cys$:  Cys-Arg-Arg-Arg-Arg-Arg-Arg-Arg-Arg-Arg-Arg-Arg-Cys (SEQ ID NO.6)
$CysArg_{13}Cys$:  Cys-Arg-Arg-Arg-Arg-Arg-Arg-Arg-Arg-Arg-Arg-Arg-Arg-Arg-Cys (SEQ ID NO.7)
$CysArg_{14}Cys$:  Cys-Arg-Arg-Arg-Arg-Arg-Arg-Arg-Arg-Arg-Arg-Arg-Arg-Cys (SEQ ID NO.8)
$CysArg_{15}Cys$:  Cys-Arg-Arg-Arg-Arg-Arg-Arg-Arg-Arg-Arg-Arg-Arg-Arg-Arg-Cys (SEQ ID NO.9)

CysArg$_{16}$Cys: Cys-Arg-Arg-Arg-Arg-Arg-Arg-Arg-Arg-Arg-Arg-Arg-Arg-Arg-Arg-Arg-Cys (SEQ ID NO. 10)

CysArg$_{17}$Cys: Cys-Arg-Arg-Arg-Arg-Arg-Arg-Arg-Arg-Arg-Arg-Arg-Arg-Arg-Arg-Arg-Arg-Cys (SEQ ID NO. 11)

CysArg$_{18}$Cys: Cys-Arg-Arg-Arg-Arg-Arg-Arg-Arg-Arg-Arg-Arg-Arg-Arg-Arg-Arg-Arg-Arg-Arg-Cys (SEQ ID NO. 12)

CysArg$_{19}$Cys: Cys-Arg-Arg-Arg-Arg-Arg-Arg-Arg-Arg-Arg-Arg-Arg-Arg-Arg-Arg-Arg-Arg-Arg-Arg-Cys (SEQ ID NO. 13)

CysArg$_{20}$Cys: Cys-Arg-Arg-Arg-Arg-Arg-Arg-Arg-Arg-Arg-Arg-Arg-Arg-Arg-Arg-Arg-Arg-Arg-Arg-Arg Cys (SEQ ID NO. 14)

This embodiment may apply to situations, wherein the cationic or polycationic peptide or protein of the polymeric carrier, e.g. when defined according to empirical formula $(Arg)_l;(Lys)_m;(His)_n;(Orn)_o;(Xaa)_x$ (formula (III)) as shown above, has been modified with at least two cysteines as -SH moieties in the above meaning such that the cationic or polycationic peptide of the inventive polymeric carrier cargo complex as cationic component carries at least two (terminal) cysteines, which are capable to form a disulfide bond with other components of the polymeric carrier.

According to a further alternative, at least one cationic (or polycationic) component of the polymeric carrier may be selected from e.g. any (non-peptidic) cationic or polycationic polymer suitable in this context, provided that this (non-peptidic) cationic or polycationic polymer exhibits or is modified to exhibit at least one -SH-moiety, which provide for a disulfide bond linking the cationic or polycationic polymer with another component of the polymeric carrier as defined herein. Thus, likewise as defined herein, the polymeric carrier may comprise the same or different cationic or polycationic polymers.

In the specific case that the cationic component of the polymeric carrier comprises a (non-peptidic) cationic or polycationic polymer the cationic properties of the (non-peptidic) cationic or polycationic polymer may be determined upon its content of cationic charges when compared to the overall charges of the components of the cationic polymer. Preferably, the content of cationic charges in the cationic polymer at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 30% to 100%, even preferably in the range of about 50% to 100%, e.g. 50, 60, 70, 80, 90% or 100%, or in a range formed by any two of the afore mentioned values, provided, that the content of all charges, e.g. positive and negative charges at a (physiological) pH as defined herein, in the entire cationic polymer is 100%.

Preferably, the (non-peptidic) cationic component of the polymeric carrier represents a cationic or polycationic polymer, typically exhibiting a molecular weight of about 0.1 or 0.5 kDa to about 100 kDa, preferably of about 1 kDa to about 75 kDa, more preferably of about 5 kDa to about 50 kDa, even more preferably of about 5 kDa to about 30 kDa, or a molecular weight of about 10 kDa to about 50 kDa, even more preferably of about 10 kDa to about 30 kDa. Additionally, the (non-peptidic) cationic or polycationic polymer typically exhibits at least one -SH-moiety, which is capable to form a disulfide linkage upon condensation with either other cationic components or other components of the polymeric carrier as defined herein.

In the above context, the (non-peptidic) cationic component of the polymeric carrier may be selected from acrylates, modified acrylates, such as pDMAEMA (poly(dimethylaminoethyl methylacrylate)), chitosanes, aziridines or 2-ethyl-2-oxazoline (forming oligo ethylenimines or modifed oligoethylenimines), polymers obtained by reaction of bisacrylates with amines forming oligo beta aminoesters or poly amido amines, or other polymers like polyesters, polycarbonates, etc. Each molecule of these (non-peptidic) cationic or polycationic polymers typically exhibits at least one -SH-moiety, wherein these at least one -SH-moiety may be introduced into the (non-peptidic) cationic or polycationic polymer by chemical modifications, e.g. using imonothiolan, 3-thio propionic acid or introduction of -SH-moieties containing amino acids, such as cysteine or any further (modified) amino acid. Such -SH-moieties are preferably as already defined above.

In the context of the polymeric carrier, the cationic components, which form basis for the polymeric carrier by disulfide-crosslinkage, may be the same or different from each other. It is also particularly preferred that the polymeric carrier of the present invention comprises mixtures of cationic peptides, proteins or polymers and optionally further components as defined herein, which are crosslinked by disulfide bonds as described herein.

In this context, the polymeric carrier cargo complex due to its variable polymeric carrier advantageously allows to combine desired properties of different (short) cationic or polycationic peptides, proteins or polymers or other components. The polymeric carrier, e.g., allows to efficiently compact nucleic acids for the purpose of efficient transfection of nucleic acids and for adjuvant therapy, without loss of activity, particularly exhibiting an efficient transfection of a nucleic acid into different cell lines *in vitro* but particularly transfection *in vivo.* The polymeric carrier and thus the polymeric carrier cargo complex is furthermore not toxic to cells, provides for efficient release of its nucleic acid cargo, is stable during lyophilization and is applicable as adjuvant. Preferably, the polymer carrier cargo complex may induce the antiviral cytokine IFN-alpha.

In particular, the polymeric carrier formed by disulfide-linked cationic components allows considerably to vary its peptide or polymeric content and thus to modulate its biophysical/biochemical properties, particularly the cationic properties of the polymeric carrier, quite easily and fast, e.g. by incorporating as cationic components the same or different cationic peptide(s) or polymer(s) and optionally adding other components into the polymeric carrier. Even though consisting of quite small non-toxic monomer units the polymeric carrier forms a long cationic binding sequence providing a strong condensation of the nucleic acid cargo and complex stability. Under the reducing conditions of the cytosole (e.g. cytosolic GSH), the complex is rapidly degraded into its (cationic) components, which are further degraded (e.g. oligopeptides). This supports deliberation of the nucleic acid cargo in the cytosol. Due to degradation into small oligopeptides or polymers in the cytosol, no toxicity is observed as known for high-molecular oligopeptides or polymers, e.g. from high-molecular polyarginine. Accordingly, the polymeric carrier of the polymeric carrier cargo complex may comprise different (short) cationic or polycationic peptides, proteins or polymers selected from cationic or polycationic peptides, proteins or (non-peptidic) polymers as defined above, optionally together with further components as defined herein.

Additionally, the polymeric carrier of the polymeric carrier cargo complex as defined above, more preferably at least one of the different (short) cationic or polycationic peptides or (non-peptidic) polymers forming basis for the polymeric carrier via disulfide-crosslinking, may be, preferably prior to the disulfide-crosslinking, be modified with at least one further component. Alternatively, the polymeric carrier as such may be modified with at least one further component. It may also optionally comprise at least one further component, which typically forms the polymeric carrier disulfide together with the other the (short) cationic or polycationic peptides as defined above via disulfide crosslinking.

To allow modification of a cationic or polycationic peptide or a (non-peptidic) polymer as defined above, each of the components of the polymeric carrier may (preferably already prior to disulfide-crosslinking) also contain at least one further functional moiety, which allows attaching such further components as defined herein. Such functional moieties may be selected from functionalities which allow the attachment of further components, e.g. functionalities as defined herein, e.g. by amide formation (e.g. carboxylic acids, sulphonic acids, amines, etc.), by Michael addition (e.g maleinimide moieties, $\alpha,\beta$ unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow $S_n$-type substitution reactions (e.g halogenalkans, thiols, alcohols, amines, hydrazines, hydrazides, sulphonic acid esters, oxyphosphonium salts) or other chemical moieties which can be utilized in the attachment of further components.

According to a particularly preferred embodiment, the further component, which may be contained in the polymeric carrier or which may be used to modify the different (short) cationic or polycationic peptides or (non-peptidic) polymers forming basis for the polymeric carrier of the inventive polymeric carrier cargo complex is an amino acid component (AA), which may e.g. modify the biophysical/biochemical properties of the polymeric carrier as defined herein. According to the present invention, the amino acid component (AA) comprises a number of amino acids preferably in a range of about 1 to 100, preferably in a range of about 1 to 50, more preferably selected from a number comprising 1, 2, 3, 4, 5, 6, 7, 8, 9, 10, 11, 12, 13, 14 or 15-20, or may be selected from a range formed by any two of the afore mentioned values. In this context the amino acids of amino acid component (AA) can be chosen independently from each other. For example if in the polymeric carrier two or more (AA) components are present they can be the same or can be different from each other.

The amino acid component (AA) may contain or may be flanked (e.g. terminally) by a -SH containing moiety, which allows introducing this component (AA) via a disulfide bond into the polymeric carrier as defined herein. In the specific case that the -SH containing moiety represents a cysteine, the amino acid component (AA) may also be read as -Cys-(AA)-Cys- wherein Cys represents Cysteine and provides for the necessary -SH-moiety for a disulfide bond. The -SH containing moiety may be also introduced into amino acid component (AA) using any of modifications or reactions as shown above for the cationic component or any of its components.

Furthermore, the amino acid component (AA) may be provided with two -SH-moieties (or even more), e.g. in a form represented by formula HS-(AA)-SH to allow binding to two functionalities via disulfide bonds, e.g. if the amino acid component (AA) is used as a linker between two further components (e.g. as a linker between two cationic polymers). In this case, one -SH moiety is preferably protected in a first step using a protecting group as known in the art, leading to an amino acid component (AA) of formula HS-(AA)-S-protecting group. Then, the amino acid component (AA) may be bound to a further component of the polymeric carrier, to form a first disulfide bond via the non-protected -SH moiety. The protected-SH-moiety is then typically deprotected and bound to a further free -SH-moiety of a further component of the polymeric carrier to form a second disulfide bond.

Alternatively, the amino acid component (AA) may be provided with other functionalities as already described above for the other components of the polymeric carrier, which allow binding of the amino acid component (AA) to any of components of the polymeric carrier.

Thus, the amino acid component (AA) may be bound to further components of the polymeric carrier with or without using a disulfide linkage. Binding without using a disulfide linkage may be accomplished by any of the reactions described above, preferably by binding the amino acid component (AA) to the other component of the polymeric carrier using an amid-chemistry as defined herein. If desired or necessary, the other terminus of the amino acid component (AA), e.g. the N- or C-terminus, may be used to couple another component, e.g. a ligand L. For this purpose, the other terminus of the amino acid component (AA) preferably comprises or is modified to comprise a further functionality, e.g. an alkyn-species (see above), which may be used to add the other component via e.g. click-chemistry. If the ligand is bound via an acid-labile bond, the bond is preferably cleaved off in the endosome and the polymeric carrier presents amino acid component (AA) at its surface.

The amino acid component (AA) may occur as a further component of the polymeric carrier as defined above, e.g. as a linker between cationic components e.g. as a linker between one cationic peptide and a further cationic peptide, as a linker between one cationic polymer and a further cationic polymer, as a linker between one cationic peptide and a cationic polymer, all preferably as defined herein, or as an additional component of the polymeric carrier, e.g. by binding the amino acid component (AA) to the polymeric carrier or a component thereof, e.g. via side chains, SH-moieties or via further moieties as defined herein, wherein the amino acid component (AA) is preferably accordingly modified.

According to a further and particularly preferred alternative, the amino acid component (AA), may be used to modify the polymeric carrier, particularly the content of cationic components in the polymeric carrier as defined above.

In this context it is preferable, that the content of cationic components in the polymeric carrier is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 30% to 100%, more preferably in the range of about 50% to 100%, even preferably in the range of about 70% to 100%, e.g. 70, 80, 90 or 100%, or in a range formed by any two of the afore mentioned values, provided, that the content of all components in the polymeric carrier is 100%.

In the context of the present invention, the amino acid component (AA) may be selected from the following alternatives.

According to a first alternative, the amino acid component (AA) may be an aromatic amino acid component (AA). The incorporation of aromatic amino acids or sequences as amino aromatic acid component (AA) into the polymeric carrier of the present invention enables a different (second) binding of the polymeric carrier to the nucleic acid due to interactions of the aromatic amino acids with the bases of the nucleic acid cargo in contrast to the binding thereof by cationic charged sequences of the polymeric carrier molecule to the phosphate backbone. This interaction may occur e.g. by intercalations or by minor or major groove binding. This kind of interaction is not prone to decompaction by anionic complexing partners (e.g. Heparin, Hyaluronic acids) which are found mainly in the extracellular matrix *in vivo* and is also less susceptible to salt effects.

For this purpose, the amino acids in the aromatic amino acid component (AA) may be selected from either the same or different aromatic amino acids e.g. selected from Trp, Tyr, or Phe.

Additionally, the aromatic amino acid component (AA) may contain or may be flanked by a -SH containing moiety, which allows introducing this component via a disulfide bond as a further part of the polymeric carrier as defined above, e.g. as a linker. Such a -SH containing moiety may be any moiety as defined herein suitable to couple one component as defined herein to a further component as defined herein. As an example, such a -SH containing moiety may be a cysteine.

Additionally, the aromatic amino acid component (AA) may contain or represent at least one proline, which may serve as a structure breaker of longer sequences of Trp, Tyr and Phe in the aromatic amino acid component (AA), preferably two, three or more prolines.

According to a second alternative, the amino acid component (AA) may be a hydrophilic (and preferably non charged polar) amino acid component (AA). The incorporation of hydrophilic (and preferably non charged polar) amino acids or sequences as amino hydrophilic (and preferably non charged polar) acid component (AA) into the polymeric carrier of the present invention enables a more flexible binding to the nucleic acid cargo. This leads to a more effective compaction of the nucleic acid cargo and hence to a better protection against nucleases and unwanted decompaction. It also allows provision of a (long) polymeric carrier which exhibits a reduced cationic charge over the entire carrier and in this context to better adjusted binding properties, if desired or necessary.

For this purpose, the amino acids in the hydrophilic (and preferably non charged polar) amino acid component (AA) may be selected from either the same or different hydrophilic (and preferably non charged polar) amino acids e.g. selected from Thr, Ser, Asn or Gln.

Additionally, the hydrophilic (and preferably non-charged polar) amino acid component (AA) may contain or may be flanked by a -SH containing moiety, which allows introducing this component via a disulfide bond as a further part of generic formula (III) above, e.g. as a linker. Such a -SH containing moiety may be any moiety as defined herein suitable to couple one component as defined herein to a further component as defined herein. As an example, such a - SH containing moiety may be a cysteine.

Additionally, the hydrophilic (and preferably non-charged polar) amino acid component (AA) may contain at least one proline, which may serve as a structure breaker of longer sequences of Ser, Thr and Asn in the hydrophilic (and preferably non charged polar) amino acid component (AA), preferably two, three or more prolines.

According to a third alternative, the amino acid component (AA) may be a lipohilic amino acid component (AA).

The incorporation of lipohilic amino acids or sequences as amino lipohilic acid component (AA) into the polymeric carrier of the present invention enables a stronger compaction of the nucleic acid cargo and/or the polymeric carrier and its nucleic acid cargo when forming a complex. This is particularly due to interactions of one or more polymer strands of the polymeric carrier, particularly of lipophilic sections of lipohilic amino acid component (AA) and the nucleic acid cargo. This interaction will preferably add an additional stability to the complex between the polymeric carrier and its nucleic acid cargo. This stabilization may somehow be compared to a sort of non covalent crosslinking between different polymerstrands. Especially in aqueous environment this interaction is typically strong and provides a significant effect.

For this purpose, the amino acids in the lipophilic amino acid component (AA) may be selected from either the same or different lipophilic amino acids e.g. selected from Leu, Val, Ile, Ala, Met.

Additionally, the lipophilic amino acid component (AA) may contain or may be flanked by a -SH containing moiety, which allows introducing this component via a disulfide bond as a further part of the polymeric carrier above, e.g. as a linker. Such a -SH containing moiety may be any moiety as defined herein suitable to couple one component as defined herein to a further component as defined herein. As an example, such a -SH containing moiety may be a cysteine.

Additionally, the lipophilic amino acid component (AA) may contain at least one proline, which may serve as a structure breaker of longer sequences of Leu, Val, Ile, Ala and Met in the lipophilic amino acid component (AA), preferably two, three or more prolines.

Finally, according to a fourth alternative, the amino acid component (AA) may be a weak basic amino acid component (AA). The incorporation of weak basic amino acids or sequences as weak basic amino acid component (AA) into the polymeric carrier of the present invention may serve as a proton sponge and facilitates endosomal escape (also called endosomal release) (proton sponge effect). Incorporation of such a weak basic amino acid component (AA) preferably enhances transfection efficiency.

For this purpose, the amino acids in the weak basic amino acid component (AA) may be selected from either the same or different weak amino acids e.g. selected from histidine or aspartate (aspartic acid).

Additionally, the weak basic amino acid component (AA) may contain or may be flanked by a - SH containing moiety, which allows introducing this component via a disulfide bond as a further part of generic formula (III) above, e.g. as a linker. Such a -SH containing moiety may be any moiety as defined herein suitable to couple one component as defined herein to a further component as defined herein.

Additionally, the weak basic amino acid component (AA) may contain at least one proline, which may serve as a structure breaker of longer sequences of histidine or aspartate (aspartic acid) in the weak basic amino acid component (AA), preferably two, three or more prolines.

According to a fifth alternative, the amino acid component (AA) may be a signal peptide or signal sequence, a localization signal or sequence, a nuclear localization signal or sequence (NLS), an antibody, a cell penetrating peptide, (e.g. TAT), etc. Preferably such an amino acid component (AA) is bound to the polymeric carrier or to another component of the polymeric carrier via a (reversible) disulfide bond. In this context the signal peptide or signal sequence, a localization signal or sequence, a nuclear localization signal or sequence (NLS), an antibody, a cell penetrating peptide, (e.g. TAT), etc.; additionally comprises at least one -SH-moiety. In this context a signal peptide, a localization signal or sequence or a nuclear localization signal or sequence (NLS), may be used to direct the inventive polymeric carrier cargo complex to specific target cells (e.g. hepatocytes or antigen-presenting cells) and preferably allows a translocalization of the polymeric carrier to a specific target, e.g. into the cell, into the nucleus, into the endosomal compartment, sequences for the mitochondrial matrix, localisation sequences for the plasma membrane, localisation sequences for the Golgi apparatus, the nucleus, the cytoplasm and the cytosceleton, etc. Such signal peptide, a localization signal or sequence or a nuclear localization signal may be used for the transport of any of the herein defined nucleic acids, preferably an RNA or a DNA, more preferably an shRNA or a pDNA, e.g. into the nucleus. Without being limited thereto, such a signal peptide, a localization signal or sequence or a nuclear localization signal may comprise, e.g., localisation sequences for the endoplasmic reticulum. Particular localization signals or sequences or a nuclear localization signals may include e.g. KDEL, DDEL, DEEL, QEDL, RDEL, and GQNLSTSN, nuclear localisation sequences, including PKKKRKV, PQKKIKS, QPKKP, RKKR, RKKRRQRRRAHQ, RQARRNRRRRWRERQR, MPLTRRRPAASQALAPPTP, GAALTILV, and GAALTLLG, localisation sequences for the endosomal compartment, including MDDQRDLISNNEQLP, localisation sequences for the mitochondrial matrix, including MLFNLRXXLNNAAFRHGHNFMVRNFRCGQPLX, localisation sequences for the plasma membrane: GCVCSSNP, GQTVTTPL, GQELSQHE, GNSPSYNP, GVSGSKGQ, GQTITTPL, GQTLTTPL, GQIFSRSA, GQIHGLSP, GARASVLS, and GCTLSAEE, localisation sequences for the endoplasmic reticulum and the nucleus, including GAQVSSQK, and GAQLSRNT, localisation sequences for the Golgi apparatus, the nucleus, the cytoplasm and the cytosceleton, including GNAAAAKK, localisation sequences for the cytoplasm and cytosceleton, including GNEASYPL, localisation sequences for the plasma membrane and cytosceleton, including GSSKSKPK, etc. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulins as defined herein, signal sequences of the invariant chain of immunoglobulins or antibodies as defined herein, signal sequences of Lamp1, Tapasin, Erp57, Calreticulin, Calnexin, and further membrane associated proteins or of proteins associated with the endoplasmic reticulum (ER) or the endosomal-lysosomal compartment. Particularly preferably, signal sequences of MHC class I molecule HLA-A*0201 may be used according to the present invention. Such an additional component may be bound e.g. to a cationic polymer or to any other component of the polymeric carrier as defined herein. Preferably this signal peptide, localization signal or sequence or nuclear localization signal or sequence (NLS), is bound to the polymeric carrier or to another component of the polymeric carrier via a (reversible) disulfide bond. For this purpose the (AA) component additionally comprises at least one -SH moiety as defined herein. The binding to any of components of the polymeric carrier may also be accomplished using an acid-labile bond, preferably via a side chain of any of components of the polymeric carrier, which allows to detach or release the additional component at lower pH-values, e.g. at physiological pH-values as defined herein.

Additionally, according to another alternative, the amino acid component (AA) may be a functional peptide or protein, which may modulate the functionality of the polymeric carrier accordingly. Such functional peptides or proteins as the amino acid component (AA) preferably comprise any peptides or proteins as defined herein, e.g. as defined below as therapeutically active proteins. According to one alternative, such further functional peptides or proteins may comprise so called cell penetrating peptides (CPPs) or cationic peptides for transportation. Particularly preferred are CPPs, which induce a pH-mediated conformational change in the endosome and lead to an improved release of the polymeric carrier (in complex with a nucleic acid) from the endosome by insertion into the lipid layer of the liposome. These cell penetrating peptides (CPPs) or cationic peptides for transportation, may include, without being limited thereto protamine, nucleoline, spermine or spermidine, oligo- or poly-L-lysine (PLL), basic polypeptides, oligo or poly-arginine, cell penetrating peptides (CPPs), chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia),* pAntp, pIsl, etc., antimicrobial-derived CPPs e.g. Buforin-2, Bac715-24, SynB, SynB(1), pVEC, hCT-derived peptides, SAP, MAP, KALA, PpTG20, Loligomere, FGF, Lactoferrin, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc. Such an amino acid component (AA) may also be bound to any component of the polymeric carrier as defined herein. Preferably it is bound to the polymeric carrier or to another component of the polymeric carrier via a (reversible) disulfide bond. For the above purpose, the amino acid component (AA) preferably comprises at least one -SH moiety as defined herein. The binding to any of components of the polymeric carrier may also be accomplished using an SH-moiety or an acid-labile bond, preferably via a side chain of any of components of the polymeric carrier which allows to detach or release the additional component at lower pH-values, e.g. at physiological pH-values as defined herein.

According to a last alternative, the amino acid component (AA) may consist of any peptide or protein which can execute any favourable function in the cell. Particularly preferred are peptides or proteins selected from therapeutically active proteins or peptides, from antigens, e.g. tumour antigens, pathogenic antigens (animal antigens, viral antigens, protozoan antigens, bacterial antigens, allergic antigens), autoimmune antigens, or further antigens, from allergens, from antibodies, from immunostimulatory proteins or peptides, from antigen-specific T-cell receptors, or from any other protein or peptide suitable for a specific (therapeutic) application as defined below for coding nucleic acids. Particularly preferred are peptide epitopes from the idiotype antigens (idiotpype immunoglobulins or idiotype T cell receptors) as defined herein.

Due to the peptidic nature of the amino acid component also the definition of peptide, protein, or fragment, variant and derivative thereof applies accordingly and are explicitly encompassed. Furthermore said (AA) components may be prepared by all methods known to a person of ordinary skill or by recombinant peptide or protein production as defined herein or by peptide synthesis as defined herein.

The polymeric carrier may comprise at least one of the above mentioned cationic or polycationic peptides, proteins or polymers or further components, e.g. (AA), wherein any of the above alternatives may be combined with each other, and may be formed by polymerizing same in a polymerization condensation reaction via their -SH-moieties.

According to another embodiment, the polymeric carrier of the polymeric carrier cargo complex or single components thereof, e.g. of the above mentioned cationic or polycationic peptides, proteins or polymers or further components, e.g. (AA), may be further modified with a ligand, preferably a carbohydrate, more preferably a sugar, even more preferably mannose. Preferably this ligand is bound to the polymeric carrier or to a component of the polymeric carrier via a (reversible) disulfide bond or via Michael addition. In the case that the ligand is bound by a disulfide bond the ligand additionally comprises at least one -SH-moiety. These ligands may be used to direct the inventive polymeric carrier cargo complex to specific target cells (e.g. hepatocytes or antigen-presenting cells). In this context mannose is particular preferred as ligand in the case that dendritic cells are the target especially for vaccination or adjuvant purposes.

According to a further embodiment of the invention, the polymeric carrier cargo complex may comprise (AA) components as defined above which do not comprise -SH moieties. These (AA) components can be added before or during the complexation reaction of the at least one nucleic acid molecule. Thereby, the (AA) component(s) is/are (non-covalently) incorporated into the inventive polymeric carrier cargo complex without inclusion of the (AA) component(s) in the polymeric carrier itself by (covalent) polymerization.

According to one specific embodiment, the entire polymeric carrier cargo complex may be formed by a polymerization condensation (of at least one) of the above mentioned cationic or polycationic peptides, proteins or polymers or further components, e.g. (AA), via their -SH- moieties in a first step and complexing the nucleic acid cargo to such a polymeric carrier in a second step. The polymeric carrier may thus contain a number of at least one or even more of the same or different of the above defined cationic or polycationic peptides, proteins or polymers or further components, e.g. (AA), the number preferably determined by the above range. According to one alternative specific embodiment, the polymeric carrier cargo complex is formed by carrying out the polymerization condensation of at least one of the above mentioned cationic or polycationic peptides, proteins or polymers or further components, e.g. (AA), via their -SH- moieties simultaneously to complexing the nucleic acid cargo to the (in situ prepared) polymeric carrier. Likewise, the polymeric carrier may thus also here contain a number of at least one or even more of the same or different of the above defined cationic or polycationic peptides, proteins or polymers or further components, e.g. (AA), the number preferably determined by the above range.

The polymeric carrier cargo complex additionally comprises as a cargo at least one nucleic acid (molecule). In the context of the present invention, such a nucleic acid molecule may be any suitable nucleic acid, selected e.g. from any (single-stranded or double-stranded) DNA, preferably, without being limited thereto, e.g. genomic DNA, single-stranded DNA molecules, double-stranded DNA molecules, coding DNA, DNA primers, DNA probes, immunostimulatory DNA, a (short) DNA oligonucleotide ((short) oligodesoxyribonucleotides), or may be selected e.g. from any PNA (peptide nucleic acid) or may be selected e.g. from any (single-stranded or double-stranded) RNA, preferably, without being limited thereto, a (short) RNA oligonucleotide ((short) oligoribonucleotide), a coding RNA, a messenger RNA (mRNA), an immunostimulatory RNA, a small interfering RNA (siRNA), an antisense RNA, a micro RNA, a small nuclear RNA (snRNA), a small-hairpin (sh) RNA or riboswitches, ribozymes or aptamers; etc. The nucleic acid molecule of the inventive polymeric carrier cargo complex may also be a ribosomal RNA (rRNA), a transfer RNA (tRNA), a messenger RNA (mRNA), or a viral RNA (vRNA). Preferably, the nucleic acid molecule of the polymeric carrier cargo complex is an RNA. More preferably, the nucleic acid molecule of the polymeric carrier cargo complex is a (linear) single-stranded RNA, even more preferably an mRNA or an immunostimulatory RNA. In the context of the present invention, an mRNA is typically an RNA, which is composed of several structural elements, e.g. an optional 5'-CAP structure, an optional 5'-UTR region, an upstream positioned ribosomal binding site followed by a coding region, an optional 3'-UTR region, which may be followed by a poly-A tail (and/or a poly-C-tail). An mRNA may occur as a mono-, di-, or even multicistronic RNA, i.e. a RNA which carries the coding sequences of one, two or more proteins or peptides. Such coding sequences in di-, or even multicistronic mRNA may be separated by at least one IRES sequence, e.g. as defined herein.

Furthermore, the nucleic acid of the polymeric carrier cargo complex may be a single- or a double-stranded nucleic acid (molecule) (which may also be regarded as a nucleic acid (molecule) due to non-covalent association of two single-stranded nucleic acid(s) (molecules)) or a partially double-stranded or partially single stranded nucleic acid, which are at least partially self complementary (both of these partially double-stranded or partially single stranded nucleic acid molecules are typically formed by a longer and a shorter single-stranded nucleic acid molecule or by two single stranded nucleic acid molecules, which are about equal in length, wherein one single-stranded nucleic acid molecule is in part complementary to the other single-stranded nucleic acid molecule and both thus form a double-stranded nucleic acid molecule in this region, i.e. a partially double-stranded or partially single stranded nucleic acid (molecule). Preferably, the nucleic acid (molecule) may be a single-stranded nucleic acid molecule. Furthermore, the nucleic acid (molecule) may be a circular or linear nucleic acid molecule, preferably a linear nucleic acid molecule.

According to one alternative, the nucleic acid molecule of the polymeric carrier cargo complex may be a coding nucleic acid, e.g. a DNA or RNA. Such a coding DNA or RNA may be any DNA or RNA as defined herein. Preferably, such a coding DNA or RNA may be a single- or a double-stranded DNA or RNA, more preferably a single-stranded DNA or RNA, and/or a circular or linear DNA or RNA, more preferably a linear DNA or RNA. Even more preferably, the coding DNA or RNA may be a (linear) single-stranded DNA or RNA. Most preferably, the nucleic acid molecule according to the present invention may be a ((linear) single-stranded) messenger RNA (mRNA). Such an mRNA may occur as a mono-, di-, or even multicistronic RNA, i.e. an RNA which carries the coding sequences of one, two or more proteins or peptides. Such coding sequences in di-, or even multicistronic mRNA may be separated by at least one IRES sequence, e.g. as defined herein.

**Coding nucleic acids:**

The nucleic acid molecule of the polymeric carrier cargo complex may encode a protein or a peptide, which may be selected, without being restricted thereto, e.g. from therapeutically active proteins or peptides, including adjuvant proteins, from antigens, e.g. tumour antigens, pathogenic antigens (e.g. selected, from animal antigens, from viral antigens, from protozoan antigens, from bacterial antigens), allergenic antigens, autoimmune antigens, or further antigens, from allergens, from antibodies, from immunostimulatory proteins or peptides, from antigen-specific T-cell receptors, or from any other protein or peptide suitable for the treatment of lymphoma, wherein the coding nucleic acid may be transported into a cell, a tissue or an organism and the protein may be expressed subsequently in this cell, tissue or organism. In this context the coding nucleic acid may additionally code for a signal peptide as defined herein.

*a) Therapeutically active proteins*

In the context of the present invention, therapeutically active proteins or peptides may be encoded by the nucleic acid molecule of the herein defined polymeric carrier cargo complex. Therapeutically active proteins are defined herein as proteins which have an effect on healing, or treat therapeutically lymphoma, preferably as defined herein. These may be selected from any naturally or synthetically designed occurring recombinant or isolated protein known to a skilled person from the prior art. Without being restricted thereto therapeutically active proteins may comprise proteins, capable of stimulating or inhibiting the signal transduction in the cell, e.g. cytokines, lymphokines, monokines, growth factors, receptors, signal transduction molecules, transcription factors, etc; anticoagulants; antithrombins; antiallergic proteins; apoptotic factors or apoptosis related proteins, therapeutic active enzymes and any protein or peptide connected with any acquired disease or any hereditary disease or favourable for the treatment of any acquired disease or any hereditary disease, particularly any peptide or protein beneficial in the treatment of lymphoma.

A therapeutically active protein, which may be encoded by the nucleic acid molecule of the herein defined polymeric carrier cargo complex, may also be an adjuvant protein. In this context, an adjuvant protein is preferably to be understood as any protein, which is capable to elicit an innate immune response as defined herein. Preferably, such an innate immune response comprises activation of a pattern recognition receptor, such as e.g. a receptor selected from the Toll-like receptor (TLR) family, including e.g. a Toll like receptor selected from human TLR1 to TLR10 or from murine Toll like receptors TLR1 to TLR13. More preferably, the adjuvant protein is selected from human adjuvant proteins or from pathogenic adjuvant proteins, selected from the group consisting of, without being limited thereto, bacterial proteins, protozoan proteins, viral proteins, or fungal proteins, animal proteins, in particular from bacterial adjuvant proteins. In addition, nucleic acids encoding human proteins involved in adjuvant effects (e.g. ligands of pattern recognition receptors, pattern recognition receptors, proteins of the signal transduction pathways, transcription factors or cytokines) may be used as well.

*b) Antigens*

The nucleic acid molecule of the herein defined polymeric carrier cargo complex may alternatively encode an antigen, particularly preferred in this context is an idiotype immunoglobulin or an idiotype T cell receptor as defined herein.

In the context of the present invention, antigens as encoded by the nucleic acid molecule of the herein defined polymeric carrier cargo complex typically comprise any antigen, antigenic epitope or antigenic peptide, falling under the above definition, more preferably protein and peptide antigens, e.g. tumour antigens, allergenic antigens, auto-immune self-antigens, pathogenic antigens, etc. In particular antigens as encoded by the nucleic acid molecule of the herein defined polymeric carrier cargo complex may be antigens generated outside the cell, more typically antigens not derived from the host organism (e.g. a human) itself (i.e. non-self antigens) but rather derived from host cells outside the host organism, e.g. viral antigens, bacterial antigens, fungal antigens, protozoological antigens, animal antigens, allergenic antigens, etc. Allergenic antigens (allergy antigens) are typically antigens, which cause an allergy in a human and may be derived from either a human or other sources. Additionally, antigens as encoded by the nucleic acid molecule of the herein defined polymeric carrier cargo complex may be furthermore antigens generated inside the cell, the tissue or the body. Such antigens include antigens derived from the host organism (e.g. a human) itself, e.g. tumour antigens, self-antigens or auto-antigens, such as auto-immune self-antigens, etc., but also (non-self) antigens as defined herein, which have been originally been derived from host cells outside the host organism, but which are fragmented or degraded inside the body, tissue or cell, e.g. by (protease) degradation, metabolism, etc. Particularly preferred in this context are idiotype antigens selected from idiotype immunoglobulins or idiotype T cell receptors or a fragment, variant and/or derivative thereof as defined herein as encoded by the nucleic acid molecule of the herein defined polymeric carrier cargo complex. In this context an antigen as encoded by the nucleic acid cargo comprised in the polymeric carrier cargo complex is defined as described below for the at least one antigen, the second ingredient of the inventive pharmaceutical composition.

Particularly preferred in this context is, that the antigen (the idiotype immunoglobulin or the idiotype T cell receptor or a fragment, variant and/or derivative thereof) encoded by the nucleic acid cargo is the same antigen as the at least one antigen as defined herein comprised in the inventive pharmaceutical composition as second ingredient (the idiotype immunoglobulin or the idiotype T cell receptor or a fragment, variant and/or derivative thereof). In alternative embodiments however, the antigen encoded by the nucleic acid cargo is a different antigen as the at least one idiotype immunoglobulin or idiotype T cell receptor or a fragment, variant and/or derivative thereof as defined herein as comprised in the inventive pharmaceutical composition as second ingredient. In the specific case that an antigen is encoded by the nucleic acid cargo, the nucleic acid molecule together with the polymeric carrier serves as adjuvant or imunostimulating agent to induce an unspecific innate immune response, whereas the encoded protein or peptide antigen which is expressed by the nucleic acid cargo serves as antigen to induce an antigen-specific adaptive immune response.

c) Antibodies

According to a further alternative, the nucleic acid molecule of the herein defined polymeric carrier cargo complex may encode an antibody or an antibody fragment. According to the present invention, such an antibody may be selected from any antibody, e.g. any recombinantly produced or naturally occurring antibodies, known in the art, in particular antibodies suitable for therapeutic, diagnostic or scientific purposes, or antibodies which have been identified in relation to specific cancer diseases. Herein, the term "antibody" is used in its broadest sense and specifically covers monoclonal and polyclonal antibodies (including agonist, antagonist, and blocking or neutralizing antibodies) and antibody species with polyepitopic specificity. According to the invention, the term "antibody" typically comprises any antibody known in the art (e.g. IgM, IgD, IgG, IgA and IgE antibodies), such as naturally occurring antibodies, antibodies generated by immunization in a host organism, antibodies which were isolated and identified from naturally occurring antibodies or antibodies generated by immunization in a host organism and recombinantly produced by biomolecular methods known in the art, as well as chimeric antibodies, human antibodies, humanized antibodies, bispecific antibodies, intrabodies, i.e. antibodies expressed in cells and optionally localized in specific cell compartments, and fragments and variants of the aforementioned antibodies. In general, an antibody consists of a light chain and a heavy chain both having variable and constant domains. The light chain consists of an N-terminal variable domain, $V_L$, and a C-terminal constant domain, $C_L$. In contrast, the heavy chain of the IgG antibody, for example, is comprised of an N-terminal variable domain, $V_H$, and three constant domains, $C_H1$, $C_H2$ und $C_H3$.

In the context of the present invention, antibodies as encoded by the nucleic acid molecule of the herein defined polymeric carrier cargo complex may preferably comprise full-length antibodies, i.e. antibodies composed of the full heavy and full light chains, as described above. However, derivatives of antibodies such as antibody fragments, variants or adducts may also be encoded by the nucleic acid molecule of the herein defined polymeric carrier cargo complex. Antibody fragments are preferably selected from Fab, Fab', F(ab')$_2$, Fc, Facb, pFc', Fd and Fv fragments of the aforementioned (full-length) antibodies. In general, antibody fragments are known in the art. For example, a Fab ("fragment, antigen binding") fragment is composed of one constant and one variable domain of each of the heavy and the light chain. The two variable domains bind the epitope on specific antigens. The two chains are connected via a disulfide linkage. A scFv ("single chain variable fragment") fragment, for example, typically consists of the variable domains of the light and heavy chains. The domains are linked by an artificial linkage, in general a polypeptide linkage such as a peptide composed of 15-25 glycine, proline and/or serine residues.

In the present context it is preferable that the different chains of the antibody or antibody fragment are encoded by a multicistronic nucleic acid molecule. Alternatively, the different strains of the antibody or antibody fragment are encoded by several monocistronic nucleic acid(s) (sequences).

siRNA:

According to a further alternative, the nucleic acid molecule of the herein defined polymeric carrier cargo complex may be in the form of dsRNA, preferably siRNA. A dsRNA, or a siRNA, is of interest particularly in connection with the phenomenon of RNA interference. The *in vitro* technique of RNA interference (RNAi) is based on double-stranded RNA molecules (dsRNA), which trigger the sequence-specific suppression of gene expression (Zamore (2001) Nat. Struct. Biol. 9: 746-750; Sharp (2001) Genes Dev. 5:485-490: Hannon (2002) Nature 41: 244-251). In the transfection of mammalian cells with long dsRNA, the activation of protein kinase R and RnaseL brings about unspecific effects, such as, for example, an interferon response (Stark et al. (1998) Annu. Rev. Biochem. 67: 227-264; He and Katze (2002) Viral Immunol. 15: 95-119). These unspecific effects are avoided when shorter, for example 21- to 23-mer, so-called siRNA (small interfering RNA), is used, because unspecific effects are not triggered by siRNA that is shorter than 30 bp (Elbashir et al. (2001) Nature 411: 494-498).

The nucleic acid molecule of the herein polymeric carrier cargo complex may thus be a double-stranded RNA (dsRNA) having a length of from 17 to 29, preferably from 19 to 25, and preferably is at least 90%, more preferably 95% and especially 100% (of the nucleotides of a dsRNA) complementary to a section of the nucleic acid molecule of a (therapeutically relevant) protein or antigen described (as active ingredient) hereinbefore or of any further protein as described herein, either a coding or a non-coding section, preferably a coding section. Such a (section of the) nucleic acid molecule may be termed herein a "target sequence" and may be any nucleic acid molecule as defined herein, preferably a genomic DNA, a cDNA, a RNA, e.g. an mRNA, etc. 90% complementary means that with a length of a dsRNA described herein of, for example, 20 nucleotides, the dsRNA contains not more than 2 nucleotides showing no complementarity with the corresponding section of the target sequence. The sequence of the double-stranded RNA used according to the invention is, however, preferably wholly complementary in its general structure with a section of the target sequence. In this context the nucleic acid molecule of the inventive polymeric carrier cargo complex may be a dsRNA having the general structure 5'-$(N_{17-29})$-3', preferably having the general structure 5'-$(N_{19-25})$-3', more preferably having the general structure 5'-$(N_{19-24})$-3', or yet more preferably having the general structure 5'-$(N_{21-23})$-3', wherein for each general structure each N is a (preferably different) nucleotide of a section of the target sequence, preferably being selected from a continuous number of 17 to 29 nucleotides of a section of the target sequence, and being present in the general structure 5'-$(N_{17-29})$-3' in their natural order. In principle, all the sections having a length of from 17 to 29, preferably from 19 to 25, base pairs that occur in the target sequence can serve for preparation of a dsRNA as defined herein. Equally, dsRNAs used as nucleic acid molecule of the inventive polymeric carrier cargo complex can also be directed against nucleotide sequences of a (therapeutically relevant) protein or antigen described (as active ingredient) hereinbefore that do not lie in the coding region, in particular in the 5' non-coding region of the target sequence, for example, therefore, against non-coding regions of the target sequence having a regulatory function. The target sequence of the dsRNA used as nucleic acid molecule of the inventive polymeric carrier cargo complex can therefore lie in the translated and untranslated region of the target sequence and/or in the region of the control elements of a protein or antigen described hereinbefore. The target sequence for a dsRNA used as the nucleic acid molecule of the polymeric carrier cargo complex can also lie in the overlapping region of untranslated and translated sequence; in particular, the target sequence can comprise at least one nucleotide upstream of the start triplet of the coding region, e.g. of a genomic DNA, a cDNA, a RNA, or an mRNA, etc.

**Immunostimulatory nucleic acids:**

a) Immunostimulatory CpG nucleic acids:

According to another alternative, the nucleic acid molecule of the herein defined polymeric carrier cargo complex may be in the form of a(n) (immunostimulatory) CpG nucleic acid, in particular CpG-RNA or CpG-DNA, which preferably induces an innate immune response. A CpG-RNA or CpG-DNA used according to the invention can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid used according to the invention is preferably in the form of CpG-RNA, more preferably in the form of single-stranded CpG-RNA (ss CpG-RNA). Also preferably, such CpG nucleic acids have a length as described above. Preferably the CpG motifs are unmethylated.

b) Immunostimulatory RNA (isRNA):

Likewise, according to a further alternative, the (immunostimulatory) nucleic acid molecule of the polymeric carrier cargo complex may be in the form of an immunostimulatory RNA (isRNA), which preferably elicits an innate immune response. Such an immunostimulatory RNA may be any (double-stranded or single-stranded) RNA, e.g. a coding RNA, as defined herein. Preferably, the immunostimulatory RNA may be a single-stranded, a double-stranded or a partially double-stranded RNA, more preferably a single-stranded RNA, and/or a circular or linear RNA, more preferably a linear RNA. More preferably, the immunostimulatory RNA may be a (linear) single-stranded RNA. Even more preferably, the immunostimulatory RNA may be a (long) (linear) single-stranded) non-coding RNA. In this context it is particular preferred that the isRNA carries a triphosphate at its 5'-end which is the case for *in vitro* transcribed RNA. An immunostimulatory RNA may also occur as a short RNA oligonucleotide as defined herein. An immunostimulatory RNA as used herein may furthermore be selected from any class of RNA molecules, found in nature or being prepared synthetically, and which can induce an innate immune response and may support an adaptive immune response induced by an antigen. In this context, an immune response may occur in various ways. A substantial factor for a suitable (adaptive) immune response is the stimulation of different T-cell sub-populations. T-lymphocytes are typically divided into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of the effector proteins (cytokines) produced by them. Thus, Th1 cells assist the cellular immune response by activation of macrophages and cytotoxic T-cells. Th2 cells, on the other hand, promote the humoral immune response by stimulation of B-cells for conversion into plasma cells and by formation of antibodies (e.g. against antigens). The Th1/Th2 ratio is therefore of great importance in the induction and maintenance of an adaptive immune response. In connection with the present invention, the Th1/Th2 ratio of the (adaptive) immune response is preferably shifted in the direction towards the cellular response (Th1 response) and a cellular immune response is thereby induced. According to one example, the innate immune system which may support an adaptive immune response may be activated by ligands of Toll-like receptors (TLRs). TLRs are a family of highly conserved pattern recognition receptor (PRR) polypeptides that recognize pathogen-associated molecular patterns (PAMPs) and play a critical role in innate immunity in mammals. Currently at least thirteen family members, designated TLR1 - TLR13 (Toll-like receptors: TLR1, TLR2, TLR3, TLR4, TLR5, TLR6, TLR7, TLR8, TLR9, TLR10, TLR11, TLR12 or TLR13), have been identified. Furthermore, a number of specific TLR ligands have been identified. It was e.g. found that unmethylated bacterial DNA and synthetic analogs thereof (CpG DNA) are ligands for TLR9 (Hemmi H et al. (2000) Nature 408:740-5; Bauer S et al. (2001) Proc NatlAcadSci USA 98, 9237-42). Furthermore, it has been reported that ligands for certain TLRs include certain nucleic acid molecules and that certain types of RNA are immunostimulatory in a sequence-independent or sequence-dependent manner, wherein these various immunostimulatory RNAs may e.g. stimulate TLR3, TLR7, or TLR8, or intracellular receptors such as RIG-1, MDA-5, etc. E.g. Lipford *et al.* determined certain G,U-containing oligoribonucleotides as immunostimulatory by acting via TLR7 and TLR8 (see WO 03/086280). The immunostimulatory G,U-containing oligoribonucleotides described by Lipford *et al.* were believed to be derivable from RNA sources including ribosomal RNA, transfer RNA, messenger RNA, and viral RNA.

The immunostimulatory RNA (isRNA) used as the nucleic acid molecule of the herein defined polymeric carrier cargo complex may thus comprise any RNA sequence known to be immunostimulatory, including, without being limited thereto, RNA sequences representing and/or encoding ligands of TLRs, preferably selected from human family members TLR1 - TLR10 or murine family members TLR1 - TLR13, more preferably selected from (human) family members TLR1 - TLR10, even more preferably from TLR7 and TLR8, ligands for intracellular receptors for RNA (such as RIG-I or MDA-5, etc.) (see e.g. Meylan, E., Tschopp, J. (2006). Toll-like receptors and RNA helicases: two parallel ways to trigger antiviral responses. Mol. Cell 22, 561-569), or any other immunostimulatory RNA sequence. Furthermore, (classes of) immunostimulatory RNA molecules, used as the nucleic acid molecule of the polymeric carrier cargo complex may include any other RNA capable of eliciting an innate immune response. Without being limited thereto, such an immunostimulatory RNA may include ribosomal RNA (rRNA), transfer RNA (tRNA), messenger RNA (mRNA), and viral RNA (vRNA). Such an immunostimulatory RNA may comprise a length of 1000 to 5000, of 500 to 5000, of 5 to 5000, or of 5 to 1000, 5 to 500, 5 to 250, of 5 to 100, of 5 to 50 or of 5 to 30 nucleotides.

According to a particularly preferred embodiment, such immunostimulatory nucleic acid sequences is preferably RNA preferably consisting of or comprising a nucleic acid of formula (IV) or (V):

$$G_l X_m G_n, \qquad \text{(formula (IV))}$$

wherein:

G    is guanosine, uracil or an analogue of guanosine or uracil;
X    is guanosine, uracil, adenosine, thymidine, cytosine or an analogue of the above-mentioned nucleotides;
l    is an integer from 1 to 40,
    wherein
    when 1 = 1 G is guanosine or an analogue thereof,
    when 1 > 1 at least 50% of the nucleotides are guanosine or an analogue thereof;
m    is an integer and is at least 3;
    wherein
    when m = 3 X is uracil or an analogue thereof,
    when m > 3 at least 3 successive uracils or analogues of uracil occur;
n    is an integer from 1 to 40,
    wherein
    when n = 1 G is guanosine or an analogue thereof,
    when n > 1 at least 50% of the nucleotides are guanosine or an analogue thereof.

$$C_l X_m C_n, \qquad \text{(formula (V))}$$

wherein:

C    is cytosine, uracil or an analogue of cytosine or uracil;
X    is guanosine, uracil, adenosine, thymidine, cytosine or an analogue of the above-mentioned nucleotides;
l    is an integer from 1 to 40,
    wherein
    when 1 = 1 C is cytosine or an analogue thereof,
    when 1 > 1 at least 50% of the nucleotides are cytosine or an analogue thereof;
m    is an integer and is at least 3;
    wherein
    when m = 3 X is uracil or an analogue thereof,
    when m > 3 at least 3 successive uracils or analogues of uracil occur;

n    is an integer from 1 to 40,
     wherein
     when n = 1 C is cytosine or an analogue thereof,
     when n > 1 at least 50% of the nucleotides are cytosine or an analogue thereof.

The nucleic acids of formula (IV) or (V), which may be used the nucleic acid cargo of the polymeric carrier cargo complex may be relatively short nucleic acid molecules with a typical length of approximately from 5 to 100 (but may also be longer than 100 nucleotides for specific embodiments, e.g. up to 200 nucleotides), from 5 to 90 or from 5 to 80 nucleotides, preferably a length of approximately from 5 to 70, more preferably a length of approximately from 8 to 60 and, more preferably a length of approximately from 15 to 60 nucleotides, more preferably from 20 to 60, most preferably from 30 to 60 nucleotides. If the nucleic acid of the inventive nucleic acid cargo complex has a maximum length of e.g. 100 nucleotides, m will typically be <=98. The number of nucleotides G in the nucleic acid of formula (IV) is determined by l or n. l and n, independently of one another, are each an integer from 1 to 40, wherein when l or n = 1 G is guanosine or an analogue thereof, and when l or n > 1 at least 50% of the nucleotides are guanosine or an analogue thereof. For example, without implying any limitation, when l or n = 4 $G_l$ or $G_n$ can be, for example, a GUGU, GGUU, UGUG, UUGG, GUUG, GGGU, GGUG, GUGG, UGGG or GGGG, etc.; when l or n = 5 $G_l$ or $G_n$ can be, for example, a GGGUU, GGUGU, GUGGU, UGGGU, UGGUG, UGUGG, UUGGG, GUGUG, GGGGU, GGGUG, GGUGG, GUGGG, UGGGG, or GGGGG, etc.; etc. A nucleotide adjacent to $X_m$ in the nucleic acid of formula (IV) according to the invention is preferably not a uracil. Similarly, the number of nucleotides C in the nucleic acid of formula (V) according to the invention is determined by l or n. l and n, independently of one another, are each an integer from 1 to 40, wherein when l or n = 1 C is cytosine or an analogue thereof, and when l or n > 1 at least 50% of the nucleotides are cytosine or an analogue thereof. For example, without implying any limitation, when l or n = 4, $C_l$ or $C_n$ can be, for example, a CUCU, CCUU, UCUC, UUCC, CUUC, CCCU, CCUC, CUCC, UCCC or CCCC, etc.; when l or n = 5 $C_l$ or $C_n$ can be, for example, a CCCUU, CCUCU, CUCCU, UCCCU, UCCUC, UCUCC, UUCCC, CUCUC, CCCCU, CCCUC, CCUCC, CUCCC, UCCCC, or CCCCC, etc.; etc. A nucleotide adjacent to $X_m$ in the nucleic acid of formula (V) according to the invention is preferably not a uracil. Preferably, for formula (IV), when l or n > 1, at least 60%, 70%, 80%, 90% or even 100% of the nucleotides are guanosine or an analogue thereof, as defined above. The remaining nucleotides to 100% (when guanosine constitutes less than 100% of the nucleotides) in the flanking sequences $G_l$ and/or $G_n$ are uracil or an analogue thereof, as defined hereinbefore. Also preferably, l and n, independently of one another, are each an integer from 2 to 30, more preferably an integer from 2 to 20 and yet more preferably an integer from 2 to 15. The lower limit of l or n can be varied if necessary and is at least 1, preferably at least 2, more preferably at least 3, 4, 5, 6, 7, 8, 9 or 10. This definition applies correspondingly to formula (V).

According to a particularly preferred embodiment, a nucleic acid according to any of formulas (IV) or (V) above, which may be used as nucleic acid of the polymeric carrier cargo complex, may be selected from a sequence consisting or comprising any of the following sequences:

- GGUUUUUUUUUUUUUUUGGG (SEQ ID NO: 15);
- GGGGGUUUUUUUUUGGGGG (SEQ ID NO: 16);
- GGGGGUUUUUUUUUUUUUUUUUUUUUUUUUUGGGGG (SEQ ID NO: 17);
- GUGUGUGUGUGUUUUUUUUUUUUUUUGUGUGUGUGU (SEQ ID NO: 18);
- GGUUGGUUGGUUUUUUUUUUUUUUUUGGUUGGUUGGUU (SEQ ID NO: 19);
- GGGGGGGGGUUUGGGGGGGGG (SEQ ID NO: 20);
- GGGGGGGGUUUUGGGGGGGGG (SEQ ID NO: 21);
- GGGGGGGUUUUUUGGGGGGG (SEQ ID NO: 22);
- GGGGGGGUUUUUUGGGGGG (SEQ ID NO: 23);
- GGGGGGUUUUUUUGGGGGG (SEQ ID NO: 24);
- GGGGGGUUUUUUUUGGGGG (SEQ ID NO: 25);
- GGGGGGUUUUUUUUUGGGG (SEQ ID NO: 26);
- GGGGGUUUUUUUUUUUGGGG (SEQ ID NO: 27);
- GGGGGUUUUUUUUUUUUGGG (SEQ ID NO: 28);
- GGGGUUUUUUUUUUUUUGGG (SEQ ID NO: 29);
- GGGGUUUUUUUUUUUUUUGG (SEQ ID NO: 30);
- GGUUUUUUUUUUUUUUUUGG (SEQ ID NO: 31);
- GUUUUUUUUUUUUUUUUUUG (SEQ ID NO: 32);
- GGGGGGGGGUUUGGGGGGGGG (SEQ ID NO: 33);
- GGGGGGGGGUUUUGGGGGGGG (SEQ ID NO: 34);
- GGGGGGGGUUUUUUGGGGGGGG (SEQ ID NO: 35);
- GGGGGGGGUUUUUUGGGGGGGG (SEQ ID NO: 36);
- GGGGGGGUUUUUUUGGGGGGG (SEQ ID NO: 37);

- GGGGGGGUUUUUUUUGGGGGG (SEQ ID NO: 38);
- GGGGGGGUUUUUUUUUGGGGG (SEQ ID NO: 39);
- GGGGGGUUUUUUUUUGGGGG (SEQ ID NO: 40);
- GGGGGGUUUUUUUUUUGGGG (SEQ ID NO: 41);
- GGGGGUUUUUUUUUUUGGGG (SEQ ID NO: 42);
- GGGGGUUUUUUUUUUUUGGG (SEQ ID NO: 43);
- GGGUUUUUUUUUUUUUUUGGG (SEQ ID NO: 44);
- GGUUUUUUUUUUUUUUUUGG (SEQ ID NO: 45);
- GGGGGGGGGGUUUGGGGGGGGGG (SEQ ID NO: 46);
- GGGGGGGGGGUUUUGGGGGGGGG (SEQ ID NO: 47);
- GGGGGGGGGUUUUUGGGGGGGGG (SEQ ID NO: 48);
- GGGGGGGGGUUUUUUGGGGGGGG (SEQ ID NO: 49);
- GGGGGGGGUUUUUUUGGGGGGGG (SEQ ID NO: 50);
- GGGGGGGGUUUUUUUUGGGGGGG (SEQ ID NO: 51);
- GGGGGGGUUUUUUUUUGGGGGG (SEQ ID NO: 52);
- GGGGGGGUUUUUUUUUUGGGGG (SEQ ID NO: 53);
- GGGGGGGUUUUUUUUUUUGGGG (SEQ ID NO: 54);
- GGGGGGUUUUUUUUUUUUGGGG (SEQ ID NO: 55);
- GGGGGGUUUUUUUUUUUUUGGG (SEQ ID NO: 56);
- GGGGUUUUUUUUUUUUUUUGGG (SEQ ID NO: 57);
- GGGUUUUUUUUUUUUUUUUUGGG (SEQ ID NO: 58);
- GUUUUUUUUUUUUUUUUUUUUUUUG (SEQ ID NO: 59);
- GGUUUUUUUUUUUUUUUUUUUUUUUUGG (SEQ ID NO: 60);
- GGGUUUUUUUUUUUUUUUUUUUUUUUUUGGG (SEQ ID NO: 61);
- GGGGUUUUUUUUUUUUUUUUUUUUUUUUUUGGG (SEQ ID NO: 62);
- GGGGGUUUUUUUUUUUUUUUUUUUUUUUUUUUGGGG (SEQ ID NO: 63);
- GGGGGGUUUUUUUUUUUUUUUUUUUUUUUUUUUUGGGG (SEQ ID NO: 64);
- GGGGGGGUUUUUUUUUUUUUUUUUUUUUUUUUUUUUUGGGGG (SEQ ID NO: 65);
- GGGGGGGGUUUUUUUUUUUUUUUUUUUUUUUUUUUUUUGGGGGG (SEQ ID NO: 66);
- GGGGGGGGGUUUUUUUUUUUUUUUUUUUUUUUUUUUUUUUUGGGGGGG (SEQ ID NO: 67);
- GGUUUGG (SEQ ID NO:68);
- GGUUUUGG (SEQ ID NO: 69);
- GGUUUUUGG (SEQ ID NO: 70);
- GGUUUUUUGG (SEQ ID NO: 71);
- GGUUUUUUUGG (SEQ ID NO: 72);
- GGUUUUUUUUGG (SEQ ID NO: 73);
- GGUUUUUUUUUGG (SEQ ID NO: 74);
- GGUUUUUUUUUUGG (SEQ ID NO: 75);
- GGUUUUUUUUUUUGG (SEQ ID NO: 76);
- GGUUUUUUUUUUUUGG (SEQ ID NO: 77);
- GGUUUUUUUUUUUUUGG (SEQ ID NO: 78);
- GGUUUUUUUUUUUUUUGG (SEQ ID NO: 79);
- GGUUUUUUUUUUUUUUUGG (SEQ ID NO: 80);
- GGGUUUGGG (SEQ ID NO: 81);
- GGGUUUUGGG (SEQ ID NO: 82);
- GGGUUUUUGGG (SEQ ID NO: 83);
- GGGUUUUUUGGG (SEQ ID NO: 84);
- GGGUUUUUUUGGG (SEQ ID NO: 85);
- GGGUUUUUUUUGGG (SEQ ID NO: 86);
- GGGUUUUUUUUUGGG (SEQ ID NO: 87);
- GGGUUUUUUUUUUGGG (SEQ ID NO: 88);
- GGGUUUUUUUUUUUGGG (SEQ ID NO: 89);
- GGGUUUUUUUUUUUUGGG (SEQ ID NO: 90);
- GGGUUUUUUUUUUUUUGGG (SEQ ID NO: 91);
- GGGUUUUUUUUUUUUUUGGGUUUUUUUUUUUUUUGGGUUUUUUUUUUU UUUGGG (SEQ ID NO: 92);

- GGGUUUUUUUUUUUUUUUGGGGGGUUUUUUUUUUUUUUUUGGG (SEQ ID NO: 93);
- GGGUUUGGGUUUGGGUUUGGGUUUGGGUUUGGGUUUGGGUUUGGGUUUG GG (SEQ ID NO: 94);

- GGUUUUUUUUUUUUUUUUGGG (short GU-rich, SEQ ID NO: 95)
  or
- CCCUUUUUUUUUUUUUUUUCCCUUUUUUUUUUUUUUUUCCCUUUUUUUUUUUU UUUCCC (SEQ ID NO: 96)

- CCCUUUCCCUUUCCCUUUCCCUUUCCCUUUCCCUUUCCCUUUCCC (SEQ ID NO: 97)
- CCCUUUUUUUUUUUUUUUUCCCCCCUUUUUUUUUUUUUUUUUCCC (SEQ ID NO: 98)

or from a sequence having at least 60%, 70%, 80%, 90%, or even 95% sequence identity with any of these sequences

According to a further particularly preferred embodiment, such immunostimulatory nucleic acid sequences particularly isRNA consist of or comprise a nucleic acid of formula (VI) or (VII):

$$(N_u G_l X_m G_n N_v)_a, \qquad \text{(formula (VI))}$$

wherein:

G is guanosine (guanine), uridine (uracil) or an analogue of guanosine (guanine) or uridine (uracil), preferably guanosine (guanine) or an analogue thereof;

X is guanosine (guanine), uridine (uracil), adenosine (adenine), thymidine (thymine), cytidine (cytosine), or an analogue of these nucleotides (nucleosides), preferably uridine (uracil) or an analogue thereof;

N is a nucleic acid sequence having a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);

a is an integer from 1 to 20, preferably from 1 to 15, most preferably from 1 to 10;

l is an integer from 1 to 40,
wherein when l = 1, G is guanosine (guanine) or an analogue thereof,
when l > 1, at least 50% of these nucleotides (nucleosides) are guanosine (guanine) or an analogue thereof;

m is an integer and is at least 3;
wherein when m = 3, X is uridine (uracil) or an analogue thereof, and
when m > 3, at least 3 successive uridines (uracils) or analogues of uridine (uracil) occur;

n is an integer from 1 to 40,
wherein when n = 1, G is guanosine (guanine) or an analogue thereof,
when n > 1, at least 50% of these nucleotides (nucleosides) are guanosine (guanine) or an analogue thereof;

u,v may be independently from each other an integer from 0 to 50,
preferably wherein when u = 0, v ≥ 1, or
when v = 0, u ≥ 1;

wherein the nucleic acid molecule of formula (VI) has a length of at least 50 nucleotides, preferably of at least 100 nucleotides, more preferably of at least 150 nucleotides, even more preferably of at least 200 nucleotides and most preferably of at least 250 nucleotides.

$$(N_u C_l X_m C_n N_v)_a, \qquad \text{(formula (VII))}$$

wherein:

C is cytidine (cytosine), uridine (uracil) or an analogue of cytidine (cytosine) or uridine (uracil), preferably cytidine (cytosine) or an analogue thereof;

X is guanosine (guanine), uridine (uracil), adenosine (adenine), thymidine (thymine), cytidine (cytosine) or an analogue of the above-mentioned nucleotides (nucleosides), preferably uridine (uracil) or an analogue thereof;

N    is each a nucleic acid sequence having independent from each other a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);

a    is an integer from 1 to 20, preferably from 1 to 15, most preferably from 1 to 10;

l    is an integer from 1 to 40,
     wherein when l = 1, C is cytidine (cytosine) or an analogue thereof,
     when l > 1, at least 50% of these nucleotides (nucleosides) are cytidine (cytosine) or an analogue
     thereof;

m    is an integer and is at least 3;
     wherein when m = 3, X is uridine (uracil) or an analogue thereof,
     when m > 3, at least 3 successive uridines (uracils) or analogues of uridine (uracil) occur;

n    is an integer from 1 to 40,
     wherein when n = 1, C is cytidine (cytosine) or an analogue thereof,
     when n > 1, at least 50% of these nucleotides (nucleosides) are cytidine (cytosine) or an analogue
     thereof.

u, v    may be independently from each other an integer from 0 to 50,
     preferably wherein when u = 0, v $\geq$ 1, or
     when v = 0, u $\geq$ 1;

wherein the nucleic acid molecule of formula (VII) according to the invention has a length of at least 50 nucleotides, preferably of at least 100 nucleotides, more preferably of at least 150 nucleotides, even more preferably of at least 200 nucleotides and most preferably of at least 250 nucleotides.

For formula (VII), any of the definitions given above for elements N (i.e. $N_u$ and $N_v$) and X ($X_m$), particularly the core structure as defined above, as well as for integers a, l, m, n, u and v, similarly apply to elements of formula (VII) correspondingly, wherein in formula (VII) the core structure is defined by $C_l X_m C_n$. The definition of bordering elements $N_u$ and $N_v$ is identical to the definitions given above for $N_u$ and $N_v$.

According to a very particularly preferred embodiment, the nucleic acid molecule according to formula (VI) may be selected from e.g. any of the following sequences:

UAGCGAAGCUCUUGGACCUAGGUUUUUUUUUUUUUUUGGGUGCGUUCCUAGAA

GUACACG (SEQ ID NO: 99)

UAGCGAAGCUCUUGGACCUAGGUUUUUUUUUUUUUUUGGGUGCGUUCCUAGAA

GUACACGAUCGCUUCGAGAACCUGGAUCCAAAAAAAAAAAAAAAACCCACGCAAGGA

UCUUCAUGUGC (SEQ ID NO: 100)

GGGAGAAAGCUCAAGCUUGGAGCAAUGCCCGCACAUUGAGGAAACCGAGUUGCAU

AUCUCAGAGUAUUGGCCCCCGUGUAGGUUAUUCUUGACAGACAGUGGAGCUUAU

UCACUCCCAGGAUCCGAGUCGCAUACUACGGUACUGGUGACAGACCUAGGUCGUC

AGUUGACCAGUCCGCCACUAGACGUGAGUCCGUCAAAGCAGUUAGAUGUUACACU

CUAUUAGAUC (SEQ ID NO: 101)

GGGAGAAAGCUCAAGCUUGGAGCAAUGCCCGCACAUUGAGGAAACCGAGUUGCAU

AUCUCAGAGUAUUGGCCCCCGUGUAGGUUAUUCUUGACAGACAGUGGAGCUUAU

UCACUCCCAGGAUCCGAGUCGCAUACUACGGUACUGGUGACAGACCUAGGUCGUC

AGUUGACCAGUCCGCCACUAGACGUGAGUCCGUCAAAGCAGUUAGAUGUUACACU

CUAUUAGAUCUCGGAUUACAGCUGGAAGGAGCAGGAGUAGUGUUCUUGCUCUAA

GUACCGAGUGUGCCCAAUACCCGAUCAGCUUAUUAACGAACGGCUCCUCCUCUUA

GACUGCAGCGUAAGUGCGGAAUCGGGGAUCAAAUUACUGACUGCCUGGAUUACC

CUCGGACAUAUAACCUUGUAGCACGCUGUUGCUGUAUAGGUGACCAACGCCCACU

CGAGUAGACCAGCUCUCUUAGUCCGGACAAUGAUAGGAGGCGCGGUCAAUCUACU

UCUGGCUAGUUAAGAAUAGGCUGCACCGACCUCUAUAAGUAGCGUGUCCUCUAG

(SEQ ID NO: 102)

GGGAGAAAGCUCAAGCUUGGAGCAAUGCCCGCACAUUGAGGAAACCGAGUUGCAU

AUCUCAGAGUAUUGGCCCCCGUGUAGGUUAUUCUUGACAGACAGUGGAGCUUAU

UCACUCCCAGGAUCCGAGUCGCAUACUACGGUACUGGUGACAGACCUAGGUCGUC

AGUUGACCAGUCCGCCACUAGACGUGAGUCCGUCAAAGCAGUUAGAUGUUACACU

CUAUUAGAUCUCGGAUUACAGCUGGAAGGAGCAGGAGUAGUGUUCUUGCUCUAA

GUACCGAGUGUGCCCAAUACCCGAUCAGCUUAUUAACGAACGGCUCCUCCUCUUA

DACUGCAGCGUAAGUGCGGAAUCGGGGAUCAAAUUACUGACUGCCUGGAUUACC

CUCGGACAUAUAACCUUGUAGCACGCUGUUGCUGUAUAGGUGACCAACGCCCACU

CGAGUAGACCAGCUCUCUUAGUCCGGACAAUGAUAGGAGGCGCGGUCAAUCUACU

UCUGGCUAGUUAAGAAUAGGCUGCACCGACCUCUAUAAGUAGCGUGUCCUCUAGA

GCUACGCAGGUUCGCAAUAAAAGCGUUGAUUAGUGUGCAUAGAACAGACCUCUUA

UUCGGUGAAACGCCAGAAUGCUAAAUUCCAAUAACUCUUCCCAAAACGCGUACGGC

CGAAGACGCGCGCUUAUCUUGUGUACGUUCUCGCACAUGGAAGAAUCAGCGGGCA

UGGUGGUAGGGCAAUAGGGGAGCUGGGUAGCAGCGAAAAAGGGCCCCUGCGCAC

GUAGCUUCGCUGUUCGUCUGAAACAACCCGGCAUCCGUUGUAGCGAUCCCGUUAU

CAGUGUUAUUCUUGUGCGCACUAAGAUUCAUGGUGUAGUCGACAAUAACAGCGUC

UUGGCAGAUUCUGGUCACGUGCCCUAUGCCCGGGCUUGUGCCUCUCAGGUGCACA

GCGAUACUUAAAGCCUUCAAGGUACUCGACGUGGGUACCGAUUCGUGACACUUCC

UAAGAUUAUUCCACUGUGUUAGCCCCGCACCGCCGACCUAAACUGGUCCAAUGUA

UACGCAUUCGCUGAGCGGAUCGAUAAUAAAAGCUUGAAUU (SEQ ID NO: 103)

GGGAGAAAGCUCAAGCUUAUCCAAGUAGGCUGGUCACCUGUACAACGUAGCCGGU
AUUUUUUUUUUUUUUUUUUUUUGACCGUCUCAAGGUCCAAGUUAGUCUGCCUA
UAAAGGUGCGGAUCCACAGCUGAUGAAAGACUUGUGCGGUACGGUUAAUCUCCCC
UUUUUUUUUUUUUUUUUUUUUAGUAAAUGCGUCUACUGAAUCCAGCGAUGAUGC
UGGCCCAGAUC (SEQ ID NO: 104)

GGGAGAAAGCUCAAGCUUAUCCAAGUAGGCUGGUCACCUGUACAACGUAGCCGGU
AUUUUUUUUUUUUUUUUUUUUUGACCGUCUCAAGGUCCAAGUUAGUCUGCCUA
UAAAGGUGCGGAUCCACAGCUGAUGAAAGACUUGUGCGGUACGGUUAAUCUCCCC
UUUUUUUUUUUUUUUUUUUUUAGUAAAUGCGUCUACUGAAUCCAGCGAUGAUGC
UGGCCCAGAUCUUCGACCACAAGUGCAUAUAGUAGUCAUCGAGGGUCGCCUUUUU
UUUUUUUUUUUUUUUUUGGCCCAGUUCUGAGACUUCGCUAGAGACUACAGUUA
CAGCUGCAGUAGUAACCACUGCGGCUAUUGCAGGAAAUCCCGUUCAGGUUUUUUU
UUUUUUUUUUUUUUCCGCUCACUAUGAUUAAGAACCAGGUGGAGUGUCACUGCU
CUCGAGGUCUCACGAGAGCGCUCGAUACAGUCCUGGAAGAAUCUUUUUUUUUU
UUUUUUUUUUUGUGCGACGAUCACAGAGAACUUCUAUUCAUGCAGGUCUGCUC
UA (R 722 SEQ ID NO: 105)

GGGAGAAAGCUCAAGCUUAUCCAAGUAGGCUGGUCACCUGUACAACGUAGCCGGU
AUUUUUUUUUUUUUUUUUUUUUGACCGUCUCAAGGUCCAAGUUAGUCUGCCUA
UAAAGGUGCGGAUCCACAGCUGAUGAAAGACUUGUGCGGUACGGUUAAUCUCCCC

UUUUUUUUUUUUUUUUUUUUUAGUAAAUGCGUCUACUGAAUCCAGCGAUGAUGC
UGGCCCAGAUCUUCGACCACAAGUGCAUAUAGUAGUCAUCGAGGGUCGCCUUUUU
UUUUUUUUUUUUUUUUUUUGGCCCAGUUCUGAGACUUCGCUAGAGACUACAGUUA
CAGCUGCAGUAGUAACCACUGCGGCUAUUGCAGGAAAUCCCGUUCAGGUUUUUUU
UUUUUUUUUUUUUUUCCGCUCACUAUGAUUAAGAACCAGGUGGAGUGUCACUGCU
CUCGAGGUCUCACGAGAGCGCUCGAUACAGUCCUUGGAAGAAUCUUUUUUUUUU
UUUUUUUUUUUUGUGCGACGAUCACAGAGAACUUCUAUUCAUGCAGGUCUGCUC
UAGAACGAACUGACCUGACGCCUGAACUUAUGAGCGUGCGUAUUUUUUUUUUUU
UUUUUUUUUUUCCUCCCAACAAAUGUCGAUCAAUAGCUGGGCUGUUGGAGACGC
GUCAGCAAAUGCCGUGGCUCCAUAGGACGUGUAGACUUCUAUUUUUUUUUUUUUU
UUUUUUUUCCCGGGACCACAAAUAAUAUUCUUGCUUGGUUGGGCGCAAGGGCCCC
GUAUCAGGUCAUAAACGGGUACAUGUUGCACAGGCUCCUUUUUUUUUUUUUUUUU
UUUUUUUCGCUGAGUUAUUCCGGUCUCAAAAGACGGCAGACGUCAGUCGACAACA
CGGUCUAAAGCAGUGCUACAAUCUGCCGUGUUCGUGUUUUUUUUUUUUUUUUUUU
UUGUGAACCUACACGGCGUGCACUGUAGUUCGCAAUUCAUAGGGUACCGGCUCAG
AGUUAUGCCUUGGUUGAAAACUGCCCAGCAUACUUUUUUUUUUUUUUUUUUUUUC
AUAUUCCCAUGCUAAGCAAGGGAUGCCGCGAGUCAUGUUAAGCUUGAAUU (SEQ
ID NO: 106)

According to another very particularly preferred embodiment, the nucleic acid molecule according to formula (VII) may be selected from e.g. any of the following sequences:

UAGCGAAGCUCUUGGACCUACCUUUUUUUUUUUUUUUUCCCUGCGUUCCUAGAAGU
ACACG (SEQ ID NO: 107)

or

UAGCGAAGCUCUUGGACCUACCUUUUUUUUUUUUUUUUCCCUGCGUUCCUAGAAG
UACACGAUCGCUUCGAGAACCUGGAUGGAAAAAAAAAAAAAAAGGGACGCAAGGA
UCUUCAUGUGC (SEQ ID NO: 108)

In a further preferred embodiment the nucleic acid molecule of the herein defined polymeric carrier cargo complex may also occur in the form of a modified nucleic acid.

According to a first embodiment, the nucleic acid molecule of the herein defined polymeric carrier cargo complex may be provided as a "stabilized nucleic acid", preferably as a stabilized RNA or DNA, more preferably as a RNA that is essentially resistant to *in vivo* degradation (e.g. by an exo- or endo-nuclease) as defined herein.

According to another embodiment, the nucleic acid cargo of the herein defined polymeric carrier cargo complex may be modified as defined herein, and/or stabilized, especially if the nucleic acid molecule is in the form of a coding nucleic acid e.g. an mRNA, by modifying the G/C content of the nucleic acid molecule, particularly an mRNA, preferably of the coding region thereof as defined herein.

Nucleic acid molecules used herein as cargo comprised in the polymeric carrier cargo complex as defined herein may be prepared using any method known in the art, including the methods for nucleic acid synthesis as defined herein.

Furthermore, the present invention explicitly encloses variants and fragments of nucleic acid molecules as defined herein comprised as nucleic acid cargo in the polymeric carrier cargo complex.

In the polymeric carrier cargo complex, the cationic component of the polymeric carrier as defined herein and the nucleic acid cargo are typically provided in a molar ratio of about 1 to 10000, preferably in a molar ratio of about 5 to 5000, more preferably in a molar ratio of about 10 to 2500, even more preferably in a molar ratio of about 25 to 2000, and most preferably in a molar ratio of about 25 to 1000 of polymeric carrier to nucleic acid.

Furthermore, in the polymeric carrier cargo complex, the cationic component of the polymeric carrier as defined herein and the nucleic acid cargo are preferably provided in an N/P-ratio of at least 0.1, 0.2, 0.3, 0.4, 0.5, 0.75, 1, 1.5 or 2. Preferably, the N/P- ratio lies within a range of about 0.1, 0.3, 0.4, 0.5, 0.75, 1.0, 1.5 or 2 to 20, preferably in a range of about 0.2 (0.5 or 0.75 or 1.0) to 12, and even more preferably in an N/P-ratio of about 0.4 (0.75 or 1.0) to 10. Most preferably the N/P ratio lies in a ratio between 0.1 and 0.9. In this context, the N/P ratio is a measure of the ionic charge of the cationic (side chain) component of the polymeric carrier or of the polymeric carrier as such. In particular, if the cationic properties of the cationic component are generated by nitrogens (of the amino acid side chains), the N/P ratio expresses the ratio of basic nitrogen atoms to phosphate residues in the nucleotide backbone, considering that (side chain) nitrogen atoms in the cationic component of the polymeric carrier contribute to positive charges and phosphate of the phosphate backbone of the nucleic acid contribute to the negative charge. A formula is given in the Examples. The N/P-ratio is defined as the nitrogen/phosphate ratio (N/P-ratio) of the entire inventive polymeric carrier cargo complex. This is typically illustrative for the content/amount of cationic components, in the polymeric carrier and characteristic for the content/amount of nucleic acids bound or complexed in the inventive polymeric carrier cargo complex. It may be calculated on the basis that, for example, 1 μg RNA typically contains about 3 nmol phosphate residues, provided that RNA exhibits a statistical distribution of bases. Additionally, 1 nmol peptide typically contains about x nmol nitrogen residues, dependent on the molecular weight and the number of its (cationic) amino acids.

In this context it is preferable that in the polymeric carrier cargo complex, the cationic component of the polymeric carrier as defined herein and the nucleic acid cargo are provided in an N/P-ratio of at least about 1 or, preferably, of a range of about 1 to 20 for *in vitro* applications (e.g. in the case cells extracted from the patient would be treated *in vitro* with the inventive pharmaceutical composition and subsequently administered to the patient).

For *in vivo* applications of the inventive pharmaceutical composition an N/P ratio of at least 0.1 (0.2, 0.3, 0.4, 0.5, 0.6), preferably of a range of about 0.1 (0.2, 0.3, 0.4., 0.5, or 0.6) to 1.5 is preferred. Even more preferred is an N/P ratio range of 0.1 or 0.2 to 0.9 or an N/P ratio range of 0.5 to 0.9.

In the specific case that the induction of IFN-α is intended an N/P ratio of at least 0.1 (0.2, 0.3, 0.4, 0.5, or 0.6) or an N/P ratio range of 0.1 to 1 is preferred or more preferred is an N/P ratio range of 0.1 or 0.2 to 0.9 or an N/P ratio range of 0.5 to 0.9. Otherwise if the induction of TNFα would be intended an N/P ratio of 1 to 20 is particularly preferred.

The N/P ratio significantly influences the surface charge of the resulting polymeric carrier cargo complex. Thus it is preferable that the resulting polymeric carrier cargo complex is positively charged for *in vitro* applications and negatively or neutrally charged for *in vivo* applications. The surface charge of the resulting polymeric carrier cargo complex can be indicated as Zetapotential which may be measured by Doppler electrophoresis method using a Zetasizer Nano (Malvern Instruments, Malvern, UK).

The polymeric carrier cargo complex as used in the present invention, such as for use as an adjuvant, is preferably capable of triggering a non-antigen-specific, (innate) immune reaction (as provided by the innate immune system), preferably in an immunostimulating manner. An immune reaction can generally be brought about in various ways. An important factor for a suitable immune response is the stimulation of different T-cell sub-populations. T-lymphocytes typically differentiate into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of effector proteins (cytokines) produced by them. Thus, Th1 cells assist the cellular immune response by activation of macrophages and cytotoxic T-cells. Th2 cells, on the other hand, promote the humoral immune response by stimulation of B-cells for conversion into plasma cells and by formation of antibodies (e.g. against antigens). The Th1/Th2 ratio is therefore of great importance in the immune response. In connection with the present invention, the Th1/Th2 ratio of the immune response is preferably displaced by the immune-stimulating agent, in particular the polymeric carrier cargo complex, in the direction towards the cellular response, that is to say the Th1 response, and a predominantly cellular immune response is thereby induced. As described above, the polymeric carrier cargo complex can induce an unspecific innate immune response, which may allow the support of a specific adaptive immune response elicited by the antigen.

**Determination of the (innate) immunostimulatory or adjuvant capacity of a component in the inventive pharmaceutical composition:**

For the determination of the immunostimulatory capacity of an immunostimulating agent or adjuvant (in particular of a polymeric carrier cargo complex as used in the present invention) several methods are known in the art and may be used. E.g., in vitro methods are advantageous to utilise for compounds as to their capacity to induce cytokines, which are (exclusively or at least typically) part of the innate immune system and thereby (as an additional arm of the immune system) typically improve the induction of an antigen-specific immune response caused by an antigen. For this purpose, e.g. PBMCs may be isolated from blood samples and stimulated with the particular immunostimulating agent or adjuvant. After incubation, secretion of the desired cytokines (e.g. as a reaction of an activation of the PAMP receptors) being typically part of the innate immune system (and not of the antigen-specific immune system) is determined by ELISA. These selected cytokines may be used in the art as determinants of the induction of an innate immune response in the body. In this context, the secretion of TNF-alpha and IFN-alpha is preferably measured to determine the unspecific (innate immune response) evoked by a compound or complex. Especially, IFN-alpha plays an important role in the induction of an unspecific immune response after viral infection and can be used as an indicators of induction of a Th1-shifted adaptive immune response, which is particularly preferred in the context of the treatment of cancer or tumour diseases. Accordingly, it is particularly preferred that the the immunostimulatory compound or complex tested in the screening assay, induces the secretion of e.g. IFN-alpha. Such a compound or complex may then be applied e.g. for the use as an immunotimualting agent (triggering the unspecific (innate) immune response) in vaccination therapies.

IFN-alpha is part of the family of type I interferons. Type I interferons (IFN) are pleiotropic cytokines that are essential for supporting anti-viral immune responses. They induce apoptosis of virus-infected cells and cellular resistance to viral infection, in addition to activating natural killer (NK) and T cells. Type I interferons have effects on a large set of cytokines and chemokines that i.a. influence immunocyte maturation, homing, effector functions and apoptosis. Typically, a major role of IFN-alpha is the induction of a priming state affecting the production and regulation of other mediators, including cytokines. For example, IFN-alpha$\beta$ signaling upregulates IFN-alphay production by dendritic cells (DCs) and T cells and thereby favours the induction and maintenance of Th1 cells. Shifting of an immune response in direction of a Th1 immune response may become particularly important, once protein or peptide vaccines are used, because these vaccines usually induce a Th2-based immune response which consequently prevents or decreases the induction of cytotoxic T cells.

Therefore, it is preferred that a compound or complex to be used as an adjuvant in the context of the present invention may preferably have the property of shifting an antigen-specific immune response caused by a antigen to a Th1-based immune response. The direction of an immune response induced by an antigen is usually measured by determination of the induction of several subtypes of antigen-specific antibodies and the induction of antigen-specific cytotoxic CD8$^+$ T cells. In this context, the subtype antibody IgG1 represents the induction of a Th2-based immune response and the induction of the subtype antibody IgG2a and the induction of cytotoxic T cells represent the induction of a Th1-based immune response. The induction of antigen-specific antibodies is typically determined by measurement of the antibody titer in the blood of the vaccinee by ELISA. The induction of antigen-specific cytotoxic T cells is typically determined by measurement of IFN-gamma secretion in splenocytes after stimulation with antigen-specific peptides by ELISPOT. In this context, the induction of IFN-gamma secretion provides eveidnce that antigen-specific cytotoxic T cells are present in the spleen and which can specifically attack cells that present epitopes of the antigen on MHC I molecules on their surface.

For the determination of beneficial properties of an adjuvant, in vivo vaccinations are typically performed. Therewith, it is possible to investigate if the adjuvant or immunostimulatory compound or complex improves an antigen-specific immune response caused by the vaccine and, furthermore, if it can shift an antigen-specific immune response in the desired direction to display adjuvant properties. Particularly, in the induction of an anti-tumoral immune response the induction of a Th1-shifted immune response, especially the induction of cytotoxic T cells is believed to play a major role, because the induction of antigen-specific cytotoxic T cells are believed to represent an indispensable prerequisite for the successful combat of a tumour.

Accordingly, the methods to screen for, test and/or investigate compound or complexes which exhibit properties as adjuvants are well known in the art and may readily be applied e.g. by ELISA tests measuring the immune response elicited by the tested compounds/complexes.

In another aspect, the present invention relates to a method of preparing a pharmaceutical composition of the invention, said method comprising the steps of: (i) providing at least one polymeric carrier cargo complex as defined anywhere herein; (ii) providing at least one antigen associated with a tumour or cancer disease, wherein said antigen is (an idiotype antigen) selected from: (x) an idiotype immunoglobulin or; (y) an idiotype T cell receptor (or in each case a fragment, variant and/or derivative of said antigen), in each case as defined anywhere herein; and (iii) combining said polymeric carrier cargo complex and said idiotype immunoglobulin or idiotype T cell receptor. The combining step of (iii) may occur briefly before administration to a patient (such as about 1, 5, 15, 30 or 60 minutes prior to, up to 72 hours before, said administration), or may occur during manufacture of said pharmaceutical composition. The respective person of ordinary skill (e.g. a doctor or health professional, or a manufacturer) will be aware of the routine methodologies suitable for such combining step.

In the context of the present invention a method of preparing the polymeric carrier cargo complex as defined herein may comprise the following steps:

a) providing at least one cationic protein or peptide as defined herein and/or at least one cationic or polycationic polymer and optionally at least one amino acid component (AA) as defined herein, each comprising at least one -SH moiety,

b) providing at least one nucleic acid molecule as defined herein, preferably in the above mentioned ratios

c) mixing the components provided in steps a) and b), preferably in a basic or neutral milieu as defined herein, preferably in the presence of oxygen or a further starter as defined herein, preferably at a pH, at a temperature and at time as defined herein, and thereby condensing and thus polymerizing the cationic components provided in step a) with each other via disulfide bonds (in a polymerization condensation or polycondensation) to obtain the polymeric carrier and complexing the nucleic acid molecule provided in step b) with the cationic components provided in step a)

d) optionally purifying the polymeric carrier cargo complex obtained according to step c), preferably using a method as defined herein;

e) optionally lyophilization of the polymeric carrier cargo complex obtained according to step c) or d).

The method of preparing the polymeric carrier cargo complex as described herein may comprise a multi-step condensation polymerization or polycondensation reaction via -SH moieties of the educts e.g. cationic peptides or polymers as defined herein and optionally further amino acid components (AA) in step c). The condensation polymerization or polycondensation reaction which occurs simultaneously to the complexation or electrostratic binding of the nucleic acid molecule preferably leads to the polymeric carrier cargo complex wherein the polymeric carrier is a condensation polymer, wherein the single components are linked by disulfide bonds.

As described herein in a step a) of the method of preparing the polymeric carrier cargo complex, at least one cationic or polycationic protein or peptide as defined herein and/or at least one cationic or polycationic polymer as defined herein are provided, preferably in the ratios indicated above. These components are mixed in step c) with the nucleic acid molecule provided in step b), preferably in a basic or neutral milieu as defined herein, preferably in the presence of oxygen or a further starter as defined herein, preferably at a pH, and at a temperature and at a time as defined herein, and thereby condensing and thus polymerizing these components with each other via disulfide bonds (in a polymerization condensation or polycondensation) to obtain a polymeric carrier complexed to the nucleic acid molecule as defined herein.

According to an alternative, in step a) of the method of preparing the polymeric carrier cargo complex at least one cationic or polycationic protein or peptide and/or at least one cationic or polycationic polymer are provided as defined herein, and optionally at least one amino acid component (AA), are provided in step a) as defined herein, and are used for a polymerization condensation or polycondensation and complexation reaction prior to adding the nucleic acid of step b) but using the same polymerization conditions outlined for step c). The polymerized polymeric carrier and the nucleic acid of step b) are then mixed in step c). Preferably, the components are all provided in the ratios indicated above and mixed, preferably in a basic or neutral milieu as defined herein, preferably in the presence of oxygen or a further starter as defined herein, preferably at a pH, at a temperature and at time as defined herein. Upon mixing and starting the reaction, the components are condensed and thus polymerized with each other via disulfide bonds (in a polymerization condensation or polycondensation) to obtain a polymeric carrier complexed to the nucleic acid molecule as defined herein.

In both of the above alternatives, different polymeric carriers, particularly different peptides and/or different polymers, and may be selected in the condensation polymerization as indicated above. In this context, the selection of different component(s) of the polymeric carrier is typically dependent upon the desired properties of the final polymeric carrier and the desired cationic strength of the final polymeric carrier. Accordingly, the content of cationic components, may furthermore be "diluted" or modified in the above alternative of step a) e.g. by introducing an amino acid component (AA) as defined herein, preferably in the above defined ratios. Thereby, a modified polymeric carrier may be obtained, wherein the cationic character of the unmodified polymeric carrier typically remains in the limitations as defined herein. The properties of the final polymeric carrier may thus be adjusted as desired with properties of components (AA) by inserting amino acid component (AA) as defined herein in steps a).

In step c), the at least one cationic or polycationic protein or peptide as defined herein and/or at least one cationic or polycationic polymer as defined herein, and optionally at least one amino acid component (AA) and the at least one nucleic acid as defined herein, are preferably contained in a basic or neutral milieu in the step a) of the inventive method of preparing the inventive polymeric carrier cargo complex. Such a basic or neutral milieu typically exhibits a pH range of about 5 to about 10, preferably a pH range of about 6 to about 9, more preferably a pH range of about 7 to about 8, e.g. about 6.5, 7, 7.5, 8, 8.5, or 9 or any range selected from any two of these or the aforementioned values.

Furthermore, the temperature of the solution in step c) is preferably in a range of about 5°C to about 60°C, more preferably in a range of about 15°C to about 40°C, even more preferably in a range of about 20°C to about 30°C, and most preferably in a range of about 20°C to about 25°C, e.g. about 25°C.

In step c) of the method of preparing the polymeric carrier cargo complex as described herein buffers may be used as suitable. Preferred buffers may comprise, but are not limited to carbonate buffers, borate buffers, Bicine buffer, CHES buffer, CAPS buffer, Ethanolamine containing buffers, HEPES, MOPS buffer, Phosphate buffer, PIPES buffer, Tris buffer, Tricine buffer, TAPS buffer, and/or TES buffer as buffering agents. Particularly preferred is a carbonate buffer.

Upon mixing the components, preferably in the presence of oxygen, preferably in the presence of a basic or neutral milieu as defined herein, the condensation polymerization or polycondensation reaction and the complexation of the at least one nucleic acid molecule is started. For this purpose, the mixture in step c) is preferably exposed to oxygen or may be started using a further starter, e.g. a catalytic amount of an oxidizing agent, e.g. DMSO, etc. Upon start of the condensation polymerization or polycondensation reaction of the at least one cationic or polycationic protein or peptide and/or at least one cationic or polycationic polymer and optionally at least one amino acid component (AA) as defined herein, are condensed and thus polymerized with each other via disulfide bonds (polymerization condensation or polycondensation). In this reaction step a) preferably linear polymers are created using monomers with at least one reactive -SH moiety, i.e. at least one cationic or polycationic protein or peptide and/or at least one cationic or polycationic polymer and optionally at least one amino acid component (AA) as defined herein, each component exhibiting at least one free -SH-moieties as defined herein, e.g. at their terminal ends. However, components with more than one, preferably two free -SH-moieties may be used, which may lead to branched polymers. Simultaneously to the polymerization reaction the cationic polymers bind to the at least one nucleic acid molecule and thereby complexing it.

According to one alternative, the polymeric carrier cargo complex additionally may be modified with a component (AA) as defined herein.

According to a first example, a component (AA) (e.g. a ligand) is attached to the cationic component prior to providing the cationic component in step a) via any functionality as defined herein, e.g. a -SH moiety. This component (AA) or (e.g. a ligand) is preferably attached to the cationic component at one terminus of these components. If the attachment is carried out via -SH bonds, the cationic components are preferably provided with two (or even more) -SH-moieties. The component (AA) or (e.g. a ligand) preferably carries only one -SH moiety. In this case, one - SH moiety of the cationic component is preferably protected in a first step using a protecting group as known in the art. Then, the cationic component may be bound to a component L to form a first disulfide bond via the non-protected -SH moiety. The protected -SH-moiety of the cationic component is then typically deprotected for further reactions.

Alternatively, the above mentioned component (AA) or (e.g. a ligand) may be used in step c) to be coupled with the cationic components provided in step a) above, e.g. via disulfide bonds without blocking the free -SH moieties. But in this context all methods known to a skilled person or defined herein may be used to attach the component (AA) to the cationic component or to the polymeric carrier.

Alternatively, a component (AA) or (e.g. a ligand) can be bound to the polymeric carrier cargo complex after step c) via any functionality as defined herein, e.g. a -SH moiety. In this context it is preferable that the component (AA) (e.g. a ligand) is bound via free -SH moieties of the polymeric carrier components.

According to step c) of the method of preparing the polymeric carrier cargo complex as described herein, at least one nucleic acid molecule as defined herein is mixed with the cationic components provided in step b), preferably in the above mentioned ratios. Typically, in the polymeric carrier cargo complex, the cationic components as defined herein, and the at least one nucleic acid molecule are provided in a molar ratio of about 5 to 10000, preferably in a molar ratio of about 5 to 5000, more preferably in a molar ratio of about 10 to 2500, even more preferably in a molar ratio of about 10 to 1000 cationic polymer to nucleic acid. The N/P ratios are preferably as indicated above. In this context it is particularly preferred that the N/P ratios are selected thereby avoiding agglomeration and toxicity *in vivo.*

In a specific embodiment, (AA) components as defined above which do not comprise -SH moieties can be added in step c) which are thereby incorporated into the polymeric carrier cargo complex without polymerization by (terminal) -SH moieties. Thereby these (AA) components are typically not covalently linked and included non-covalently in the complex as a further component. Particularly preferred in this context is the incorporation of the at least idiotype immunoglobulin or idiotype T cell receptor or a fragment, variant and/or derivative provided as protein or peptide in the polymeric carrier cargo complex as a (AA) component.

According to a further step d) of the method of preparing the polymeric carrier cargo complex as described herein, the polymeric carrier cargo complex obtained according to step c) is optionally purified. Purification may occur by using chromatographic methods, such as HPLC, FPLC, GPS, dialysis, etc.

According to a further step e) of the method of preparing the polymeric carrier cargo complex as described herein, the polymeric carrier cargo complex obtained according to step c) or d) is optionally lyophilized. For this purpose any suitable cryoprotectant or lyoprotectant may be added to the polymeric carrier cargo complex obtained in step c) or d).

The method of preparing the polymeric carrier cargo complex as described herein is particularly suitable to adapt the chemical properties of the desired polymeric carrier cargo complex due to specific selection of its components of the polymeric carrier thereby avoiding agglomeration and toxicity *in vivo.*

As a second ingredient the inventive pharmaceutical composition comprises at least one antigen associated with cancer or tumour disease, wherein said antigen is (e.g. an idiotype antigen) selected from: (x) an idiotype immunoglobulin; or (y) an idiotype T cell receptor, or in each case a fragment, variant and/or derivative of said antigen.

In other embodiments the preferably human, (idiotype) antigen is associated with lymphoma, most preferably a lymphoma selected from the list consisting of: B-cell lymphoma, T-cell lymphoma and non-hodgkin's lymphoma; and/or may be an (idiotype) antigen derived from a malignant cell, preferably derived from a malignant B or T cell. For example, such antigen may have been extracted, fractioned, isolated and/purified from tumour cells, or may be produced following cloning of nucleic acid encoding said antigen from said tumour cells. Such methods are known to the person of ordinary skill, and/or include those methods described herein.

This at least one idiotype immunoglobulin (e.g. an antibody or a B-cell receptor) or at least one idiotype T cell receptor or a fragment, variant and/or derivative thereof can be provided as protein or peptide; can be represented as a nucleic acid coding for the at least one idiotype or at least one idiotype T cell receptor or a fragment, variant and/or derivative thereof; or can be provided as antigenic cells, antigenic cellular fragments, or cellular fractions comprising the at least one idiotype or the at least one idiotype T cell receptor or a fragment, variant and/or derivative thereof.

Preferably, the at least one (idiotype) antigen that is an idiotype immunoglobulin, an idiotype T cell receptor or a fragment, variant and/or derivative of said antigen, is or is provided as, a protein or peptide (eg a protein or peptide antigen).

In connection with all aspects of the present invention, the at least one idiotype immunoglobulin (eg, that comprised in the inventive pharmaceutical composition) may be selected from an idiotype antibody or an idiotype B cell receptor (both typically considered as immunoglobulins) (or a fragment, variant and/or derivative thereof) Such idiotype immunoglobulin may be one expressed on or by, or purified, isolated or otherwise derived from, a B cell of a patient suffering from B cell lymphoma. For example, such B-cell may be a malignant B-cell.

In this context, immunoglobulins typically comprise a light chain and a heavy chain, both of which have variable and constant domains. The light chain comprises the N-terminal variable domain $V_L$ and the C-terminal constant domain $C_L$. The heavy chain of an IgG antibody, in contrast, can be divided into an N-terminal variable domain $V_H$ and three constant domains $C_H1$, $C_H2$ and $C_H3$.

Immunoglobulins in the context of the present invention are typically monoclonal antibodies (or B cell receptors) which are therefore typically antibodies which are specific for a particular antigen or epitope, i.e. bind this antigen or epitope with a high affinity. Furthermore, the immunoglobulins must not consist of the full-length antibody or B cell receptor but can be provided as antibody fragments known to a person skilled in the art, e.g. Fab, Fab', $F(ab')_2$, Fc, Facb, pFc', Fd, und Fv fragments of the above mentioned antibodies etc. Protein fragments consisting of the minimal binding subunit of antibodies known as single-chain antibodies (scFvs) can also be used in the context of the present invention, as well as variants and/or derivates thereof.

In this context, a Fab (fragment antigen binding) fragment typically comprises the variable and a constant domain of a light and a heavy chain, e.g. the $C_H1$ and the $V_H$ domain of the heavy chain and the complete light chain. The two chains are bonded to one another via a disulfide bridge. An Fab fragment thus conventionally contains the complete antigen-binding region of the original antibody and usually has the same affinity for the antigen, the immunogen or an epitope of a protein.

Furthermore in this context it is particularly preferred that the idiotype immunoglobulin included in the inventive pharmaceutical composition comprises or consists of the variable domains of the idiotype antibody or idiotype B cell receptor.

In the context of the present invention idiotype T cell receptors of a (e.g. malignant) T cell means the T cell receptor displayed by the (e.g. malignant) T cell of a patient, such as one suffering from T cell lymphoma.

In contrast to immunoglobulins, which interact with pathogens or antigens in the extracellular space of cells, T cells only recognize antigens that are displayed on the surfaces of the body's own cells. T cells can detect the presence of an antigen by their T cell receptor because fragments of the antigen are displayed on the cell by major histocompatibility complex (MHC) molecules. Most of the T cell receptors consist of two different polypeptide chains, termed alpha and beta chain. These $\alpha{:}\beta$ heterodimers are very similar in structure to the Fab fragment of immunoglobulins. Both chains of the T cell receptor have an amino-terminal variable region with homology to an immunoglobulin V domain. But in contrast to immunoglobulins T cell receptors do not recognize the native antigen but a MHC bounded peptide (T cell epitope) of the antigen. Nevertheless, also this binding is specific for the displayed antigen-derived epitope.

As described above for immunoglobulins also in the case of T cell receptors, also fragments, variants and/or derivatives thereof, particularly the variable domains of the T cell receptors can be provided in the inventive pharmaceutical composition as antigen.

Immunoglobulins or T cell receptors or a fragment, variant and/or derivative thereof, as described above, can be prepared using recombinant production, such as those described herein, or e.g. with the aid of molecular biology methods known to a person of ordinary skill. Such an (idiotype) antigen can be described, as applicable, as a "recombinant protein antigen" and/or a "recombinant peptide antigen.

For production of the recombinant idiotypic vaccine obtaining the sequence of the idiotype that is specifically and uniquely found on the surface of the patient's (e.g. malignant) B or T cells is obtained. The preferred method is the RNA isolation from (e.g. tumour) cells" subsequently followed by synthesis of cDNA and specific amplification of sequences coding for immunoglobulins or T cell receptors. This is followed by subcloning and sequencing of a library specific for immunoglobulins or T cell receptors. The DNA sequence of the particular immunoglobulin or T cell receptor of the malignant B or T cell is determined by e.g. statistical analysis of a sequence library. The original protocols for cloning and determination of idiotype immunoglobulin genes from biopsy tissues were described in numerous publications (e.g. Osterroth et al., 1999, J. Immunol Methods., 229:141-153; McCormick et al., 2003, J. Immunol Methods., 278: 95-104). Furthermore, the improved procedure for identifying and cloning variable regions of immunoglobulins is described in WO2010040531. Idiotypes can be expressed in suitable host cells, including plants as scFv (McCormick et al., 2003, J. Immunol Methods., 278:95-104) or as full-length immunoglobulin (Bendandi et al., 2010, Ann. Oncol., 21: 2420-2427). In this context, the DNA sequences which code for the idiotype immunoglobulin or the idiotype T cell receptor (or a fragment, variant and/or derivative thereof) are cloned into a specific expression vector. The immunoglobulins or T cell receptors can then be expressed e.g. in suitable host cells. Suitable host cells in connection with the present invention include, inter alia, *E. coli,* yeasts, hamster, insect, plant and mammalian cells. Particularly promising is the rapid manufacturing of idiotype antigens in plants using transient expression. Accordingly, in certain embodiments of all aspects of the present invention, the idiotype immunoglobulins or idiotype T cell receptors (or a fragment, variant and/or derivative thereof) is derived or produced using transgenic plants or plant cells.

Particularly in this context, the transient, recombinant expression of the idiotype immunoglobulins or idiotype T cell receptors or a fragment, variant and/or derivative thereof in plants (e.g. tobacco) is preferred.

A preferred method for transient expression of recombinant proteins in plants is the magnICON system i.e. by vacuum-infiltration of plants using suitable *Agrobacterium* strains harbouring and transferring into the plant the expression cassette based on plant viral vectors. This system is broadly used for the expression of recombinant proteins (Marillonnet et al., 2005, Nat. Biotechnol., 23:718-723; Santi et al., 2006, Proc.Natl. Acad. Sci. USA, 103:861-866; Webster et al., 2009, Plant Biotechnol. J., 7:846-855; Kalthoff et al., 2010, J. Virol., 84:12002-12010; for review see: Gleba et al., 2005, Vaccine, 23:2042-2048; Gleba et al., 2007, Curr. Opin. Biotechnol., 18:134-141;) and is also suitable for the production of hetero-oligomeric proteins including recombinant immunoglobulins in plant leaves (Giritch et al., 2006, Proc. Natl. Acad. Sci. USA., 103:14701-14706; Bendandi et al., 2010, Ann. Oncol., 21:2420-2427; Castilho et al., 2011, PLoS One, 6:e26040). Accordingly, in another embodiments of all aspects of the present invention, the said recombinant protein or peptide (idiotype) antigen is derived or produced using vacuum-infiltration of tobacco plants using *Agrobacterium* strains comprising and transferring into said plant at least one expression cassette encoding for said protein or peptide antigen, where said expression cassette is based on at least one plant virus vector.

Alternatively, immunoglobulins, as well as T cell receptors, may be expressed by (ie derived and/or produced) a hybridoma cell. For the preparation of such idiotype immunoglobulins, the (eg malign) B lymphocytes are then conventionally selectively purified from the spleen or other organs or fluids (e.g. blood) suitable for this from the patient, and such B lymphocytes are fused with an immortal myeloma cell to give the so-called hybridoma cell (using techniques known to the person or ordinary skill). After selection methods and cloning of the hybridomas or hybridoma cells formed, those clones which secernate, i.e. express and secrete, antibodies of the desired specificity can be determined. Accordingly, in another embodiments of all aspects of the present invention, the (idiotype) antigen is derived or produced using a hybridoma cell Immunoglobulins or T cell receptors or a fragment, variant and/or derivative thereof, as described above can be prepared using peptide synthesis methods such as those described herein, or e.g. with other methodologies known to the person of ordinary skill. Such an (idiotype) antigen can be described, as applicable, as a "synthetic protein antigen" and/or a "synthetic peptide antigen.

In case that the at least idiotype immunoglobulin or T cell receptor (or a fragment, variant and/or derivative thereof) is provided as protein or peptide antigen, the peptide or protein antigen can be provided in a fist alternative in a separate component of the pharmaceutical composition. In this case the at least one protein or peptide antigen is not part of the polymeric carrier cargo complex or in other words: in this case the polymeric carrier cargo complex does not include the at least one idiotype immunoglobulin or T cell receptor (or a fragment, variant and/or derivative thereof). In a second alternative the at least one idiotype immunoglobulin or T cell receptor (or a fragment, variant and/or derivative thereof) can be provided as component of the polymeric carrier cargo complex. In this case the peptide or protein antigen can be added to the polymeric carrier cargo complex during the polymerization step c) of the method of preparing of the polymeric carrier cargo complex. Thus, the peptide or protein antigen is integrated in the polymeric carrier cargo complex. Particularly preferred in this context is that the peptide or protein antigen bears at least one SH-moiety for polymerization with the other components of the polymeric carrier in the polymeric carrier cargo complex. Furthermore, in a further alternative the at least one idiotype immunoglobulin or T cell receptor (or a fragment, variant and/or derivative thereof) if provided as a protein or peptide antigen is provided as component of the polymeric carrier of the polymeric carrier cargo complex and at least one additional idiotype immunoglobulin or T cell receptor (or a fragment, variant and/or derivative thereof) provided as a protein or peptide antigen (the same or a different) is provided in a separate component of the inventive pharmaceutical composition which is not part of the polymeric carrier cargo complex.

Additionally, the at least one idiotype immunoglobulin or T cell receptor (or a fragment, variant and/or derivative thereof) can be represented in the inventive pharmaceutical composition in the form of nucleic acids coding for the at least one idiotype immunoglobulin or T cell receptor (or a fragment, variant and/or derivative thereof). In this context the nucleic acids coding for the at least one idiotype immunoglobulin or T cell receptor (or a fragment, variant and/or derivative thereof) are defined as disclosed above for the nucleic acid cargo comprised in the polymeric carrier cargo complex used as an adjuvant in the inventive pharmaceutical composition. Therefore also fragments, variants, derivatives and modifications of a nucleic acid as described herein are explicitly encompassed.

In this context the at least one idiotype immunoglobulin or T cell receptor (or a fragment, variant and/or derivative thereof) if represented in the inventive pharmaceutical composition in the form of nucleic acids coding for the at least one idiotype immunoglobulin or T cell receptor (or a fragment, variant and/or derivative thereof), can be prepared with all methods for nucleic acid synthesis known for a skilled person. Particularly preferred are methods for nucleic acid synthesis as described herein.

Also in this case two alternatives exist. The first alternative provides the nucleic acid coding for the at least one idiotype immunoglobulin or T cell receptor (or a fragment, variant and/or derivative thereof) as part of the polymeric carrier cargo complex (e.g. as nucleic acid cargo molecule) and the second alternative provides the nucleic acid coding for the at least one idiotype immunoglobulin or T cell receptor (or a fragment, variant and/or derivative thereof) as separate component of the inventive pharmaceutical composition. Thus, in this case the nucleic acid coding for the at least one idiotype immunoglobulin or T cell receptor (or a fragment, variant and/or derivative thereof) is not part of the polymeric carrier cargo complex.

In a further embodiment of the present invention the at least one idiotype immunoglobulin or T cell receptor (or a fragment, variant and/or derivative thereof) coded by a nucleic acid can be provided as part of the (adjuvant) polymeric carrier cargo complex (e.g. as nucleic acid cargo coding for the at least one idiotype immunoglobulin or T cell receptor (or a fragment, variant and/or derivative thereof)) and additionally at least one idiotype immunoglobulin or T cell receptor (or a fragment, variant and/or derivative thereof) coded by a nucleic acid can be provided in a separate component which is not part of the polymeric carrier cargo complex.

The invention further provides the alternative that at least one idiotype immunoglobulin or T cell receptor (or a fragment, variant and/or derivative thereof) is provided as a nucleic acid (as part of the polymeric carrier cargo complex or not) and that additionally at least one idiotype immunoglobulin or T cell receptor (or a fragment, variant and/or derivative thereof) is provided as protein or peptide antigen (as part of the polymeric carrier cargo complex or not).

Furthermore, the inventive pharmaceutical composition may comprise a pharmaceutically acceptable carrier and/or vehicle. In the context of the present invention, a pharmaceutically acceptable carrier typically includes the liquid or non-liquid basis of the inventive pharmaceutical composition. If the inventive pharmaceutical composition is provided in liquid form, the carrier will typically be pyrogen-free water; isotonic saline or buffered (aqueous) solutions, e.g phosphate, citrate etc. buffered solutions. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds may be used as well for the inventive pharmaceutical composition, which are suitable for administration to a patient to be treated. The term "compatible" as used here means that these constituents of the inventive pharmaceutical composition are capable of being mixed with the inventive polymeric carrier cargo complex as defined herein in such a manner that no interaction occurs which would substantially reduce the pharmaceutical effectiveness of the inventive pharmaceutical composition under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

Furthermore as an additional ingredient the inventive pharmaceutical composition may comprise at least one additional pharmaceutically active component. A pharmaceutically active component in this connection may be a compound that has a therapeutic effect to heal or ameliorate particular indication, preferably tumour or cancer diseases such as lymphoma. Such compounds include, without implying any limitation, peptides or proteins, preferably as defined herein, nucleic acids, preferably as defined herein, (therapeutically active) low molecular weight organic or inorganic compounds (molecular weight less than 5000, preferably less than 1000), sugars, antigens or antibodies, preferably as defined herein, therapeutic agents already known in the prior art, antigenic cells, antigenic cellular fragments, cellular fractions; cell wall components (e.g. polysaccharides), modified, attenuated or de-activated (e.g. chemically or by irradiation) pathogens (virus, bacteria etc.), adjuvants, preferably as defined herein, etc. For example, a pharmaceutically active component in this connection may be an anti-CD20 antibody such s rituximab.

According to a specific embodiment, the inventive pharmaceutical composition may comprise an (additional) adjuvant. In this context, an adjuvant may be understood as any compound, which is suitable to initiate or increase an immune response of the innate immune system, i.e. a non-specific immune response. With other words, when administered, the inventive pharmaceutical composition typically elicits an innate immune response due to the adjuvant, optionally contained therein. Such an adjuvant may be selected from any adjuvant known to a skilled person and suitable for the present case, i.e. supporting the induction of an innate immune response in a mammal.

The inventive pharmaceutical composition (the inventive pharmaceutical composition) can additionally contain one or more auxiliary substances in order to increase its immunogenicity or immunostimulatory capacity, if desired. A synergistic action of the polymeric carrier cargo complex as defined herein and of an auxiliary substance, which may be optionally contained in the inventive pharmaceutical composition, as defined herein, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, INF-alpha, IFN-beta, INF-gamma, GM-CSF, G-CSF, M-CSF, LT-beta or TNF-alpha, growth factors, such as hGH.

Further additives which may be included in the inventive pharmaceutical composition (the inventive pharmaceutical composition) are emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives.

The inventive pharmaceutical composition (the inventive pharmaceutical composition) can also additionally contain any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

The inventive pharmaceutical composition (the inventive pharmaceutical composition) can also additionally or alternatively contain an immunostimulatory RNA, i.e. an RNA derived from an immunostimulatory RNA, which triggers or increases an (innate) immune response. Preferably, such an immunostimulatory RNA may be in general be as defined hereinbefore.

Another class of compounds, which may be added to the inventive pharmaceutical composition (the inventive pharmaceutical composition) in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

The inventive pharmaceutical composition may be administered orally, parenterally, by inhalation spray, topically, rectally, nasally, buccally, vaginally or via an implanted reservoir. The term parenteral as used herein includes subcutaneous, intravenous, intramuscular, intra-articular, intra-nodal, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Preferably, the inventive pharmaceutical composition may be administered by parenteral injection, more preferably by subcutaneous, intravenous, intramuscular, intra-articular, intra-nodal, intra-synovial, intrasternal, intrathecal, intrahepatic, intralesional, intracranial, transdermal, intradermal, intrapulmonal, intraperitoneal, intracardial, intraarterial, and sublingual injection or via infusion techniques. Particularly preferred is intradermal, subcutaneous and intramuscular injection. Sterile injectable forms of the inventive pharmaceutical compositions may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation of the inventive pharmaceutical composition.

The inventive pharmaceutical composition as defined herein may also be administered orally in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient, i.e. the inventive polymeric carrier cargo complex, is combined with emulsifying and suspending agents. If desired, certain sweetening, flavoring or coloring agents may also be added.

The inventive pharmaceutical composition may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the inventive pharmaceutical composition may be formulated in a suitable ointment, containing the inventive polymeric carrier cargo complex suspended or dissolved in one or more carriers. Carriers for topical administration include, but are not limited to, mineral oil, liquid petrolatum, white petrolatum, propylene glycol, polyoxyethylene, polyoxypropylene compound, emulsifying wax and water. Alternatively, the inventive pharmaceutical composition can be formulated in a suitable lotion or cream. In the context of the present invention, suitable carriers include, but are not limited to, mineral oil, sorbitan monostearate, polysorbate 60, cetyl esters wax, cetearyl alcohol, 2-octyldodecanol, benzyl alcohol and water.

The inventive pharmaceutical composition typically comprises a "safe and effective amount" of the idiotype immunoglobulin or idiotype T cell receptor (or fragments, variants and/or derivative thereof) and the polymeric carrier cargo complex as defined herein. As used herein, a "safe and effective amount" means an amount of the idiotype immunoglobulin or idiotype T cell receptor and the polymeric carrier cargo complex that is sufficient to significantly induce a positive modification of the particular condition, such as cancer or tumour disease e.g. lymphoma. At the same time, however, a "safe and effective amount" is small enough to avoid serious side-effects and to permit a sensible relationship between advantage and risk. The determination of these limits typically lies within the scope of sensible medical judgment. A "safe and effective amount" of the components of the inventive pharmaceutical composition, particularly of the inventive pharmaceutical composition as defined herein, will furthermore vary in connection with the particular condition to be treated and also with the age and physical condition of the patient to be treated, the body weight, general health, sex, diet, time of administration, rate of excretion, drug combination, the activity of the inventive pharmaceutical composition, the severity of the lymphoma condition, the duration of the treatment, the nature of the accompanying therapy, of the particular pharmaceutically acceptable carrier used, and similar factors, within the knowledge and experience of the accompanying doctor. The inventive pharmaceutical composition may be used for human and/or for veterinary medical purposes, preferably for human medical purposes, as a pharmaceutical composition.

In the context of the present invention the nucleic acid cargo in the polymeric carrier cargo complex comprised in the inventive pharmaceutical composition is preferably as defined above. More preferably, the nucleic acid of the polymeric carrier cargo complex, preferably contained in the pharmaceutical composition, is typically an immunostimulatory nucleic acid as defined herein, e.g. a CpG-DNA or an immunostimulatory RNA (isRNA). Alternatively or additionally, the nucleic acid of the polymeric carrier cargo complex, preferably contained in the pharmaceutical composition, is a coding nucleic acid as defined herein, preferably a cDNA or an mRNA, more preferably encoding an adjuvant protein preferably as defined herein. In this context, the polymeric carrier cargo complex, typically initiates an innate immune response in the patient to be treated.

In a specific embodiment in this context, it is preferred that an adjuvant protein is a component of the polymeric carrier cargo complex and, preferably, of the polymeric carrier.

According to a further aspect, the present invention also provides kits, particularly kits of parts, comprising as components alone or in combination with optional further ingredients, and including (as a first component):

(A) a polymeric carrier cargo complex, comprising:

a) as a carrier a polymeric carrier formed by disulfide-crosslinked cationic components, and

b) as a cargo at least one nucleic acid molecule;

and (as a second component):

43

(B) at least one antigen associated with a cancer or tumour disease, wherein said (idiotype) antigen is selected from: (x) an idiotype immunoglobulin; (e.g. an idiotype antibody or an idiotype B cell receptor); and (y) an idiotype T cell receptor or in each case a fragment, variant and/or derivative of said antigen.

in each case as defined anywhere herein, and optionally technical instructions with information on the administration and dosage of the idiotype immunoglobulin, the idiotype T cell receptor and the polymeric carrier cargo complex. Such kits, preferably kits of parts, may be applied, e.g., for any of the applications or uses as defined herein. Such kits, when occurring as a kit of parts, may further contain each component of inventive pharmaceutical composition in a different part of the kit.

In certain embodiments of the kits of the present invention, the at least one (idiotype) antigen being an idiotype immunoglobulin (an idiotype antibody or an idiotype B cell receptor) or an idiotype T cell receptor (or a fragment, variant and/or derivative thereof) is comprised in a vaccine.

The present invention furthermore provides several applications and uses of the inventive pharmaceutical composition or of kits or kits of parts comprising same as defined anywhere herein.

In this context, the present invention also provides a method for transfecting and/or treating a cell, a tissue or an organism, thereby applying or administering the inventive pharmaceutical composition, particularly for therapeutic purposes. In this context, typically after preparing the inventive pharmaceutical composition, the inventive pharmaceutical composition is preferably administered to a cell, a tissue or an organism, preferably using any of the administration modes as described herein. The method for transfecting and/or treating a cell may be carried out in *vitro, in vivo* or *ex vivo.*

Furthermore the present invention provides the use of a pharmaceutical composition or of kits or kits of parts comprising same in each case as defined anywhere herein, in therapy and/or as a medicament, preferably as a vaccine such as an adjuvanted vaccine.

In this aspect of the present invention, particularly preferred is the use of the inventive pharmaceutical composition or of the kits or kits of parts comprising same as defined anywhere herein in therapy, preferably for use in the therapy of cancer or tumour disease, more preferably a cancer or tumour disease selected from the group consisting of: lymphoma, B cell lymphoma, T cell lymphoma and Non-Hodgkin's lymphoma.

In this context, B cell lymphoma may be selected from mature B cell neoplasms including chronic lymphatic leukemia/small lymphocytic lymphoma), B cell prolymphocytic leukemia, splenic marginal zone lymphoma, hairy cell leukemia, splenic lymphoma/unclassifiable leukemia (e.g. splenic diffuse red pulp small B cell lymphoma and hairy cell leukemia variant), lymphoplasmacytic lymphoma (e.g. Waldenström macroglobulinemia), heavy chain diseases (e.g. $\alpha$ heavy chain disease, $\gamma$ heavy chain disease and $\mu$ heavy chain disease), plasma cell myeloma, solitary plasmacytoma of bone, extraosseous plasmacytoma, extranodal marginal zone lymphoma of mucosa-associated lymphoid tissue (MALT lymphoma), Nodal marginal zone lymphoma (e.g. pediatric nodal marginal zone lymphoma), follicular lymphoma (e.g. pediatric follicular lymphoma), primary cutaneous follicle centre lymphoma, mantle cell lymphoma, diffuse large B cell lymphoma (DLBCL) (e.g. T cell/histiocyte rich large B cell lymphoma, primary diffuse large B cell lymphoma of the CNS, primary cutaneous diffuse large B cell lymphoma (leg type), and EBV-positive diffuse large B cell lymphoma of the elderly), diffuse large B cell lymphoma associated with chronic inflammation, lymphomatoid granulomatosis, primary mediastinal (thymic) large B cell lymphoma, intravascular large B cell lymphoma, ALK-positive large B cell lymphoma, plasmablastic lymphoma, large B cell lymphoma arising in HHV8-associated multicentric Castleman disease, primary effusion lymphoma, Burkitt lymphoma, B cell lymphoma unclassifiable with features intermediate between diffuse large B cell lymphoma and Burkitt lymphoma, B cell lymphoma unclassifiable with features intermediate between diffuse large B cell lymphoma, classical Hodgkin lymphoma and posttransplantation lymphoproliferative disorders concerning B cells.

T cell lymphoma may be selected from mature T cell neoplasms including T cell prolymphocytic leukemia, T cell large granular lymphocytic leukemia, EBV-positive T cell lymphoproliferative disease of childhood, Hydroa vacciniform-like lymphoma, adult T cell leukemia/lymphoma, extranodal nasal type T cell lymphoma, enteropathy-associated T cell lymphoma, hepatosplenic T cell lymphoma, subcutaneous panniculitis-like T cell lymphoma, mycosis fungoides, Sézary syndrome, primary cutaneous CD30+ T cell lymphoproliferative disorders (e.g. Lymphomatoid papulosis, primary cutaneous anaplastic large cell lymphoma), primary cutaneous $\gamma\delta$ T-cell lymphoma, primary cutaneous CD8+ aggressive epidermotropic cytotoxic T-cell lymphoma, primary cutaneous CD4+ small/medium T-cell lymphoma, peripheral T-cell lymphoma, angioimmunoblastic T-cell lymphoma, ALK-positive anaplastic large cell lymphoma, ALK-negative anaplastic large cell lymphoma, and posttransplantation lymphoproliferative disorders concerning T cells.

Non-Hodgkin lymphomas (NHLs) are a diverse group of blood cancers that include any kind of lymphoma (such as those described above) except Hodgkin's lymphomas. Hodgkin's disease, (or lymphomas) is a type of lymphoma, which is a cancer originating from white blood cells called lymphocytes. Hodgkin's lymphoma is characterized by the orderly spread of disease from one lymph node group to another and by the development of systemic symptoms with advanced disease. When Hodgkins cells are examined microscopically, multinucleated Reed-Sternberg cells (RS cells) are the characteristic histopathologic finding.

In a further aspect, the present invention provides a polymeric carrier cargo complex as defined anywhere herein, such as one comprising:

> a) (as a carrier) a polymeric carrier formed by disulfide-crosslinked cationic components, <u>and</u>
> b) (as a cargo) at least one nucleic acid molecule

for use in therapy in combination with at least one antigen associated with a cancer or tumour disease, wherein said (idiotpye) antigen is selected from: (x) an idiotype immunoglobulin; or (y) an idiotype T cell receptor (or a fragment, variant and/or derivative thereof), in each case as defined anywhere herein, particularly in the therapy of cancer or a tumour disease such as lymphoma.

Additionally, the present invention provides at least one antigen associated with a cancer or tumour disease, wherein said (idiotpye) antigen is selected from: (x) an idiotype immunoglobulin; or an idiotype T cell receptor (or a fragment, variant and/or derivative thereof,) in each case as defined anywhere herein, for use in therapy in combination with a polymeric carrier cargo complex as defined anywhere herein, such as one comprising:

> a) (as a carrier) a polymeric carrier formed by disulfide-crosslinked cationic components, <u>and</u>
> b) (as a cargo) at least one nucleic acid molecule,

particularly in the therapy of cancer or a tumour disease such as lymphoma.

In this context "in combination" means that the different components (the polymeric carrier cargo complex and the (idiotype) antigen such as an idiotype immunoglobulin or idiotyp T cell receptor (or a fragment, variant and/or derivative thereof)) can be provided together in the same composition, or can be formulated separately in different compositions, i.e. one composition comprising or representing the polymeric carrier cargo complex as defined herein, and one further composition comprising the (idiotype) antigen (or a fragment, variant and/or derivative thereof) as defined herein. If provided in different compositions the polymeric carrier cargo complex and the (idiotype) antigen (or a fragment, variant and/or derivative thereof) may be administered separated in time (in a time-staggered manner) and/or may be administered at different administration sites. This means that the polymeric carrier cargo complex may be administrated e.g. prior, concurrent or subsequent to the at least one (idiotype) antigen (or a fragment, variant and/or derivative thereof), or vice versa. Subsequent administration includes that each component used in the therapy is administered within about 48 hours, 24 hours, 12 hours, 8 hours, 6 hours, 4 hours, 2 hours, 1 hour, 30 mins, 15 mins or 5 mins of each other.

In a further aspect the present invention provides a pharmaceutical package, including:

> (A) a polymeric carrier cargo complex, comprising:
>
> > a) (as a carrier) a polymeric carrier formed by disulfide-crosslinked cationic components, <u>and</u>
> > b) (as a cargo) at least one nucleic acid molecule,
>
> as defined anywhere herein;
> <u>and</u>
>
> (B) instructions describing the use of said polymeric carrier cargo complex in therapy in combination with at least one one antigen associated with a cancer or tumour disease, wherein said (idiotpye) antigen is selected from: (x) an idiotype immunoglobulin; or (y) an idiotype T cell receptor (or a fragment, variant and/or derivative thereof) in each case as defined anywhere herein.

Furthermore the present invention provides in an additional embodiment a pharmaceutical package, including:

> (A) at least one one antigen associated with a cancer or tumour disease, wherein said (idiotpye) antigen is selected from: (x) an idiotype immunoglobulin; or (y) an idiotype T cell receptor (or a fragment, variant and/or derivative thereof), in each case, as defined anywhere herein;
> <u>and</u>
>
> (B) instructions describing the use of said (idiotype) antigen (or a fragment, variant and/or derivative thereof) in therapy in combination with a polymeric carrier cargo complex, in each case as defined anywhere herein.

In this context the invention furthermore provides the use of the components included in the above defined pharmaceutical packages in the therapy of cancer or tumour disease such as lymphoma, wherein said instructions further describe the use in the therapy of cancer or tumour disease such as lymphoma.

In certain embodiments of the therapeutic aspects of the present invention, the therapy using a polymeric carrier cargo complex and a (idiotype) antigen (in each case as defined anywhere herein), also comprises their us in combination with an additional pharmaceutically active component. As described above, such additional pharmaceutically active component may be included in the pharmaceutical composition of the present invention. Alternatively, it may be included in the kit or kit of parts as an additional component. Furthermore, the additional pharmaceutically active component may be used in separate but concurrent or sequential administration with one or more of the other components. For example, the additional pharmaceutically active component may be administered within about 10 days, 7 days, 5 days, 3 days, 2 days or 1 day of another component used in the therapy; or it may be administered within each other about 48 hours, 24 hours, 12 hours, 8 hours, 6 hours, 4 hours, 2 hours, 1 hour, 30 mins, 15 mins or 5 mins of said other component.

In other certain embodiments, said additional pharmaceutically active component is an antiproliferative agent such as one that is or may be used in the therapy of a cancer or tumour disease, as defined herein. Exemplary antiproliferative agents include vinca alkaloids (vinblastine), the anthracyclines (adriamycin), the epipodophyllotoxins (etoposide), antibiotics (actinomycin D and gramicidin D), antimicrotubule drugs (colchicine), protein synthesis inhibitors (puromycin), toxic peptides (valinomycin), topoisomerase I inhibitors (topotecan), DNA intercalators (ethidium bromide), antimitotics, vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine, nocodazole, platinum-based agents (cisplatin, paraplatin, carboplatin, but not the subject platinum-based chemotherapeutic agents as defined herein), camptothecin, 9-nitrocamptothecin (Orethecin, rubitecan), 9-aminocamptothecin (IDEC-131), exatecan (DX-8951f), lurtotecan (GI-147211C), BAY 38-3441, the homocamptothecins such as diflomotecan (BN-80915) and BN-80927, topotecan (Hycamptin), NB-506, J107088, pyrazolo [1,5-a] indole derivatives, such as GS-5, lamellarin D, irinotecan (Camptosar, CPT- 11), and antibodies, such as 1D10, Hu1D10, 1D09C3, 1C7277, 305D3, rituximab, 4D5, Mab225, C225, Daclizumab (Zenapax), Antegren, CDP 870, CMB-401, MDX-33, MDX-220, MDX-477, CEA-CIDE, AHM, Vitaxin, 3622W94, Therex, 5G1.1, IDEC-131, HU-901, Mylotarg, Zamyl (SMART M195), MDX-210, Humicade, LymphoCIDE, ABX-EGF, 17-1A, Trastuzumab (Herceptin ®, rhuMAb), Epratuzumab, Cetuximab (Erbitux ®), Pertuzumab (Omnitarg®, 2C4), R3, CDP860, Bevacizumab (Avastin ®), tositumomab (Bexxar ®), Ibritumomab tiuxetan (Zevalin ®), M195, 1 D10, Hu1 D10 (Remitogen®, apolizumab), Danton/DN1924, an "HD" antibody such as HD4 or HD8, CAMPATH-1 and CAMPATH-1H or other variants, fragments, conjugates, derivatives and modifications thereof, or other equivalent compositions with improved or optimized properties. In particular embodiments, such as when the present invention is used in therapy of lymphomas, the additional pharmaceutically active component may be an anti-CD 20 antibody, for example rituximab.

In all aspects of the invention relating to therapy or use as a medicament, in preferred embodiments said therapy or use is the therapy of, or the use as a medicament for, a patient in need thereof. In particular, the patient is an individual having the same idiotype of the (idiotype) antigen as defined herein. Typically, the patient will be the same individual from whom the (idiotype) antigen was derived from (e.g. malignant) cell, such as a malignant B or T cell.

In the present invention, if not otherwise indicated, different features of alternatives and embodiments may be combined with each other, where suitable. Furthermore, the term "comprising" shall not be construed as meaning "consisting of", if not specifically mentioned. However, in the context of the present invention, term "comprising" may be substituted with the term "consisting of", where suitable.

**Figures:**

The following Figures are intended to illustrate the invention further. They are not intended to limit the subject matter of the invention thereto.

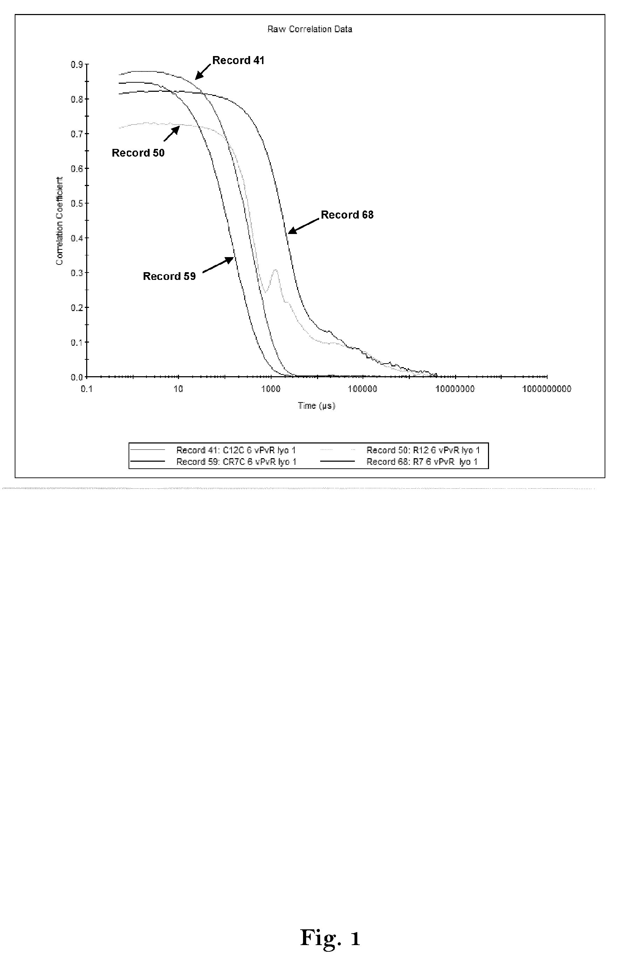

**Figure 1:** shows the raw correlation curve of polymeric carrier cargo complexes formed by the disulfide-crosslinked cationic peptides $CR_{12}C$ and $CR_7C$ as carrier after lyophilisation compared to complexes with non-polymerizing cationic peptides as carrier ($R_{12}$ and $R_7$) by dynamic light scattering using a Zetasizer Nano (Malvern Instruments, Malvern, UK). The hydrodynamic diameters were measured with fresh prepared complexes and with reconstituted complexes after lyophilisation The mass ratio of peptide:RNA was 1: 2. As result it can be shown that the polymeric carrier cargo complexes comprising cystein-containing peptides as cationic components which lead to a polymerization of the polymeric carrier by disulfide bonds do not change in size in contrast to the complexes formed by non-polymerizing peptides which increase in size and therefore are not stable during the lyophilization step. Therefore complexes with polymerized peptides as polymeric carriers show advantageous properties for lyophilization.

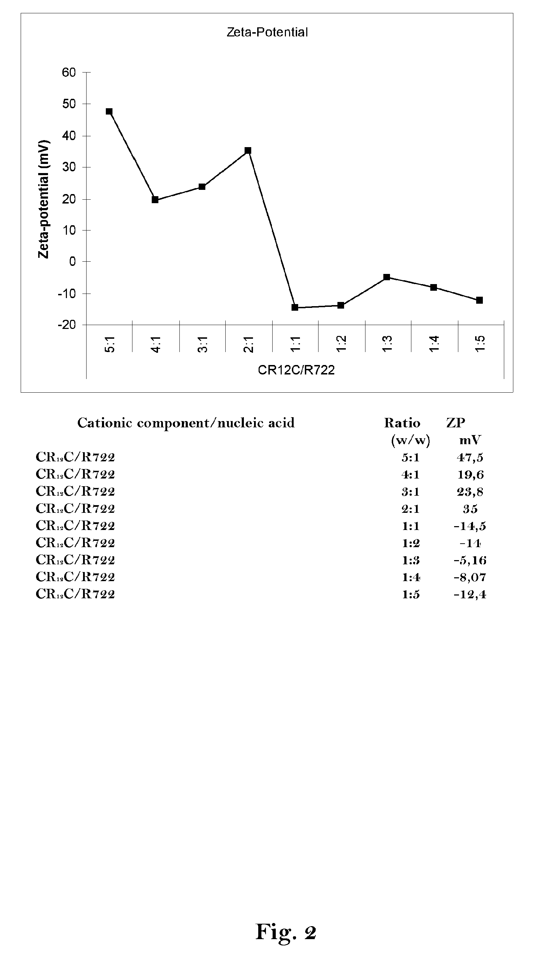

**Figure 2:** shows the Zeta-potential of polymeric carrier cargo complexes formed by the disulfide-cross-linked cationic peptide $CR_{12}C$ and the R722 as nucleic acid cargo at different w/w ratios. As can be seen, the zeta potential changes from positive to negative when the w/w ratio is changed from excess peptide to a 1:1 ratio (peptide/RNA).

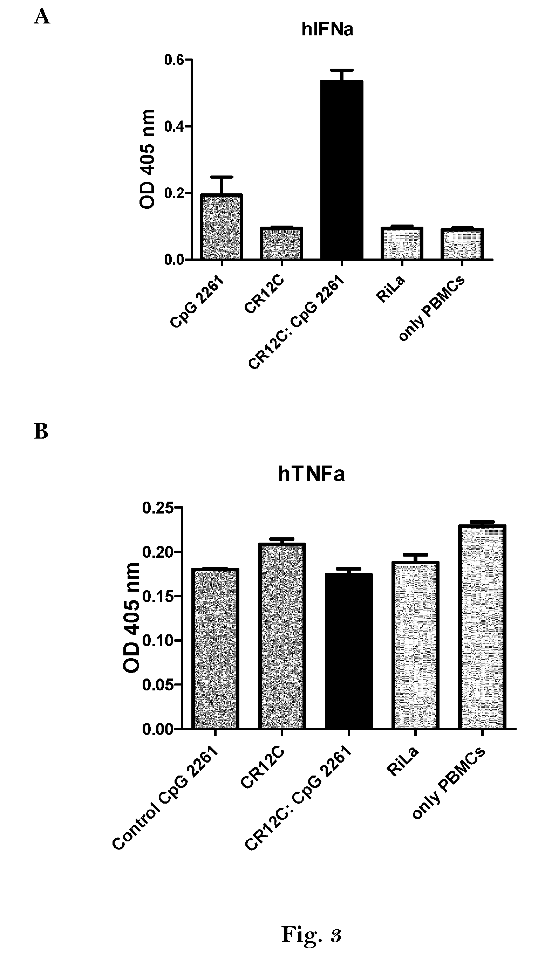

**Figure 3A**: shows the secretion of hIFNa cytokine *(in vitro)* in hPBMCs after stimulation with polymeric carrier cargo complexes formed by the disulfide-crosslinked cationic peptide $CR_{12}C$ and the CpG 2216 as nucleic acid cargo in a mass ratio of 1:2,5 (w/w) ($CR_{12}C$/CpG 2216). As can be seen, the polymeric carrier cargo complexes lead to an increase of hIFNa cytokine release in hPBMCs compared to the nucleic acid cargo alone or the cationic peptide alone.

**Figure 3B**: shows the secretion of hTNFa cytokine *(in vitro)* in hPBMCs after stimulation with polymeric carrier cargo complexes formed by the disulfide-crosslinked cationic peptide $CR_{12}C$ and the CpG 2216 as nucleic acid cargo in a mass ratio of 1:2,5 (w/w) ($CR_{12}C$/CpG 2216). As can be seen, the polymeric carrier cargo complexes do not lead to an increase in hTNFa cytokine release in hPBMCs compared to the nucleic acid cargo alone or the cationic peptide alone.

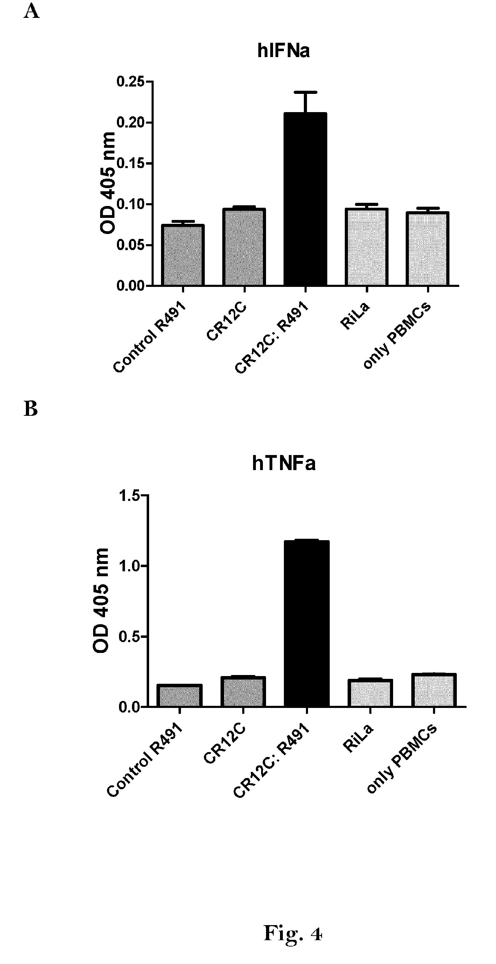

**Figure 4A**: shows the secretion of hIFNa cytokine *(in vitro)* in hPBMCs after stimulation with polymeric carrier cargo complexes formed by the disulfide-crosslinked cationic peptide $CR_{12}C$ and the mRNA R491 coding for luciferase as nucleic acid cargo in a mass ratio of 1:2 (w/w) ($CR_{12}C$/R491). As can be seen, the polymeric carrier cargo complexes lead to an increase of hIFNa cytokine release in hPBMCs compared to the nucleic acid cargo alone or the cationic peptide alone.

**Figure 4B**: shows the secretion of hTNFa cytokine *(in vitro)* in hPBMCs after stimulation with polymeric carrier cargo complexes formed by the disulfide-crosslinked cationic peptide $CR_{12}C$ and the mRNA R491 coding for luciferase as nucleic acid cargo in a mass ratio of 1:2 (w/w) ($CR_{12}C$/R491). As can be seen, the polymeric carrier cargo complexes lead to an increase of hTNFa cytokine release in hPBMCs compared to the nucleic acid cargo alone or the cationic peptide alone.

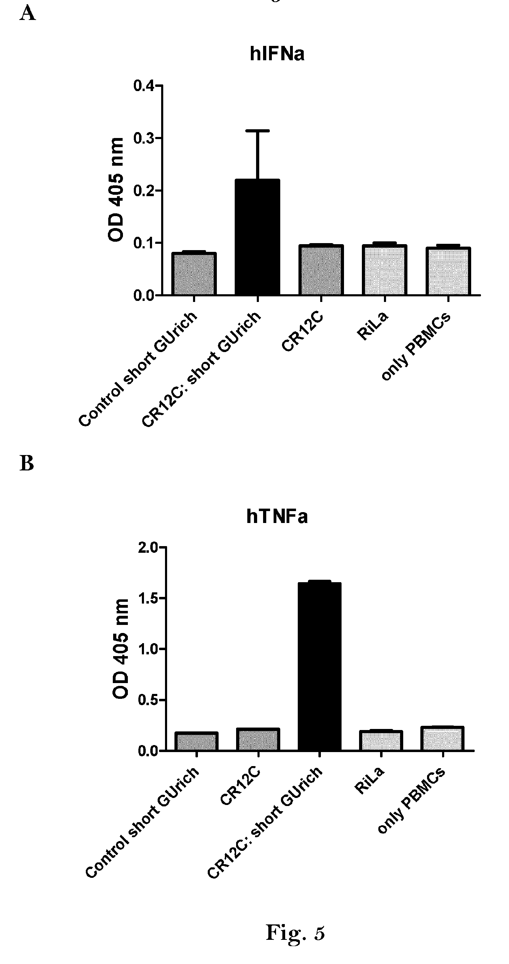

**Figure 5A**: shows the secretion of hIFNa cytokine *(in vitro)* in hPBMCs after stimulation with polymeric carrier cargo complexes formed by the disulfide-crosslinked cationic peptide $CR_{12}C$ and a short GU rich RNA oligonucleotide (short GU rich) as nucleic acid cargo in a mass ratio of 1:2,5 (w/w) ($CR_{12}C$/short GU rich). As can be seen, the polymeric carrier cargo complexes lead to an increase of hIFNa cytokine release in hPBMCs compared to the nucleic acid cargo alone or the cationic peptide alone.

**Figure 5B**: shows the secretion of hTNFa cytokine *(in vitro)* in hPBMCs after stimulation with polymeric carrier cargo complexes formed by the disulfide-crosslinked cationic peptide $CR_{12}C$ and a short GU rich RNA oligonucleotide (short GU rich) as nucleic acid cargo in a mass ratio of 1:2,5 (w/w) ($CR_{12}C$/short GU rich). As can be seen, the polymeric carrier cargo complexes lead to an increase of hTNFa cytokine release in hPBMCs compared to the nucleic acid cargo alone or the cationic peptide alone.

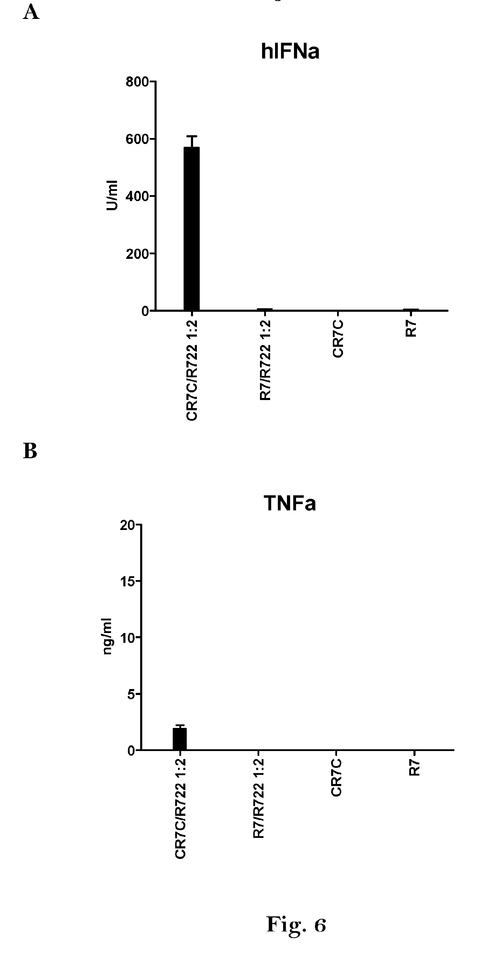

**Figure 6A**: shows the secretion of hIFNa cytokine *(in vitro)* in hPBMCs after stimulation with polymeric carrier cargo complexes formed by the disulfide-crosslinked cationic peptide $CR_7C$ and the long non-coding GU-rich isRNA R722 as nucleic acid cargo. As can be seen, the polymeric carrier cargo complexes ($CR_7C$/R722) lead to an increase of hIFNa cytokine release in hPBMCs compared to cargo complexes ($R_7$/R722) formed by the non-polymerized peptide $R_7$.

**Figure 6B**: shows the secretion of hTNFa cytokine *(in vitro)* in hPBMCs after stimulation with polymeric carrier cargo complexes formed by the disulfide-crosslinked cationic peptide $CR_7C$ and the long non-coding GU-rich isRNA R722 as nucleic acid cargo. As can be seen, the polymeric carrier cargo complexes ($CR_7C$/R722) only leads to a weak increase of hTNFa cytokine release in hPBMCs compared to carrier cargo complexes ($R_7$/R722) formed by the non-polymerized peptide $R_7$.

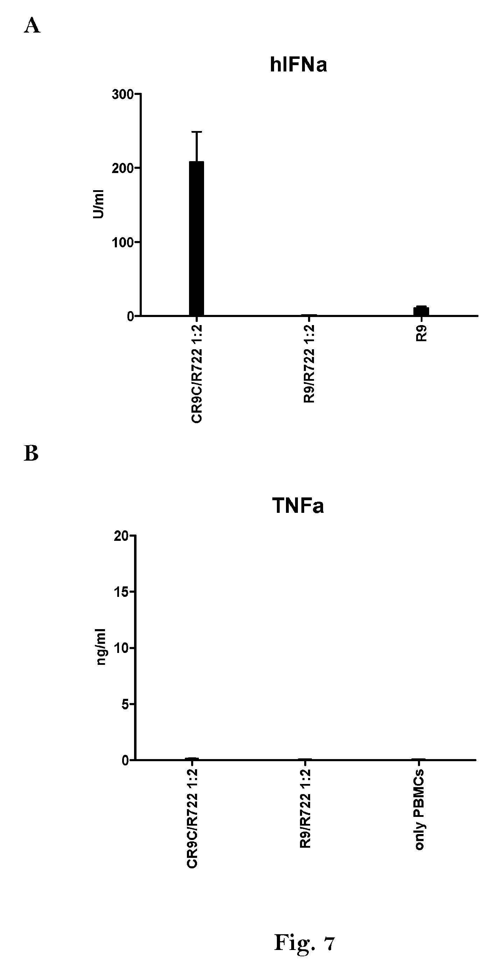

**Figure 7A**: shows the secretion of hIFNa cytokine *(in vitro)* in hPBMCs after stimulation with polymeric carrier cargo complexes formed by the disulfide-crosslinked cationic peptide $CR_9C$ and the long non-coding GU-rich isRNA R722 as nucleic acid cargo. As can be seen, the inventive polymeric carrier cargo complexes ($CR_9C$/R722) lead to an increase of hIFNa cytokine release in hPBMCs compared to carrier cargo complexes ($R_9$/R722) formed by the non-polymerized peptide $R_9$.

**Figure 7B**: shows the secretion of hTNFa cytokine *(in vitro)* in hPBMCs after stimulation with polymeric carrier cargo complexes formed by the disulfide-crosslinked cationic peptide $CR_9C$ and the long non-coding GU-rich isRNA R722 as nucleic acid cargo. As can be seen, the polymeric carrier cargo complexes ($CR_9C$/R722) do not lead to an increase of hTNFa cytokine release in hPBMCs compared to carrier cargo complexes

(R$_9$/R722) formed by the non-polymerized peptide R$_9$.

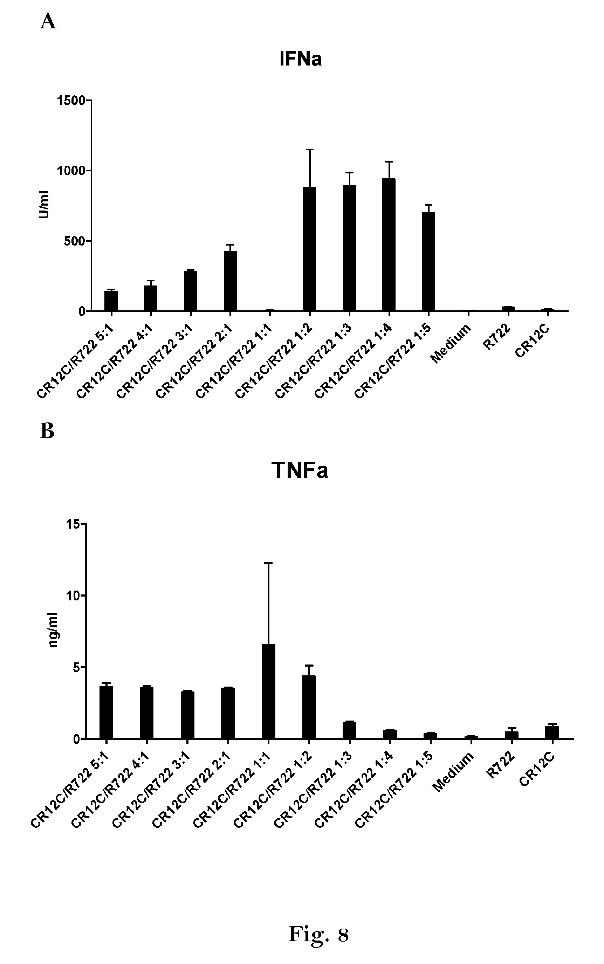

**Figure 8A**: shows the secretion of hIFNa cytokine *(in vitro)* in hPBMCs after stimulation with polymeric carrier cargo complexes formed by the disulfide-crosslinked cationic peptide CR$_{12}$C and the isRNA R722 as nucleic acid cargo at different w/w ratios. As can be seen, the polymeric carrier cargo complexes lead to an increase in hIFNa cytokine release in hPBMCs compared to the nucleic acid cargo alone or the cationic peptide alone.

**Figure 8B**: shows the secretion of hTNFa cytokine *(in vitro)* in hPBMCs after stimulation with polymeric carrier cargo complexes formed by the disulfide-crosslinked cationic peptide CR$_{12}$C and the isRNA R722 as nucleic acid cargo at different w/w ratios. As can be seen, the polymeric carrier cargo complexes lead to an increase in hTNFa cytokine release in hPBMCs compared to the nucleic acid cargo alone or the cationic peptide alone.

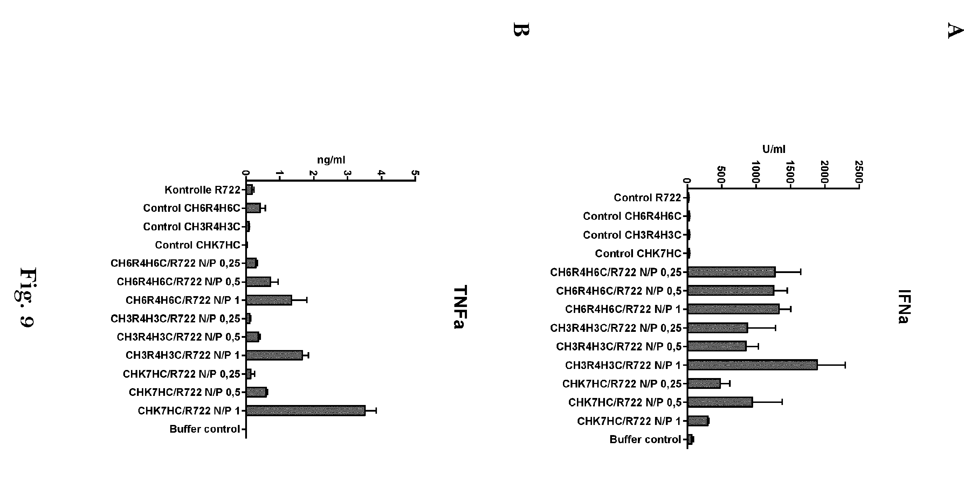

**Figure 9A**: shows the secretion of hIFNa cytokine *(in vitro)* in hPBMCs after stimulation with polymeric carrier complexes formed by the cationic peptides CH$_6$R$_4$H$_6$C, CH$_3$R$_4$H$_3$C and CHK$_7$HC and the isRNA R722 as nucleic acid cargo at different N/P ratios. As can be seen, the polymeric carrier cargo complexes lead to an increase in hIFNa cytokine release in hPBMCs compared to the nucleic acid cargo alone or the cationic peptide alone.

**Figure 9B**: shows the secretion of hTNFa cytokine *(in vitro)* in hPBMCs after stimulation with polymeric carrier complexes formed by the disulfide-crosslinked cationic peptides CH$_6$R$_4$H$_6$C, CH$_3$R$_4$H$_3$C and CHK$_7$HC and the isRNA R722 as nucleic acid cargo at different N/P ratios. As can be seen, the polymeric carrier cargo complexes lead to an increase in hTNFa cytokine release in hPBMCs compared to the nucleic acid cargo alone or the cationic peptide alone. Particularly polymeric cargo complexes with an N/P ratio greater or equal 1 result in TNFalpha secretion.

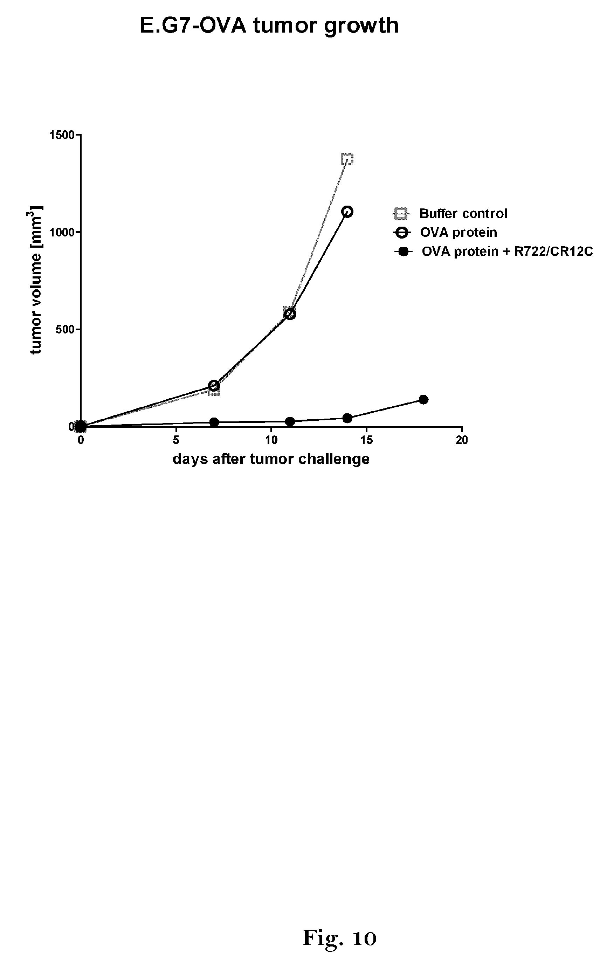

**Figure 10:** shows the *(in vivo)* effect of the addition of the polymeric carrier cargo complex formed by the disulfide-crosslinked cationic peptide CR$_{12}$C as carrier and the isRNA R722 as nucleic acid cargo to the protein antigen Ovalbumine (OVA protein) for the use as an adjuvant in tumour challenge experiments. For this purpose 7 female C57BL/6 mice per group were vaccinated three times in two weeks with μg 5 μg Ovalbumin protein combined with 45 μg CR12C/R722 (1:2; w/w). For comparison mice were injected without the polymeric cargo complexes. As can be seen, the polymeric carrier cargo complex extremely decelaterates the tumour growth compared to the protein antigen alone, which has no effect on tumor growth in comparison to the buffer control.

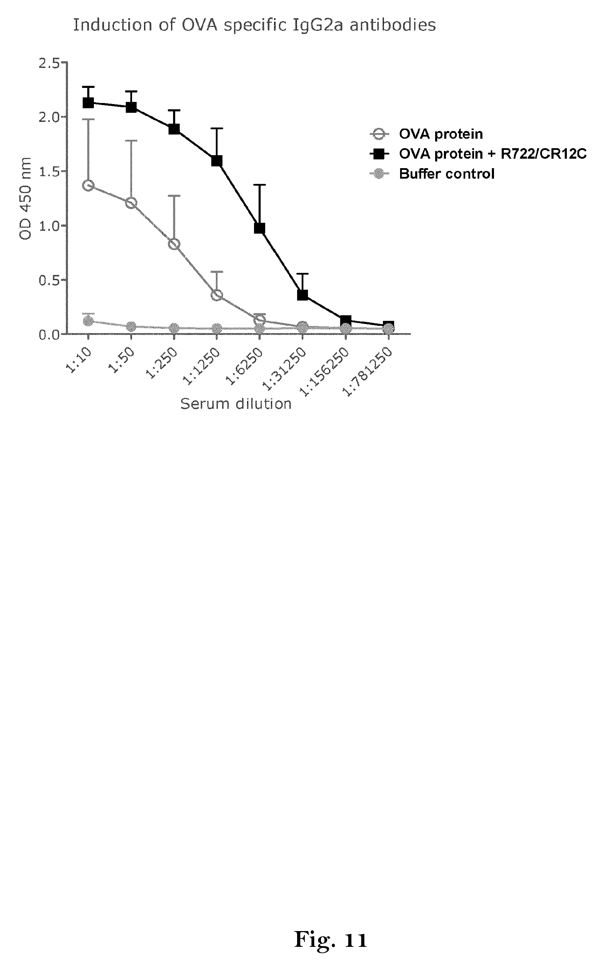

**Figure 11**: shows the *(in vivo)* effect of the addition of the polymeric carrier cargo complex formed by the disulfide-crosslinked cationic peptide CR$_{12}$C as carrier and the isRNA R722 as nucleic acid cargo to the protein antigen Ovalbumine (OVA protein) for the use as an adjuvant on the induction of Ovalbumine-specific IgG2a antibodies. For this purpose 5 female C57BL/6 mice per group were vaccinated three times in two weeks with μg 5 μg Ovalbumin protein combined with 45 μg CR12C/R722 (1:2; w/w). For comparison mice were injected without the polymeric cargo complexes. As can be seen, the polymeric carrier cargo complex strongly increases the B-cell response, which proofs the beneficial adjuvant properties of the polymeric carrier cargo complexes, particularly in regard to the induction of a Th1-shifted immune response.

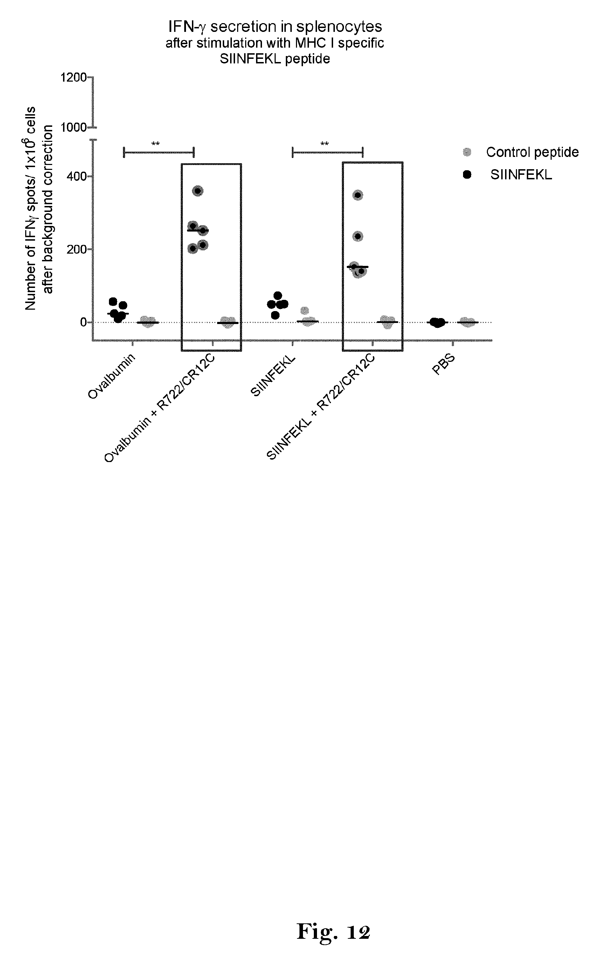

**Figure 12**: shows the *(in vivo)* effect of the addition of the polymeric carrier cargo complex formed by the disulfide-crosslinked cationic peptide CR$_{12}$C as carrier and the isRNA R722 as nucleic acid cargo to the protein antigen Ovalbumine (OVA protein) or the Ovalbumine-specific peptide antigen SIINFEKL for the use as an adjuvant on the induction of Ovalbumine-specific cytotoxic T cells (as represented by number of spots in the ELISPOT assay). For this purpose 5 female C57BL/6 mice per group were vaccinated three times in two weeks with 5 μg Ovalbumin protein or 50 μg SIINFEKEL peptide combined with 45 μg CR12C/R722 (1:2; w/w). For comparison mice were injected without the polymeric cargo complexes. As can be seen, the polymeric carrier cargo complex strongly increases the induction of Ovalbumin-specific cytotoxic T cells compared to the vaccination with protein or peptide alone, which further proofs the beneficial adjuvant properties of the polymeric carrier cargo complex, particularly in regard to the induction of a Th1-shifted immune response.

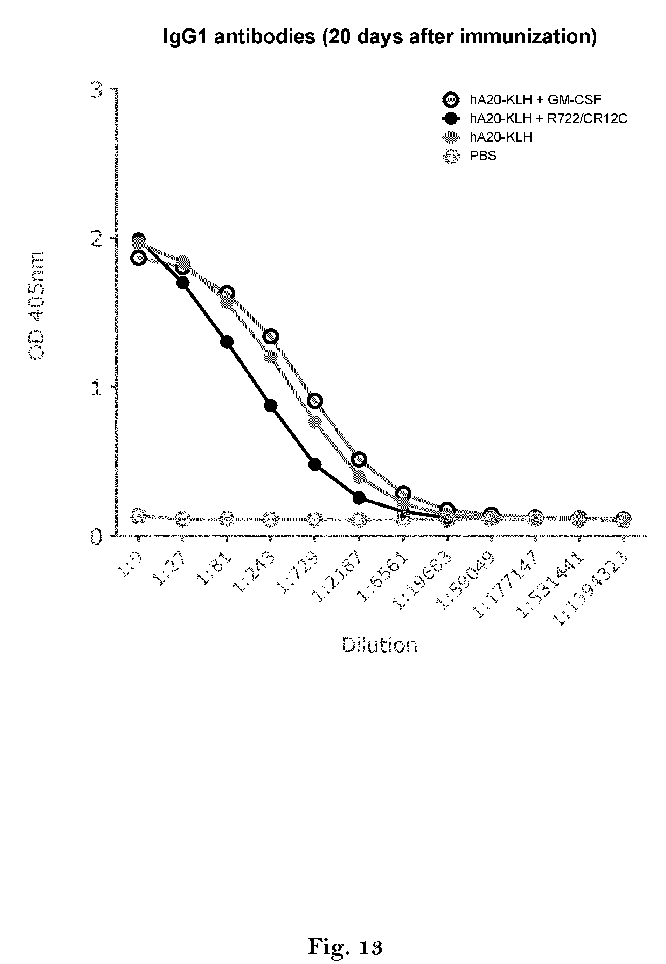

**Figure 13:** shows the *(in vivo)* effect of the addition of the polymeric carrier cargo complex formed by the disulfide-crosslinked cationic peptide $CR_{12}C$ as carrier and the isRNA R722 as nucleic acid cargo to the KLH-linked idiotype immunoglobulin of the A20 mouse B lymphoma cell line (hA20-KLH) for the use as an adjuvant on the induction of immunoglobulin hA20-specific IgG1 antibodies (as represented by OD 405nm). For this purpose 8 female BALB/c mice per group were vaccinated once with 1 μg KLH-hA20 and 30 μg R722/15 μg CR12C (2:1; w/w). hA20-specific IgG1 antibodies in pooled sera (1 pool/treatment group) were measured 20 days after vaccination. As can be seen, the polymeric carrier cargo complex does not increase the Th2-immune response directed against the protein antigen (hA20-KLH + R722/CR12C), compared to vaccination with the protein antigen alone (hA20-KLH) or the combination of the protein antigen with the adjuvant cytokine GM-CSF (hA20-KLH + GM-CSF). This result shows that the adjuvant formed by the polymeric carrier CR12C and the isRNA R722 does not improve the Th2-based immune response directed against the idiotype immunoglobulin hA20.

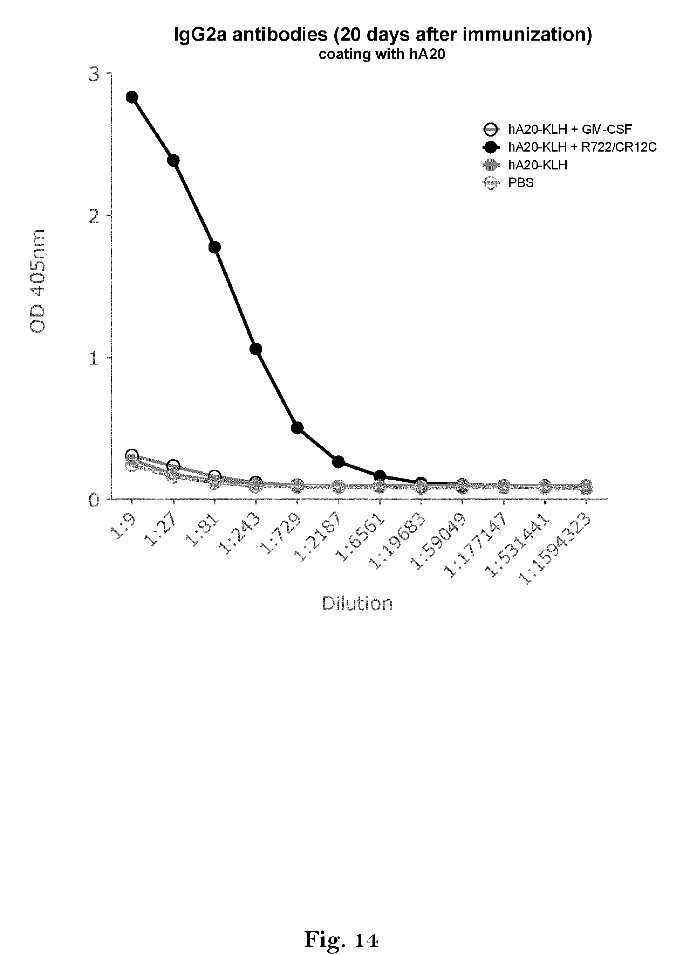

**Figure 14:** shows the *(in vivo)* effect of the addition of the polymeric carrier cargo complex formed by the disulfide-crosslinked cationic peptide $CR_{12}C$ as carrier and the isRNA R722 as nucleic acid cargo to the KLH-linked idiotype immunoglobulin of the A20 mouse B lymphoma cell line (hA20-KLH) for the use as an adjuvant on the induction of immunoglobulin hA20-specific IgG2a antibodies (as represented by OD 405nm). For this purpose 8 female BALB/c mice per group were vaccinated once with 1 μg KLH-hA20 and 30 μg R722/15 μg CR12C (2:1; w/w). hA20-specific IgG2a antibodies in pooled sera (1 pool/treatment group) were measured 20 days after vaccination. As can be seen, the polymeric carrier cargo complex strongly increases the Th1-immune response directed against the protein antigen (hA20-KLH + R722/CR12C), compared to vaccination with the protein antigen alone (hA20-KLH) or the combination of the protein antigen with the adjuvant cytokine GM-CSF (hA20-KLH + GM-CSF). This result shows that the adjuvant formed by the polymeric carrier CR12C and the isRNA R722 remarkably improves the Th1-based immune response directed against the idiotype immunoglobulin hA20.

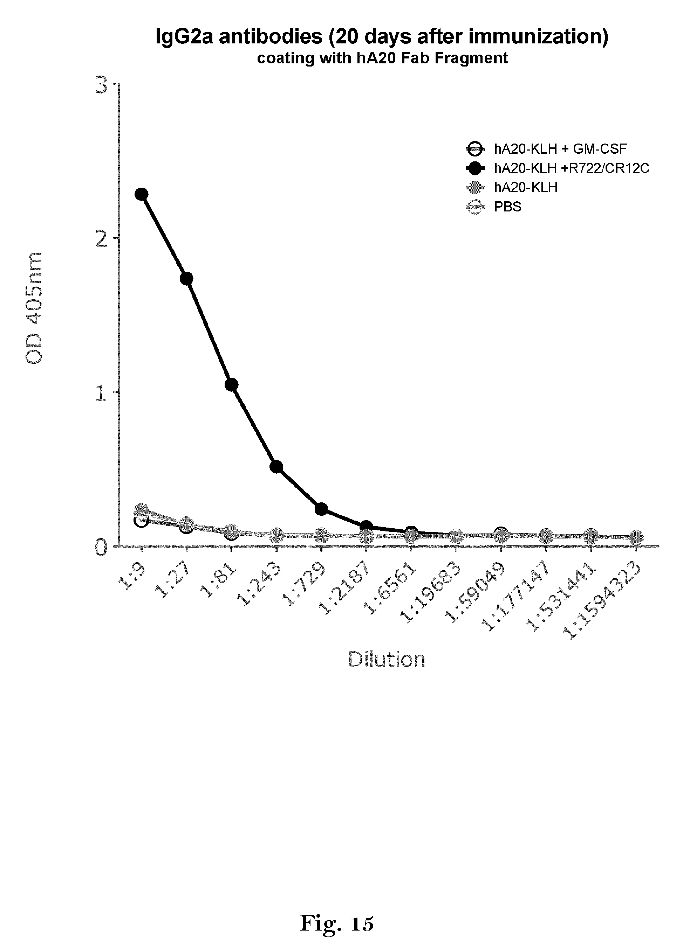

**Figure 15:** shows the *(in vivo)* effect of the addition of the polymeric carrier cargo complex formed by the disulfide-crosslinked cationic peptide $CR_{12}C$ as carrier and the isRNA R722 as nucleic acid cargo to the KLH-linked idiotype immunoglobulin of the A20 mouse B lymphoma cell line (hA20-KLH) for the use as an adjuvant on the induction of specific IgG2a antibodies directed against the Fab fragment of the idiotype immunoglobulin hA20 (as represented by OD 405nm). For this purpose 8 female BALB/c mice per group were vaccinated once with1 μg KLH-hA20 and 30 μg R722/15 μg CR12C (2:1; w/w). hA20Fab-specific IgG2a antibodies were measured 20 days after vaccination. As can be seen, the polymeric carrier cargo complex strongly increases the Th1-immune response directed against the hA20 Fab fragment, compared to vaccination with the protein antigen alone (hA20-KLH or hA20) or the combination of the protein antigen with the adjuvant cytokine GM-CSF (hA20-KLH + GM-CSF). This result shows that the adjuvant formed by the polymeric carrier CR12C and the isRNA R722 remarkably improves the Th1-based immune response directed against the idiotype immunoglobulin hA20.

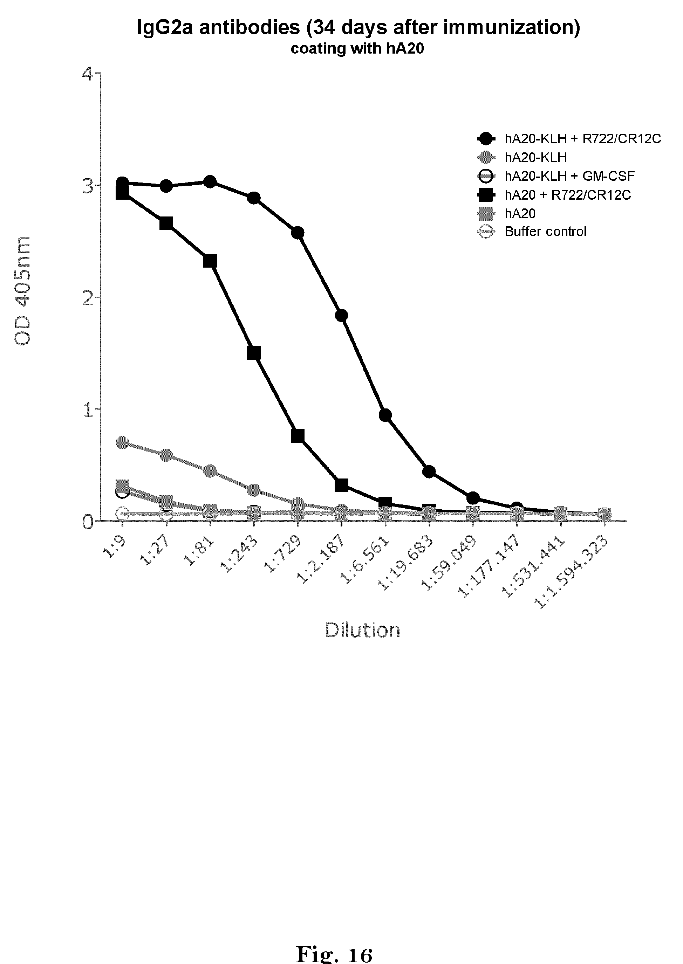

**Figure 16:** shows the *(in vivo)* effect of the addition of the polymeric carrier cargo complex formed by the disulfide-crosslinked cationic peptide $CR_{12}C$ as carrier and the isRNA R722 as nucleic acid cargo to the unconjugated idiotype immunoglobulin of the A20 mouse B lymphoma cell line (hA20) or to KLH-linked idiotype immunoglobulin of the A20 mouse B lymphoma cell line (hA20-KLH) for the use as an adjuvant on the induction of immunoglobulin A20-specific IgG2a antibodies (as represented by OD 405nm). For this purpose 8 female BALB/c mice per group were vaccinated twice with 0.5 μg hA20 or 1 μg KLH-hA20 and 30 μg R722/15 μg CR12C (2:1; w/w). A20-specific antibodies were measured 34 days after vaccination. As can be seen, the polymeric carrier cargo complex strongly increases the Th1-immune response directed against the unconjugated and the KLH-linked-protein antigen (hA20 + R722/CR12C and hA20-KLH + R722/CR12C), compared to vaccination with the protein antigen alone (hA20 and hA20-KLH) or the combination of the protein antigen with the adjuvant cytokine GM-CSF (hA20-KLH + GM-CSF). This result shows that the adjuvant formed by the polymeric carrier CR12C and the isRNA R722 remarkably improves the Th1-based immune response directed against the idiotype immunoglobulin hA20.

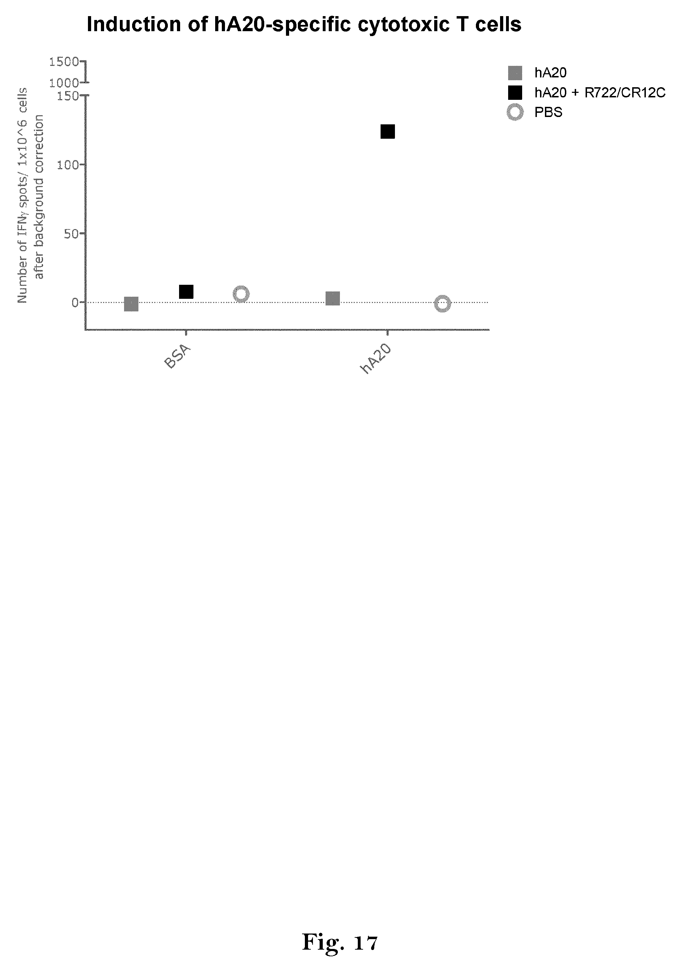

**Figure 17:** shows the *(in vivo)* effect of the addition of the polymeric carrier cargo complex formed by the disulfide-crosslinked cationic peptide $CR_{12}C$ as carrier and the isRNA R722 as nucleic acid cargo to the unconjugated idiotype immunoglobulin of the A20 mouse B lymphoma cell line (hA20) for the use as an adjuvant on the induction of immunoglobulin hA20-specific cytotoxic T cells (as represented by number of spots in the ELISPOT assay). For this purpose 8 female BALB/c mice per group were vaccinated four times in 10 weeks with 0.5 μg hA20 and 30 μg R722/15 μg CR12C (2:1; w/w). A20-specific cytotoxic T cells were measured 6 days after the last vaccination. As can be seen, the polymeric carrier cargo complex strongly increases the induction of A20-specific cytotoxic T cells (hA20 + R722/CR12C) compared to the vaccination with protein alone (hA20), which further proofs the beneficial adjuvant properties of the polymeric carrier cargo complex, particularly in regards to the induction of a Th1-shifted immune response.

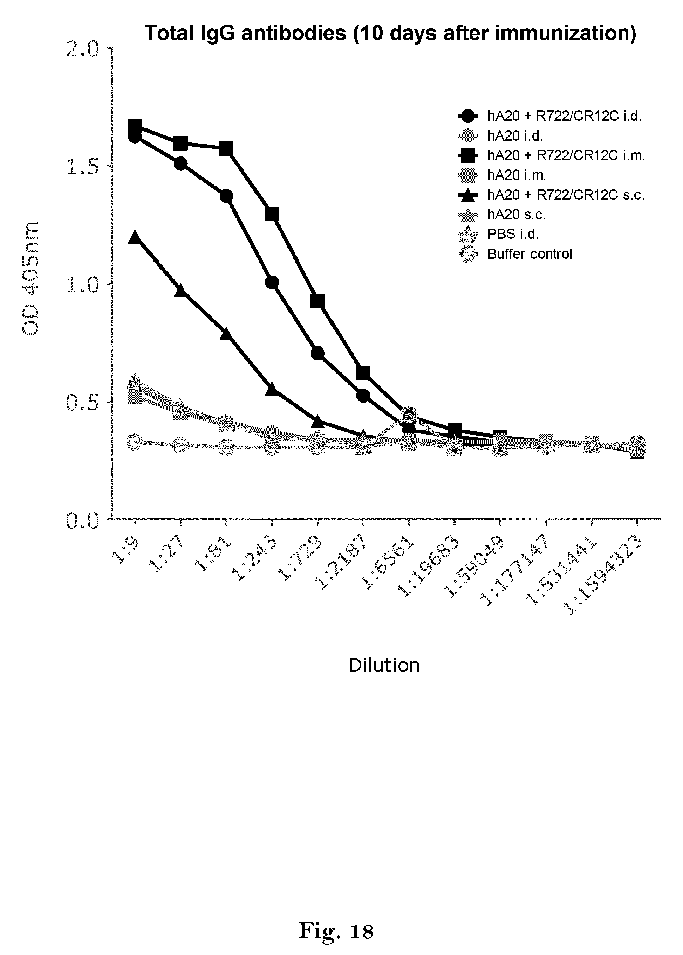

**Figure 18:** shows the *(in vivo)* effect of the addition of the polymeric carrier cargo complex formed by the disulfide-crosslinked cationic peptide $CR_{12}C$ as carrier and the isRNA R722 as nucleic acid cargo to the unconjugated idiotype immunoglobulin of the A20 mouse B lymphoma cell line (hA20) for the use as an adjuvant on the induction of immunoglobulin hA20-specific IgG antibodies (total IgG) (as represented by OD 405nm). For this purpose 8 female BALB/c mice per group were vaccinated once with 1 μg KLH-hA20 and 30 μg R722/15 μg CR12C (2:1; w/w) intradermally, intramuscularly or subcutaneously. hA20-specific IgG antibodies were measured 10 days after vaccination. As can be seen, the polymeric carrier cargo complex strongly increases the immune response directed against the protein antigen (hA20 + R722/CR12C), independently of the application route. This result shows that the adjuvant formed by the polymeric carrier CR12C and the isRNA R722 remarkably improves the immune response directed against the idiotype immunoglobulin hA20 and that the inventive vaccine can be administered intradermally, intramuscularly and subcutaneously.

## Examples

The following examples are intended to illustrate the invention further. They are not intended to limit the subject matter of the invention thereto.

## 1. <u>Reagents:</u>

<u>Cationic peptides as cationic component of the polymeric carrier:</u>

| | |
|---|---|
| $R_7$: | Arg-Arg-Arg-Arg-Arg-Arg-Arg ($Arg_7$) (SEQ ID NO. 109) |
| $CR_7C$: | Cys-Arg-Arg-Arg-Arg-Arg-Arg-Arg-Cys ($CysArg_7Cys$) (SEQ ID NO. 1) |
| $R_9$: | Arg-Arg-Arg-Arg-Arg-Arg-Arg-Arg-Arg ($Arg_9$) (SEQ ID NO. 110) |
| $R_{12}$: | Arg-Arg-Arg-Arg-Arg-Arg-Arg-Arg-Arg-Arg-Arg-Arg ($Arg_{12}$) (SEQ ID NO. 111) |
| $CR_9C$: | Cys-Arg-Arg-Arg-Arg-Arg-Arg-Arg-Arg-Arg-Cys ($Cys\text{-}Arg_9\text{-}Cys$) (SEQ ID NO. 2) |
| $CR_{12}C$: | Cys-Arg-Arg-Arg-Arg-Arg-Arg-Arg-Arg-Arg-Arg-Arg-Arg ($Cys\text{-}Arg_{12}\text{-}Cys$) (SEQ ID NO. 6) |

<u>Nucleic acids as cargo of the polymeric carrier cargo complex:</u>

| | |
|---|---|
| R1180: | mRNA coding for luciferase (SEQ ID NO. 112) |
| R722: | long non-coding isGU-rich RNA (SEQ ID NO. 105) |
| R491: | mRNA coding for luciferase (SEQ ID NO. 113) |
| CpG 2216: | CpG oligonucleotide (SEQ ID NO. 114) |
| Short GU rich: | GU-rich RNA oligonucleotide (SEQ ID NO. 115) |

Antigens and epitopes:

Ovalbumine-derived peptide SIINFEKL (SEQ ID NO. 116)

Ovalbumine:

```
MGSIGAASMEFCFDVFKELKVHHANENIFYCPIAIMSALAMVYLGAKDSTRTQINKVVRF
DKLPGFGDSIEAQCGTSVNVHSSLRDILNQITKPNDVYSFSLASRLYAEERYPILPEYLQ
CVKELYRGGLEPINFQTAADQARELINSWVESQTNGIIRNVLQPSSVDSQTAMVLVNAIV
FKGLWEKAFKDEDTQAMPFRVTEQESKPVQMMYQIGLFRVASMASEKMKILELPFASGTM
SMLVLLPDEVSGLEQLESIINFEKLTEWTSSNVMEERKIKVYLPRMKMEEKYNLTSVLMA
MGITDVFSSSANLSGISSAESLKISQAVHAAHAEINEAGREVVGSAEAGVDAASVSEEFR
ADHPFLFCIKHIATNAVLFFGRCVSP (SEQ ID NO. 117)
```

hA20: Immunoglobulin of the malign B lymphocyte mouse cell line A20 (reticulum cell sarcoma) wherein the variable domains of the immunoglobulin of the malignant cell line are combined with the constant domains of a human immunoglobulin:

huA20 heavy chain_pICH61781 (SEQ ID NO. 118):

```
EVQLQQSGPDLVKPGMSVKLSCKTLGYNFSDKWIHWIKQKPGRGLEWVGRIDPSNGDTDY
NTDFKTPATLTVDRPSNTAYLELSNLTSGDSAVYYCSISGDYSACDYWGQGTELTVSSAS
TKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQ
GNVFSCSVMHEALHNHYTQKSLSLSPGK*
```

huA20 light chain_pICH6179 (SEQ ID NO. 119)

```
DVVMTQTPLSLAVSLGDHVKMSCRCNQSLVNSHGDSFLHWFLQKPGQSPKLLIYKVSSRF
FGVPERFSGSGSGTDFTLEISRVEAEDLGVYFCSQGAHVPWTFGGGTKLEIKRTVAAPSV
FIFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC*
```

2. Preparation of the plant derived hA20 idiotype immunoglobulins:

For transient expression of the recombinant proteins hA20 (SEQ ID NO. 118 and 119) in plants the magnICON system was used by vacuum-infiltration of plants using suitable *Agrobacterium* strains harbouring and transferring into the plant the expression cassette based on plant viral vectors. This method is also described in Bendandi et al. (Bendandi et al., 2010, Ann. Oncol., 21:2420-2427). Therefore the variable regions of A20 idiotype immunoglobulin were fused to human IgG1 constant region of heavy chain and constant region of kappa light chain constant region in two separate magnICON® vectors, one based on tobacco mosaic virus (TMV) and the other on potato virus X (PVX). Finally, two separate *Agrobacterium* strains were obtained, one for the light and one for the heavy chain. Both *Agrobacterium* strains were then grown separately, mixed and vacuum-infiltrated in *Nicotiana benthamiana* plants. Plants were incubated in a controlled environment chambers for 7 days, during which time the IgG1 antibody was expressed and accumulates in infiltrated leaf tissue. The biomass was then harvested and the antigen was extracted. The recombinant idiotypic antigen was subsequently purified through a series of filtration and chromatography steps including protein A affinity chromatography.

The hA20 Fab fragment was prepared from hA20 using the commercially available kit from Thermo Scientific/Pierce (kit #20341).

### 3. Preparation of nucleic acid sequences for the polymeric carrier cargo complex:

For the present examples nucleic acid sequences as indicated in example 1 were prepared and used for formation of the polymerized polymeric carrier cargo complexes or for non-polymerized carrier cargo complexes for comparison.

According to a first preparation, the DNA sequences, coding for the corresponding RNA sequences R1180, R722 and R491 sequences were prepared. The sequences of the corresponding RNAs are shown in the sequence listing (SEQ ID NOs: 112, 105, and 113).

The short GU rich sequences and the CpG 2216 oligonucleotides were prepared by automatic solid-phase synthesis by means of phosphoramidite chemistry. The sequences are shown in the sequence listing (SEQ ID NOs: 115 and 114).

### 4. *In vitro* transcription:

The respective DNA plasmids prepared according to Example 3 for R722 and R491 were transcribed *in vitro* using T7-Polymerase (T7-Opti mRNA Kit, CureVac, Tübingen, Germany) following the manufactures instructions. Subsequently the mRNA was purified using PureMessenger® (CureVac, Tübingen, Germany).

### 5. Synthesis of polymeric carrier cargo complexes:

The nucleic acid sequences defined above in Example 1 were mixed with the cationic components as defined in Example 1. Therefore, the indicated amount of nucleic acid sequence was mixed with the respective cationic component in mass ratios as indicated, thereby forming a complex. If polymerizing cationic components were used according to the present invention polymerization of the cationic components took place simultaneously to complexation of the nucleic acid cargo. Afterwards the resulting solution was adjusted with water to a final volume of 50 $\mu$l and incubated for 30 min at room temperature. The different ratios of cationic component/nucleic acid used in the experiments are shown in Table 1.

Table 1

| Sample (cationic peptide/nucleic acid) | Mass ratio | N/P ratio | Molar ratio |
|---|---|---|---|
| $CR_7C$ | 1:2 | 0.8 | 70:1 |
| $R_7$ | 1:2 | 1.0 | 85:1 |
| $CR_{12}C$/CpG | 1:2,5 | 4.9 | 8:1 |
| $CR_{12}C$/R491 | 1:2 | 0.9 | 150:1 |
| $CR_{12}C$/short GU-rich | 1:2,5 | 4.9 | 8:1 |
| $CR_{12}C$/R722 | 5:1 | 9.6 | 444:1 |
| $CR_{12}C$/R722 | 4:1 | 7.6 | 355:1 |
| $CR_{12}C$/R722 | 3:1 | 5.7 | 266:1 |
| $CR_{12}C$/R722 | 2:1 | 3.8 | 177:1 |
| $CR_{12}C$/R722 | 1:1 | 1.9 | 88:1 |
| $CR_{12}C$/R722 | 1:2 | 0.9 | 44:1 |
| $CR_{12}C$/R722 | 1:3 | 0.6 | 29:1 |
| $CR_{12}C$/R722 | 1:4 | 0.5 | 22:1 |

(continued)

| Sample (cationic peptide/nucleic acid) | Mass ratio | N/P ratio | Molar ratio |
|---|---|---|---|
| $CR_{12}C/R722$ | 1:5 | 0.4 | 17:1 |

N/P ratio = is a measure of the ionic charge of the cationic component of the polymeric carrier or of the polymeric carrier as such. In the case that the cationic properties of the cationic component are provided by nitrogen atoms the N/P ratio is the ratio of basic nitrogen atoms to phosphate residues, considering that nitrogen atoms confer to positive charges and phosphate of the phosphate backbone of the nucleic acid confers to the negative charge.

N/P is preferably calculated by the following formula:

$$N/P = \frac{pmol\ [RNA] * ratio * cationic\ AS}{\mu g\ RNA * 3 * 1000}$$

As an example the RNA R722 according to SEQ ID NO: 385 was applied, which has a molecular weight of 186 kDa. Therefore 1 $\mu$g R722 RNA confers to 5.38 pmol RNA.

### 6. Cytokine stimulation in hPBMCs:

HPBMC cells from peripheral blood of healthy donors were isolated using a Ficoll gradient and washed subsequently with 1(PBS (phophate-buffered saline). The cells were then seeded on 96-well microtiter plates (200x103/well). The hPBMC cells were incubated for 24 h with 10 $\mu$l of the polymeric carrier cargo complex from Example 3 containing the indicated amount of nucleic acid in X-VIVO 15 Medium (BioWhittaker). The immunostimulatory effect was measured by detecting the cytokine production of the hPBMCs (Tumour necrose factor alpha and Interferon alpha). Therefore, ELISA microtiter plates (Nunc Maxisorb) were incubated over night (o/n) with binding buffer (0.02% NaN3, 15 mM Na2CO3, 15 mM NaHCO3, pH 9.7), additionally containing a specific cytokine antibody. Cells were then blocked with 1×PBS, containing 1% BSA (bovine serum albumin). The cell supernatant was added and incubated for 4 h at 37°C. Subsequently, the microtiter plate was washed with 1×PBS, containing 0.05% Tween-20 and then incubated with a Biotin-labelled secondary antibody (BD Pharmingen, Heidelberg, Germany). Streptavidin-coupled horseraddish peroxidase was added to the plate. Then, the plate was again washed with 1×PBS, containing 0.05% Tween-20 and ABTS (2,2'-azino-bis(3-ethyl-benzthiazoline-6-sulfonic acid) was added as a substrate. The amount of cytokine was determined by measuring the absorption at 405 nm (OD 405) using a standard curve with recombinant cytokines (BD Pharmingen, Heidelberg, Germany) with the Sunrise ELISA-Reader from Tecan (Crailsheim, Germany). The respective results are shown in Fig. 3-9.

### 7. Zetapotential measurements:

The Zeta potential of the polymeric carrier cargo complexes was evaluated by the laser Doppler electrophoresis method using a Zetasizer Nano (Malvern Instruments, Malvern, UK). The measurement was performed at 25°C and a scattering angle of 173° was used. The results are shown in Fig. 2.

### 8. Stability of complexes after lyophilization.

The hydrodynamic diameters of polymeric carrier cargo complexes as prepared above were measured by dynamic light scattering using a Zetasizer Nano (Malvern Instruments, Malvern, UK) according to the manufacturer's instructions. The measurements were performed at 25° C in buffer analysed by a cumulant method to obtain the hydrodynamic diameters and polydispersity indices of the polymeric carrier cargo complexes. Polymeric carrier cargo complexes were formed as indicated in Example 3 and the hydrodynamic diameters were measured with fresh prepared complexes and with reconstituted complexes after lyophilization. The respective results of the experiment are shown in Fig. 1.

### 9. Immunization experiments:

For immunization the antigens Ovalbumine protein (OVA) (5 $\mu$g), the Ovalbumin-specific peptide SIINFEKL (50 $\mu$g), the indicated dose of unconjugated hA20 idiotype immunoglobulin (hA20) or the KLH-linked A20 idiotype immunoglobulin (KLH-hA20) were combined with the indicated amount of polymeric cargo complexes R722/CR12C (in a ratio of 2:1 w/w). as adjuvant and injected into mice. For comparison mice were injected without the polymeric cargo complexes.

**10. <u>Detection of an antigen-specific immune response (B-cell immune response):</u>** Ovalbumine:

Detection of an antigen specific immune response (B-cell immune response) was carried out by detecting antigen specific antibodies. Therefore, blood samples were taken from vaccinated mice 5 days after the last vaccination and sera were prepared. MaxiSorb plates (Nalgene Nunc International) were coated with *Gallus gallus* ovalbumine protein. After blocking with 1×PBS containing 0.05% Tween-20 and 1% BSA the plates were incubated with diluted mouse serum. Subsequently a biotin-coupled secondary antibody (Antimouse-IgG2a Pharmingen) was added. After washing, the plate was incubated with Horseradish peroxidase-streptavidin and subsequently the conversion of the ABTS substrate (2,2'-azino-bis(3-ethyl-benzthiazoline-6-sulfonic acid) was measured to determine the induction of IgG2a antibodies. The results of this induction of antibodies upon vaccination with an inventive pharmaceutical composition are shown in Fig 11.

hA20 or hA20 Fab fragment:

Detection of an antigen specific immune response (B-cell immune response) was carried out by detecting antigen specific antibodies. Therefore, blood samples were taken from vaccinated mice 10 or 20 days after the last vaccination (as indicated) and sera were prepared. MaxiSorb plates (Nalgene Nunc International) were coated with 1 $\mu$g/ml hA20 protein or hA20 Fab fragment (as indicated). After blocking with 1×PBS containing 0.05% Tween-20 and 1% BSA the plates were incubated with diluted mouse serum. Subsequently a biotin-coupled secondary antibody (Anti-mouse-IgG2a Pharmingen) was added. After washing, the plate was incubated with Horseradish peroxidase-streptavidin and subsequently the conversion of the ABTS substrate (2,2'-azino-bis(3-ethyl-benzthiazoline-6-sulfonic acid) was measured to determine the induction of total IgG, IgG1, and IgG2a antibodies. The results of this induction of antibodies upon vaccination with an inventive pharmaceutical composition are shown in Fig. 13 for IgG1, in Fig. 14-16 for IgG2a, and in Fig. 18 for total IgG.

**11. <u>Detection of an antigen specific cellular immune response by ELISPOT:</u>** Ovalbumine:

5 days after the last vaccination mice were sacrificed, the spleens were removed and the splenocytes were isolated. For detection of INFgamma a coat multiscreen plate (Millipore) was incubated overnight with coating buffer (0.1 M Carbonat-Bicarbonat Buffer pH 9.6, 10.59 g/l $Na_2CO_3$, 8.4g/l $NaHCO_3$) comprising antibody against INF$\Box$ (BD Pharmingen, Heidelberg, Germany). The next day 1x $10^6$ cells/well were added and re-stimulated with 1 $\mu$g/well of relevant peptide (SIINFEKL of ovalbumine); irrelevant peptide (Connexin = control peptide) or buffer without peptide. Afterwards the cells are incubated for 24h at 37°C. The next day the plates were washed 3 times with PBS, once with water and once with PBS/0.05% Tween-20 and afterwards incubated with a biotin-coupled secondary antibody for 11-24h at 4°C. Then the plates were washed with PBS/0.05% Tween-20 and incubated for 2h at room temperature with alkaline phosphatase coupled to streptavidin in blocking buffer. After washing with PBS/0.05% Tween-20 the substrate (5-Bromo-4-Cloro-3-Indolyl Phosphate/Nitro Blue Tetrazolium Liquid Substrate System from Sigma Aldrich, Taufkirchen, Germany) was added to the plate and the conversion of the substrate could be detected visually. The reaction was then stopped by washing the plates with water. The dried plates were then read out by an ELISPOT plate reader. For visualization of the spot levels the numbers were corrected by background subtraction. The results of this induction of specific cytotoxic T-cells upon vaccination with an inventive pharmaceutical composition are shown in Fig 12.

hA20:

6 days after the last vaccination mice were sacrificed, the spleens were removed and the splenocytes were isolated. For detection of INFgamma a coat multiscreen plate (Millipore) was incubated overnight with coating buffer (0.1 M Carbonat-Bicarbonat Buffer pH 9.6, 10.59 g/l $Na_2CO_3$, 8.4g/l $NaHCO_3$) comprising antibody against INF$\Box$ (BD Pharmingen, Heidelberg, Germany). The next day 1x $10^6$ cells/well were added and re-stimulated with 1 $\mu$g/well hA20 protein or buffer without peptide. Afterwards the cells are incubated for 24h at 37°C. The next day the plates were washed 3 times with PBS, once with water and once with PBS/0.05% Tween-20 and afterwards incubated with a biotin-coupled secondary antibody for 11-24h at 4°C. Then the plates were washed with PBS/0.05% Tween-20 and incubated for 2h at room temperature with alkaline phosphatase coupled to streptavidin in blocking buffer. After washing with PBS/0.05% Tween-20 the substrate (5-Bromo-4-Cloro-3-Indolyl Phosphate/Nitro Blue Tetrazolium Liquid Substrate System from Sigma Aldrich, Taufkirchen, Germany) was added to the plate and the conversion of the substrate could be detected visually. The reaction was then stopped by washing the plates with water. The dried plates were then read out by an ELISPOT plate reader. For visualization of the spot levels the numbers were corrected by background subtraction. The results of this induction of specific cytotoxic T-cells upon vaccination with an inventive pharmaceutical composition are shown in Fig 17.

**12. <u>Tumour challenge:</u>**

One week after the last vaccination $1 \times 10^6$ E.G7-OVA cells (tumour cells which stably express ovalbumine) were implanted subcutaneously in the vaccinated mice. Tumour growth was monitored by measuring the tumour size in 3 dimensions using a calliper. The results of the induction of an anti-tumoural response upon vaccination with an inventive pharmaceutical composition are shown in Fig. 10.

**13. <u>Tumour challenge:</u>**

In 2 weeks female BALB/c mice (n=10/group) are 2 times intradermally injected with 50 $\mu$g Ig A20 idiotype plus 10$\mu$g R722/CR12C (2:1; w/w), 50 $\mu$g Ig A20 idiotype conjugated to KLH and GM-CSF, 50 $\mu$g Ig A20 idiotype alone or with buffer (negative control). Two weeks after the last immunization, mice are challenged by subcutaneous transplantation of $5 \times 10^5$ A20 syngeneic tumor cells. Upon transplantation, tumour growth is assessed by caliper measurements. When tumors reach 2000 cubic mm in size, mice are sacrificed. Protection from tumor progression is deduced from the relative rate of growth of the tumour upon tumour implantation (day 0) until the date of killing, in single animals by treatment group. Data are analyzed by Kaplan-Meier analysis and plotted as percent survival.

**Claims**

1. A pharmaceutical composition comprising:

    (A) a polymeric carrier cargo complex, comprising:

        a) a polymeric carrier formed by disulfide-crosslinked cationic components as a carrier <u>and</u>
        b) at least one nucleic acid molecule as a cargo,

    <u>and</u>
    (B) at least one antigen associated with a cancer or tumour disease, particularly lymphoma or a lymphoma associated disease, wherein said antigen is an immunoglobulin idiotype of a lymphoid blood cell or a T cell receptor idiotype of a lymphoid blood cell or a fragment, variant and/or derivative of such an immunoglobulin idiotype or T cell receptor idiotype.

2. The pharmaceutical composition of claim 1, wherein said antigen is an idiotype immunoglobulin that is an idiotype antibody or an idiotype B cell receptor.

3. The pharmaceutical composition according to claim 1 or 2, wherein the lymphoma disease is B-cell lymphoma, T-cell lymphoma or Non-Hodgkin's lymphoma.

4. The pharmaceutical composition according to any one of claims 1 to 3, wherein said antigen is derived from a malignant cell, preferably derived from a malignant B cell or a malignant T cell.

5. The pharmaceutical composition according to any one of claims 1 to 4, wherein said antigen is derived or produced using a hybridoma cell.

6. The pharmaceutical composition according to any one of claims 1 to 4, wherein said antigen is a recombinant protein or peptide antigen.

7. The pharmaceutical composition according to claim 6, wherein said recombinant protein or peptide antigen is derived or produced using transgenic plants or plant cells.

8. The pharmaceutical composition according to claim 7, wherein said recombinant protein or peptide antigen is derived or produced using vacuum-infiltration of tobacco plants using *Agrobacterium* strains comprising and transferring into said plant at least one expression cassette encoding for said protein or peptide antigen, where said expression cassette is based on at least one plant virus vector.

9. The pharmaceutical composition of any one of claims 1 to 8, wherein said polymeric carrier cargo complex is for use as an immunostimulatory agent or an adjuvant.

10. The pharmaceutical composition according to any one of claims 1 to 9, wherein the nucleic acid molecule cargo is an RNA.

11. The pharmaceutical composition according to any one of claims 1 to 10, wherein the nucleic acid molecule cargo is an immunostimulatory nucleic acid.

12. The pharmaceutical composition according to any one of claims 1 to 11, wherein the nucleic acid molecule cargo is an immunostimulatory RNA (isRNA).

13. The pharmaceutical composition according to any one of claims 1 to 12, wherein the cationic components of the polymeric carrier and the nucleic acid molecule cargo comprised in said polymeric carrier cargo complex are provided in a N/P ratio in the range of 0.1-20, or in the range of 0.1-5, or in the range of 0.1-1, or in the range of 0.5-0.9.

14. The pharmaceutical composition according to any one of claims 1 to 13, wherein the disulfide-bonds contained in the cationic components of the polymeric carrier are formed by cysteine residues.

15. The pharmaceutical composition according to claim 14, wherein the cysteine residue contained in the cationic components of the polymeric carrier is located proximal to, preferably at the terminal ends of the cationic components.

16. The pharmaceutical composition according to any one of claims 1 to 15, wherein the cationic components of the carrier are cationic peptides.

17. The pharmaceutical composition according to claim 16, wherein the cationic peptides are selected from peptides according to formula (III)

$$(Arg)_l;(Lys)_m;(His)_n;(Orn)_o;(Xaa)_x,$$

wherein

$l+m+n+o+x = 3-100,$ and
l, m, n or o = independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100, provided that the overall content of Arg, Lys, His and Orn represents at least 10% of all amino acids of the cationic peptide; and Xaa is any amino acid selected from native (= naturally occurring) or non-native amino acids except of Arg, Lys, His or Orn; and
x = any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the cationic peptide,

or the cationic peptides are selected from peptides according to subformula (IIIa)

$$\{(Arg)_l;(Lys)_m;(His)_n;(Orn)_o;(Xaa')_x\,(Cys)_y\}$$

or the cationic peptides from peptides according to subformula (IIIb)

$$Cys_1\,\{(Arg)_l;(Lys)_m;(His)_n;(Orn)_o;(Xaa)_x\}\,Cys_2$$

wherein $(Arg)_l$; $(Lys)_m$; $(His)_n$; $(Orn)o$; and x are as defined above; Xaa' is any amino acid selected from native (= naturally occurring) or non-native amino acids except of Arg, Lys, His, Orn; or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, and 81-90, provided that the overall content of Arg (Arginine), Lys (Lysine), His (Histidine) and Orn (Ornithine) represents at least 10% of all amino acids of the oligopeptide and wherein $Cys_1$ and $Cys_2$ are Cysteines proximal

to, or terminal to $(Arg)_l;(Lys)_m;(His)_n;(Orn)_o;(Xaa)_x$

18. The pharmaceutical composition according to claim 17, wherein the cationic peptide is selected from CR12C (Cys-Arg-Arg-Arg-Arg-Arg-Arg-Arg-Arg-Arg-Arg-Arg-Arg-Cys) (SEQ ID NO. 6)

19. A kit or kit of parts comprising:

   (A) a polymeric carrier cargo complex as defined according to any one of claims 1, or 9 to 18; and and
   (B) at least one antigen or a fragment, variant and/or derivative thereof ,as defined according to any one of claims 1 to 8.

20. A pharmaceutical composition as defined according to any one of claims 1 to 11, or a kit or kit of parts as defined according to claim 12, for use as a vaccine.

21. A pharmaceutical composition as defined according to any one of claims 1, or 9 to 18, or a kit or kit of parts as defined according to claim 19 for use in therapy, preferably for use in the therapy of cancer or tumour disease, more preferably a cancer or tumour disease selected from the group consisting of: lymphoma, B-cell lymphoma, T cell lymphoma and Non-Hodgkin's lymphoma.

22. A polymeric carrier cargo complex as defined according to any one of claims 1, or 9 to 18 for use in therapy in combination with at least one antigen or a fragment, variant and/or derivative thereof as defined according to any one of claims 1 to 8.

23. An antigen or a fragment, variant and/or derivative thereof as defined according to any one of claims 1 to 8 for use in therapy in combination with a polymeric carrier cargo complex as defined according to any one of claims 1, or 9 to 18.

24. The polymeric carrier cargo complex as defined according to claim 22 for use in the therapy of; or the antigen or a fragment, variant and/or derivative thereof as defined according to claim 23 for use in the therapy of:

   (i) a cancer or tumour disease;
   (ii) lymphoma;
   (iii) B-cell lymphoma;
   (iv) T cell lymphoma; or
   (v) Non-Hodgkin's lymphoma

25. A pharmaceutical package, including:

   (A) a polymeric carrier cargo complex as defined according to any one of claims 1, or 9 to 18; and
   (B) instructions describing the use of said polymeric carrier cargo complex in therapy in combination with at least one antigen or a fragment, variant and/or derivative thereof as defined according to any one of claims 1 to 8.

26. A pharmaceutical package, including:

   (A) at least one antigen or a fragment, variant and/or derivative thereof as defined according to any one of claims 1 to 8; and
   (B) instructions describing the use of said antigen or fragment or variant thereof in therapy in combination with a polymeric carrier cargo complex as defined according to any one of claims 1, or 9 to 18.

27. The pharmaceutical package of claim 25 or 26, wherein said instructions further describe the use in the therapy of:

   (i) a cancer or tumour disease;
   (ii) lymphoma;
   (iii) B-cell lymphoma;
   (iv) T cell lymphoma; or
   (v) Non-Hodgkin's lymphoma

**Fig. 1**

Fig. 2

| Cationic component/nucleic acid | Ratio (w/w) | ZP mV |
|---|---|---|
| CR$_{12}$C/R722 | 5:1 | 47,5 |
| CR$_{12}$C/R722 | 4:1 | 19,6 |
| CR$_{12}$C/R722 | 3:1 | 23,8 |
| CR$_{12}$C/R722 | 2:1 | 35 |
| CR$_{12}$C/R722 | 1:1 | −14,5 |
| CR$_{12}$C/R722 | 1:2 | −14 |
| CR$_{12}$C/R722 | 1:3 | −5,16 |
| CR$_{12}$C/R722 | 1:4 | −8,07 |
| CR$_{12}$C/R722 | 1:5 | −12,4 |

A

B

Fig. 3

A

hIFNa

B

hTNFa

Fig. 4

A

B

Fig. 5

**A**

**hIFNa**

**B**

**TNFa**

**Fig. 6**

**A**

**B**

Fig. 7

**A**

**B**

Fig. 8

**A**

IFNa

**B**

TNFa

Fig. 9

# E.G7-OVA tumor growth

**Fig. 10**

Fig. 11

IFN-γ secretion in splenocytes
after stimulation with MHC I specific
SIINFEKL peptide

**Fig. 12**

## IgG1 antibodies (20 days after immunization)

Fig. 13

IgG2a antibodies (20 days after immunization)
coating with hA20

Fig. 14

**IgG2a antibodies (20 days after immunization)**
coating with hA20 Fab Fragment

Fig. 15

Fig. 16

Fig. 17

Fig. 18

Europäisches
Patentamt
European
Patent Office
Office européen
des brevets

**EUROPEAN SEARCH REPORT**

Application Number

EP 12 15 3388

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/053829 A1 (BAUMHOF PATRICK [DE] ET AL) 3 March 2011 (2011-03-03) * whole document esp. paragraphs [19, 29,108,123,126,127,263,305f,316,410] * ----- | 1-27 | INV.<br>A61K47/48<br>C12N15/87 |
| X | WO 2009/030254 A1 (CUREVAC GMBH [DE]; FOTIN-MLECZEK MARIOLA [DE]) 12 March 2009 (2009-03-12) * whole document esp. pages 10, 24, 51-59, claims * ----- | 1-27 | |
| Y | MICHAEL J HEFFERNAN ET AL: "Disulfide-Crosslinked Polyion Micelles for Delivery of Protein Therapeutics", ANNALS OF BIOMEDICAL ENGINEERING, KLUWER ACADEMIC PUBLISHERS-PLENUM PUBLISHERS, NE, vol. 37, no. 10, 10 June 2009 (2009-06-10), pages 1993-2002, XP019727009, ISSN: 1573-9686, DOI: 10.1007/S10439-009-9734-X * whole document esp. abstract * ----- | 1-27 | |
| Y | READ M L ET AL: "A versatile reducible polycation-based system for efficient delivery of a broad range of nucleic acids", NUCLEIC ACIDS RESEARCH, OXFORD UNIVERSITY PRESS, SURREY, GB, vol. 33, no. 9, 1 May 2005 (2005-05-01), pages e86-1, XP002447464, ISSN: 0305-1048, DOI: 10.1093/NAR/GNI085 [retrieved on 2005-05-24] * whole document esp. abstract and page 15(e86) * ----- | 1-27 | TECHNICAL FIELDS SEARCHED (IPC)<br><br>A61K<br>C12N |

-/--

The present search report has been drawn up for all claims

| Place of search | Date of completion of the search | Examiner |
|---|---|---|
| Munich | 17 January 2013 | Brück, Marianne |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

# EUROPEAN SEARCH REPORT

Application Number

EP 12 15 3388

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | READ M L ET AL: "Vectors based on reducible polycations facilitate intracellular release of nucleic acids", JOURNAL OF GENE MEDICINE, JOHN WILEY & SONS, INC, US, vol. 5, no. 3, 1 March 2003 (2003-03-01), pages 232-245, XP002481542, ISSN: 1099-498X, DOI: 10.1002/JGM.331 * whole document esp. abstract and page 244 * | 1-27 | |
| Y | MCKENZIE D L ET AL: "Low molecular weight disulfide cross-linking peptides as nonviral gene delivery carriers", BIOCONJUGATE CHEMISTRY, ACS, WASHINGTON, DC, US, vol. 11, no. 6, 20 November 2000 (2000-11-20), pages 901-909, XP002609379, ISSN: 1043-1802, DOI: 10.1021/BC000056I [retrieved on 2000-11-02] * whole document esp. abstract and page 908 * | 1-27 | |

TECHNICAL FIELDS
SEARCHED (IPC)

The present search report has been drawn up for all claims

| Place of search | Date of completion of the search | Examiner |
|---|---|---|
| Munich | 17 January 2013 | Brück, Marianne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 12 15 3388

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2013

| Patent document cited in search report | | Publication date | Patent family member(s) | | Publication date |
|---|---|---|---|---|---|
| US 2011053829 | A1 | 03-03-2011 | AU | 2010291499 A1 | 19-01-2012 |
| | | | CA | 2765421 A1 | 10-03-2011 |
| | | | CN | 102573919 A | 11-07-2012 |
| | | | EP | 2331138 A1 | 15-06-2011 |
| | | | KR | 20120102586 A | 18-09-2012 |
| | | | SG | 178013 A1 | 29-03-2012 |
| | | | US | 2011053829 A1 | 03-03-2011 |
| | | | US | 2012219573 A1 | 30-08-2012 |
| | | | WO | 2011026641 A1 | 10-03-2011 |
| WO 2009030254 | A1 | 12-03-2009 | AU | 2008295071 A1 | 12-03-2009 |
| | | | CA | 2696752 A1 | 12-03-2009 |
| | | | CN | 101796191 A | 04-08-2010 |
| | | | EP | 2484770 A1 | 08-08-2012 |
| | | | JP | 2010537651 A | 09-12-2010 |
| | | | KR | 20100085020 A | 28-07-2010 |
| | | | RU | 2010112935 A | 10-10-2011 |
| | | | US | 2010203076 A1 | 12-08-2010 |
| | | | WO | 2009030254 A1 | 12-03-2009 |
| | | | WO | 2009030481 A1 | 12-03-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009030481 A, Fotin-Mleczek
- US 6770740 B1
- WO 2010003193 A
- WO 03086280 A
- WO 2010040531 A

### Non-patent literature cited in the description

- **KWAK, L. W. ; M. J. CAMPBELL et al.** Induction of immune responses in patients with B-cell lymphoma against the surface-immunoglobulin idiotype expressed by their tumors. *N Engl J Med,* 1992, vol. 327 (17), 1209-15
- **BENDANDI, M.** Idiotype vaccines for lymphoma: proof-of-principles and clinical trial failures. *Nat Rev Cancer,* 2009, vol. 9 (9), 675-81
- **HOUOT, R. ; R. LEVY.** Vaccines for lymphomas: idiotype vaccines and beyond. *Blood Rev,* 2009, vol. 23 (3), 137-42
- **GUY, B.** *Nat Rev Microbiol,* 2007, vol. 5 (7), 505-17
- **NEELAPU, S. S. ; S. T. LEE et al.** Therapeutic lymphoma vaccines: importance of T-cell immunity. *Expert Rev Vaccines,* 2006, vol. 5 (3), 381-94
- **HONG, S. ; J. QIAN et al.** CpG or IFN-alpha are more potent adjuvants than GM-CSF to promote anti-tumor immunity following idiotype vaccine in multiple myeloma. *Cancer Immunol Immunother.,* 2011
- **MEYLAN, E. ; J. TSCHOPP et al.** *Nature,* 2006, vol. 442 (7098), 39-44
- **FOERG, C. ; MERKLE, H.P.** *J Pharm Ski,* 2008, vol. 97, 144-62
- **GAO, X. ; KIM, K. ; LIU, D.** *AAPS J,* 2007, vol. 9, E92-104
- **READ, M.L. et al.** *J Gene Med.,* 2003, vol. 5, 232-245
- **READ, M.L. et al.** *Nucleic Acids Res,* 2005, vol. 33, e86
- **MCKENZIE, D. L. ; K. Y. KWOK et al.** *J Biol Chem,* 2000, vol. 275 (14), 9970-7
- **MCKENZIE, D. L. ; E. SMILEY et al.** *Bioconjug Chem,* 2000, vol. 11 (6), 901-9
- **AKASHI.** *Curr. Opin. Genet. Dev.,* 2001, vol. 11 (6), 660-666
- **HOLCIK et al.** *Proc. Natl. Acad. Sci. USA,* 1997, vol. 94, 2410-2414
- **LAI et al.** *Development,* 1995, vol. 121, 2349-2360
- Purification of PCR Products. **MEZEI ; STORTS.** PCR Technology: Current Innovation. CRC Press, 2001
- Absorption, Circular Dichroism and ORD of Polypeptides. **URRY et al.** Modern Physical Methods in Biochemistry. Elsevier, 1985
- **KARLIN et al.** *PNAS USA,* 1993, vol. 90, 5873-5877
- **ALTSCHUL et al.** *Nucleic Acids Res.,* 1997, vol. 25, 3389-3402
- **ZAMORE.** *Nat. Struct. Biol.,* 2001, vol. 9, 746-750
- **SHARP.** *Genes Dev.,* 2001, vol. 5, 485-490
- **HANNON.** *Nature,* 2002, vol. 41, 244-251
- **STARK et al.** *Annu. Rev. Biochem.,* 1998, vol. 67, 227-264
- **HE ; KATZE.** *Viral Immunol.,* 2002, vol. 15, 95-119
- **ELBASHIR et al.** *Nature,* 2001, vol. 411, 494-498
- **HEMMI H et al.** *Nature,* 2000, vol. 408, 740-5
- **BAUER S et al.** *Proc NatlAcadSci USA,* 2001, vol. 98, 9237-42
- **MEYLAN, E. ; TSCHOPP, J.** Toll-like receptors and RNA helicases: two parallel ways to trigger antiviral responses. *Mol. Cell,* 2006, vol. 22, 561-569
- **OSTERROTH et al.** *J. Immunol Methods.,* 1999, vol. 229, 141-153
- **MCCORMICK et al.** *J. Immunol Methods.,* 2003, vol. 278, 95-104
- **BENDANDI et al.** *Ann. Oncol.,* 2010, vol. 21, 2420-2427
- **MARILLONNET et al.** *Nat. Biotechnol.,* 2005, vol. 23, 718-723
- **SANTI et al.** *Proc.Natl. Acad. Sci. USA,* 2006, vol. 103, 861-866
- **WEBSTER et al.** *Plant Biotechnol. J.,* 2009, vol. 7, 846-855
- **KALTHOFF et al.** *J. Virol.,* 2010, vol. 84, 12002-12010
- **GLEBA et al.** *Vaccine,* 2005, vol. 23, 2042-2048

- **GLEBA et al.** *Curr. Opin. Biotechnol.,* 2007, vol. 18, 134-141
- **GIRITCH et al.** *Proc. Natl. Acad. Sci. USA.,* 2006, vol. 103, 14701-14706
- **CASTILHO et al.** *PLoS One,* 2011, vol. 6, e26040